# FASCICULE DE BREVET EUROPEEN

(11) **EP 1 674 073 B1**
(45) Date de publication et mention de la délivrance du brevet: **30.09.2009**
(21) Numéro de dépôt: 05292742.3
(22) Date de dépôt: 20.12.2005
(51) Int. Cl.: A61K 8/58, A61Q 5/06

(54) **Utilisation de porphyrine ou phtalocyanine particuliere pour la coloration de matieres keratiniques humaines, compositions les comprenant, procede de coloration et composes**
Verwendung bestimmter Porphyrine oder Phtalocyanine zur Färbung menschlicher keratinischer Materialien, sie enthaltende Zusammensetzungen, Färbeverfahren et Substanzen
Use of specific porphyrins or phtalocyanins for colouring human keratinic material, compositions comprising them, colouring process and components

(30) Priorité: 23.12.2004 FR 0453219
(43) Date de publication de la demande: 28.06.2006
(73) Titulaire: L'ORÉAL, 75008 Paris (FR)
(72) Inventeur: Hercouet, Leïla, 93360 Neully Plaisance (FR); Lagrange, Alain, 77700 Coupvray (FR)
(74) Mandataire: Fevrier, Murielle Françoise E.

(56) Documents cités:
- WO-A-99/13846
- US-A- 3 592 581
- US-A- 5 650 137
- US-A1- 2004 237 217

## Description

La présente invention a pour objet l'utilisation de composés de type porphyrine ou phtalocyanine particuliers pour la coloration de matières kératiniques humaines. Elle a de même pour objet une composition comprenant, dans un milieu approprié pour la teinture, au moins un composé de type porphyrine ou phtalocyanine particulier à titre de colorant direct, ainsi qu'une composition prête à l'emploi comprenant ce(s) colorant(s) direct(s). Elle concerne de plus un procédé de coloration mettant en oeuvre la composition ou la composition prête à l'emploi, ainsi qu'un dispositif pour la mise en oeuvre de ce procédé. Enfin, l'invention concerne des composés de type phtalocyanine particuliers en tant que tels.

L'invention concerne plus particulièrement le domaine de la coloration des fibres kératiniques humaines, et notamment des cheveux.

Par ailleurs, elle est relative au domaine de la coloration dite semi-permanente, encore appelée coloration directe, qui consiste à appliquer une composition tinctoriale comprenant, à titre de colorant, au moins une molécule colorée qui pénètre par diffusion à l'intérieur de la fibre et/ou reste adsorbée à sa surface. La composition est appliquée en l'absence ou en présence d'au moins un agent oxydant. Dans ce dernier cas, on parle de coloration directe en conditions éclaircissantes.

On peut rencontrer des problèmes avec des colorants directs de couleur bleue ou verte, voire marron. En effet, de tels colorants directs présentent souvent des stabilités vis-à-vis de la lumière, vis-à-vis d'agents chimiques comme les agents oxydants et/ou réducteurs, qui peuvent être considérées encore insuffisantes.

US-A-3 592 581 (ALBERT SHANSKY ET AL) 13 juillet 1971 (1971-07-13) divulgue des colorants capillaires de type phthalocyanine.

La présente invention a pour objet l'utilisation de composés différents de ceux mis en oeuvre jusqu'à présent, en tant que colorants directs pour la coloration de matières kératiniques humaines, et plus particulièrement de fibres, qui permettent d'accéder à des nuances bleues vertes ou marrons qui soient stables, tenaces, et qui, de plus, ne virent pas dans le temps.

Ainsi, la présente invention a pour objet l'utilisation d'au moins un composé de type porphyrine ou phtalocyanine cationique non métallique ou cationique métallique ne comprenant à titre d'élément métallique qu'un ou plusieurs métaux ou ions métalliques de la colonne IA, IIA de la classification périodique des éléments, le zinc et le silicium, comme colorant direct pour la coloration de matières kératiniques humaines, et notamment de fibres.

Elle a aussi pour objet une composition tinctoriale comprenant, dans un milieu approprié pour la teinture des matières kératiniques humaines, au moins un composé de type porphyrine ou phtalocyanine cationique non métallique ou cationique métallique ne comprenant à titre d'élément métallique qu'un ou plusieurs métaux ou ions métalliques de la colonne IA, IIA de la classification périodique des éléments, le zinc et le silicium, comme colorant direct, et au moins un tensioactif et/ou au moins un polymère.

Un autre objet de l'invention est constitué par une composition prête à l'emploi comprenant au moins un composé de type porphyrine ou phtalocyanine cationique non métallique ou cationique métallique ne comprenant à titre d'élément métallique qu'un ou plusieurs métaux ou ions métalliques de la colonne IA, IIA de la classification périodique des éléments, le zinc et le silicium, comme colorant direct et au moins un agent oxydant.

La présente invention a de même pour objet un procédé de teinture de matières kératiniques humaines, notamment des fibres, consistant à appliquer la composition selon l'invention, en l'absence d'agent oxydant, avec ou sans rinçage final.

Selon une autre variante de l'invention, le procédé consiste à appliquer une composition selon l'invention, en présence d'un agent oxydant, à laisser poser pendant une durée suffisante pour obtenir la coloration souhaitée.

Enfin, l'invention concerne un dispositif à plusieurs compartiments pour la coloration des matières kératiniques et notamment des fibres, comprenant un ou plusieurs compartiments avec, dans un milieu approprié pour la teinture des fibres kératiniques humaines, le ou les composés de type porphyrine et/ou phtalocyanine cationique non métallique ou cationique métallique ne comprenant à titre d'élément métallique qu'un ou plusieurs métaux ou ions métalliques de la colonne IA, IIA de la classification périodique des éléments, le zinc et le silicium, éventuellement au moins un colorant direct additionnel, éventuellement au moins une base d'oxydation et/ou au moins un coupleur ; ces composés étant présents dans une ou plusieurs compositions, associés ou non, et, d'autre part, un compartiment avec une composition comprenant, dans un milieu approprié pour la teinture des fibres kératiniques humaines, au moins un agent oxydant.

On a en effet remarqué de manière totalement inattendue, que des composés de type porphyrine et phtalocyanine cationiques non métalliques ou métalliques particulières, permettaient d'obtenir une bonne coloration de la matière traitée, peu sélective, tenace vis-à-vis, par exemple, des lavages, et ce quel que soit le pH auquel le procédé de coloration est mis en oeuvre.

On a aussi constaté que ce type de composé donnait de bons résultats, en conditions éclaircissantes ou non.

Les composés de type porphyrine ou phtalocyanine cationique non métallique ou cationique métallique tel que défini auparavant, constituent une alternative intéressante pour les colorants directs utilisés classiquement, en particulier pour la coloration de cheveux sensibilisés.

En effet, sur ce type de cheveu, ces colorants ont une très bonne résistance aux shampooings répétés.

D'une manière générale, ces porphyrines ou phtalocyanines mises en oeuvre dans le cadre de la présente invention peuvent présenter à la fois plusieurs avantages, comme permettre l'obtention d'une coloration puissante et homogène du cheveu, même en conditions éclaircissantes (présence d'un agent oxydant et d'un agent alcalin). Enfin, leur structure chimique particulière, et plus particulièrement leur masse molaire élevée, ainsi que la présence d'au moins une charge cationique, limitent leur pénétration transcutanée.

Mais d'autres avantages et caractéristiques de l'invention apparaîtront plus clairement à la lecture de la description et des exemples qui vont suivre.

Il est précisé qu'à moins d'une indication particulière différente, les bornes des domaines de valeurs qui seront donnés, sont incluses dans ces domaines.

De plus, la classification périodique des éléments à laquelle il est fait référence correspond à celle publiée dans la 12^{ème} Edition de l'ouvrage 'Merck Index'.

Par ailleurs, à moins d'une indication plus précise, par radical alkyle, alcényle, alcynyle substitué, on désigne un radical alkyle, alcényle, alcynyle, portant au moins :
* un groupe hydroxyle ;
* un radical alcoxy en C₁-C₁₀, linéaire ou ramifié ;
* un radical amino ;
* un radical amino substitué par un ou plusieurs radicaux alkyle en C₁-C₁₀, linéaires
ou ramifiés, éventuellement porteurs d'un ou plusieurs groupements hydroxyle ou alcoxy en C₁-C₁₀, linéaires ou ramifiés, lesdits radicaux pouvant former avec l'atome d'azote auquel ils sont reliés, un hétérocycle à 5 ou 6 chaînons, comprenant éventuellement un autre hétéroatome, plus particulièrement un atome d'azote ou d'oxygène ; au moins un radical aryle en C₆, aryl(C₆)alkyle(C₁-C₁₀), linéaire ou ramifié ;
* un radical ammonium portant trois radicaux identiques ou différents choisis parmi les atomes d'hydrogène, les radicaux alkyle en C₁-C₁₀, linéaires ou ramifiés, éventuellement porteurs d'un ou plusieurs groupements hydroxyle ou alcoxy en C₁-C₁₀, linéaires ou ramifiés, deux des radicaux alkyle pouvant former ensemble avec l'atome d'azote auquel ils sont reliés, un hétérocycle à 5 ou 6 chaînons, comprenant éventuellement un autre hétéroatome, plus particulièrement un atome d'azote, d'oxygène ou de soufre.

En outre, à moins d'une indication plus précise, par radical aryle substitué, on désigne un radical aryle portant au moins :
* un groupe hydroxyle ;
* un radical alcoxy en C₁-C₁₀, linéaire ou ramifié ;
* un radical amino ;
* un radical amino substitué par un ou plusieurs radicaux alkyle en C₁-C₁₀, identiques
ou différents, linéaires ou ramifiés, éventuellement porteurs d'un ou plusieurs groupements hydroxyle ou alcoxy en C₁-C₁₀, linéaires ou ramifiés, lesdits radicaux alkyle pouvant former avec l'atome d'azote auquel ils sont reliés, un hétérocycle à 5 ou 6 chaînons, comprenant éventuellement un autre hétéroatome, plus particulièrement un atome d'azote, d'oxygène ou de soufre ; au moins un radical aryle en C₆, aryl(C₆)alkyle(C₁-C₁₀) linéaire ou ramifié ;
* un radical ammonium portant trois radicaux identiques ou différents choisis parmi les atomes d'hydrogène, les radicaux alkyle en C₁-C₁₀, linéaires ou ramifiés, éventuellement porteurs d'un ou plusieurs groupements hydroxyle ou alcoxy en C₁-C₁₀. linéaires ou ramifiés, deux des radicaux alkyle pouvant former ensemble avec l'atome d'azote auquel ils sont reliés, un hétérocycle à 5 ou 6 chaînons, comprenant éventuellement un autre hétéroatome, plus particulièrement un atome d'azote, d'oxygène ou de soufre.
* un radical -CONH₂ ou -NHCOR dans laquelle R représente un radical alkyle linéaire ou ramifié en C₁-C₁₀;
* un hétérocycle saturé ou non, à 5 ou 6 chaînons, éventuellement substitué, comprenant au moins un hétéroatome choisi parmi l'azote, l'oxygène, le soufre ;
* un radical nitro ;
* un atome d'halogène, notamment le chlore.

Il est précisé que les indications données ci-dessus sont valables qu'il s'agisse d'un radical simple ou composé (par exemple de type alcoxy, arylalkyle, alkylaryle, etc.).

Enfin, par radical hétérocyclique substitué, on désigne un radical hétérocyclique portant au moins :
* un groupe hydroxyle ;
* un radical alcoxy en C₁-C₁₀, linéaire ou ramifié ;
* un radical amino ;
* un radical amino substitué par un ou plusieurs radicaux alkyle en C₁-C₁₀, linéaires
ou ramifiés, éventuellement porteurs d'un ou plusieurs groupements hydroxyle ou alcoxy en C₁-C₁₀, linéaires ou ramifiés, lesdits radicaux alkyle pouvant former avec l'atome d'azote auquel ils sont reliés, un hétérocycle à 5 ou 6 chaînons, comprenant éventuellement un autre hétéroatome, plus particulièrement un atome d'azote, d'oxygène ou de soufre ; au moins un radical aryle en C₆, aryl(C₆)alkyle(C₁-C₁₀) linéaire ou ramifié ;
* un radical ammonium portant trois radicaux identiques ou différents choisis parmi les atomes d'hydrogène, les radicaux alkyle en C₁-C₁₀, linéaires ou ramifiés, éventuellement porteurs d'un ou plusieurs groupements hydroxyle ou alcoxy, en C₁-C₁₀, linéaires ou ramifiés, deux des radicaux alkyle pouvant former ensemble avec l'atome d'azote auquel ils sont reliés, un hétérocycle à 5 ou 6 chaînons, comprenant éventuellement un autre hétéroatome, plus particulièrement un atome d'azote d'oxygène ou de soufre ,
* un atome d'halogène, notamment le chlore.

Comme indiqué auparavant, la présente invention a pour objet l'utilisation de composés de type porphyrine ou phtalocyanine cationique non métallique ou cationique métallique ne comprenant à titre d'élément métallique qu'un ou plusieurs métaux ou ions métalliques de la colonne IA, IIA, le zinc, et le silicium, en tant que colorant direct de matières kératiniques humaines, et plus particulièrement de fibres kératiniques humaines, telles que par exemple les cheveux.

Plus particulièrement, le composé porphyrine ou phtalocyanine cationique, métallique
ou non métallique, ne comprend pas de métal susceptible de dégrader les oxydants. Il est à noter que ledit composé ne comprend pas de tels métaux dans les contre-ions qui lui sont associés.

Parmi les métaux susceptibles de dégrader les oxydants, et notamment le peroxyde d'hydrogène, on peut citer tout spécialement les métaux de transition (c'est-à-dire ceux pour lesquels la couche électronique d n'est pas complète). A titre d'exemples plus particuliers, on peut citer le cuivre, le fer, le cobalt et le nickel.

Selon une caractéristique avantageuse de l'invention, le composé porphyrine ou phtalocyanine cationique, métallique ou non métallique, est soluble dans la composition, à la teneur utilisée, à température ambiante et pression atmosphérique.

Parmi les composés cationiques métalliques ou non métalliques de type porphyrine, utilisables dans le cadre de la présente invention, on peut citer plus particulièrement les composés correspondant à la formule (1) suivante, et ses formes tautomères, portant au moins une charge cationique :

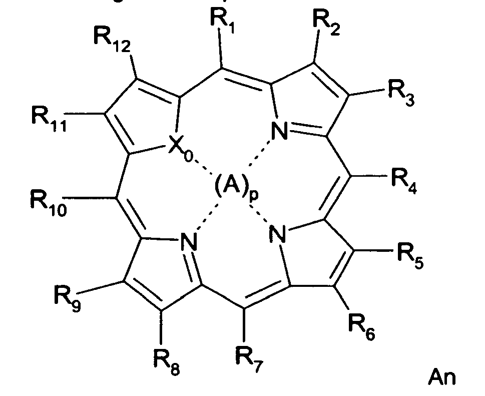

Dans laquelle :
**les radicaux R₁ à R₁₂**, identiques ou non, représentent :
   ■ un atome d'hydrogène ;
   ■ un radical alkyle en C₁-C₃₀, linéaire ou ramifié ; un radical alcényle en C₂-C₃₀, linéaire
ou ramifié ; un radical alcynyle en C₂-C₃₀, linéaire ou ramifié ;
■ lesdits radicaux alkyle, alcényle, alcynyle pouvant éventuellement être substitués par au moins un groupement monovalent choisi parmi les groupements suivants, ou leurs combinaisons :
   - hydroxyle,
   - amino ;
   - amino substitué par un ou deux radicaux alkyle identiques ou différents, en C₁-C₁₀, linéaires ou ramifiés, éventuellement porteur d'un ou plusieurs groupements hydroxyle ou alcoxy, en C₁-C₁₀, linéaires ou ramifiés ;
   - ammonium substitué par un, deux ou trois radicaux alkyle identiques ou différents, en C₁-C₁₀, linéaires ou ramifiés, éventuellement porteur d'un ou plusieurs groupements hydroxyle ou alcoxy, en C₁-C₁₀, linéaires ou ramifiés ;
   - hydrogénocarbonyle (-COH) ;
   - un hétérocycle à 5 ou 6 chaînons, comprenant un ou plusieurs hétéroatomes comme l'oxygène, l'azote, éventuellement substitué, éventuellement porteur d'au moins une charge cationique ; ledit hétérocycle pouvant être condensé avec un noyau aromatique, de préférence à 6 chaînons ;
■ et/ou lesdits radicaux alkyle, alcényle, alcynyle pouvant éventuellement être interrompus par au moins un groupement divalent choisi parmi les groupements :
   - un atome d'oxygène ;
   - amino ;
   - amino substitué par un radical alkyle en C₁-C₁₀, linéaire ou ramifié, éventuellement porteur d'un ou plusieurs groupements hydroxyle ou alcoxy, en C₁-C₁₀, linéaires ou ramifiés ;
   - ammonium substitué par un ou deux radicaux alkyle identiques ou différents, en C₁-C₁₀, linéaires ou ramifiés, éventuellement porteur d'un ou plusieurs groupements hydroxyle ou alcoxy, en C₁-C₁₀, linéaires ou ramifiés ;
   - carbonyle (-CO-) ;
   - un hétérocycle à 5 ou 6 chaînons, comprenant un ou plusieurs hétéroatomes comme l'oxygène, l'azote, éventuellement substitué, éventuellement porteur d'au moins une charge cationique
■ un radical aryle en C₆-C₃₀, éventuellement substitué ; un radical alkyl(C₁-C₃₀)aryle(C₆-C₃₀), éventuellement substitué ; un radical aryl(C₆-C₃₀)alkyle(C₁-C₃₀), éventuellement substitué ;
■ un groupe hydroxyle ;
■ un radical alcoxy en C₁-C₃₀, linéaire ou ramifié, éventuellement substitué ;
■ un radical amino ; un radical amino portant un ou plusieurs radicaux alkyles, linéaires
ou ramifiés en C₁-C₃₀, éventuellement substitués ;
■ un radical -SO₂-NH₂, -SO₂NH-alkyle, -NH-SO₂-alkyle, dans lesquels le radical alkyle est en C₁-C₃₀, linéaire ou ramifié, éventuellement substitué ;
■ un radical hétérocyclique éventuellement cationique comprenant de 5 à 6 chaînons, de préférence aromatique, comprenant au moins un atome d'azote, et éventuellement au moins un autre hétéroatome, identique ou différent ; ledit radical hétérocyclique étant éventuellement substitué ;
■ l'un au moins des radicaux R₁ à R₁₂ porte au moins une charge cationique ;
   **X₀** représente un atome d'azote ou un atome d'oxygène ou un atome de soufre ;
   **A** représente un métal ou un ion métallique de la colonne IA, IIA de la classification périodique des éléments, tels que le sodium, le potassium, le magnésium, le calcium ; le zinc
ou le silicium ;
**p** vaut 0, ou 1 ou 2 selon la nature de l'élément A ;
**An** représentant un ou plusieurs anions cosmétiquement acceptables compensant la charge cationique totale du composé.

Avantageusement, les radicaux R₂, R₃, R₅, R₆, R₈, R₉, R₁₁ R₁₂, identiques ou non, représentent :
* un atome d'hydrogène ;
* un radical alkyle en C₁-C₁₂, de préférence en C₁-C₈, linéaire ou ramifié, éventuellement substitué par au moins un groupement ammonium substitué par un, deux ou trois radicaux alkyle identiques ou différents, en C₁-C₈, linéaires ou ramifiés, éventuellement porteurs d'un ou plusieurs groupements hydroxyle ou alcoxy, en C₁-C₈, linéaires ou ramifiés ;
* -SO₂NH-alkyle dans lesquels le radical alkyle est en C₁-C₁₂ linéaire ou ramifié, éventuellement substitué par au moins un groupement ammonium substitué par un, deux ou trois radicaux alkyle identiques ou différents, en C₁-C₈, linéaires ou ramifiés, éventuellement porteurs d'un ou plusieurs groupements hydroxyle ou alcoxy, en C₁-C₈, linéaires ou ramifiés ;
* un radical phényle éventuellement substitué ;
* un radical alcoxy en C₁-C₁₂, de préférence en C₁-C₈, linéaire ou ramifié ;
* un radical amino substitué par un ou deux radicaux alkyle identiques ou différents, en C₁-C₁₀, linéaires ou ramifiés, éventuellement porteurs d'un ou plusieurs groupements hydroxyle ou alcoxy, en C₁-C₈, linéaires ou ramifiés ;
* un hétérocycle à 5 ou 6 chaînons, comprenant un ou plusieurs hétéroatomes comme l'oxygène, l'azote, éventuellement substitué, éventuellement porteur d'au moins une charge cationique.

De plus, conformément à un mode de réalisation particulier de l'invention, les radicaux R₁, R₄, R₇ et R₁₀, identiques ou non, représentent :
* un radical pyridinium rattaché de préférence au moyen de l'un des atomes de carbone de l'hétérocycle ;
* un radical quinolinium éventuellement substitué ;
* un radical phényle éventuellement substitué par au moins :
   un groupement ammonium substitué par un, deux ou trois radicaux alkyle identiques ou différents, en C₁-C₈, linéaires ou ramifiés, éventuellement porteurs d'un
      ou plusieurs groupements hydroxyle ou alcoxy, en C₁-C₈ linéaires ou ramifiés ;
      un atome de chlore,
      un groupement nitro,
      un groupement hydroxyle,
      un radical alcoxy en C₁-C₈, linéaire ou ramifié ;
      un groupement -CONH₂,
      un groupement -CO-NHR avec R représentant un radical alkyle en C₁-C₈, linéaire ou ramifié ;
      un hétérocycle à 5 ou 6 chaînons, comprenant un ou plusieurs hétéroatomes comme l'oxygène, l'azote, éventuellement substitué, éventuellement porteur d'au moins une charge cationique.

Selon une variante plus particulière de l'invention, lorsque A représente un métal non monovalent, et tout particulièrement dans le cas du silicium, ce dernier peut être lié de manière covalente à un radical hydrocarboné portant éventuellement au moins une charge cationique. Dans ce cas, ledit radical hydrocarboné représente un radical alkyle en C₁-C₃₀, linéaire ou ramifié ; un radical alcényle en C₂-C₃₀, linéaire ou ramifié ; un radical alcynyle en C₂-C₃₀, linéaire ou ramifié ; lesdits radicaux alkyle, alcényle, alcynyle pouvant éventuellement être substitués et/ou éventuellement être interrompus par au moins un atome d'oxygène, un groupement carbonyle, un groupement -NR-, -N⁺R₂, dans lesquels les radicaux R, identiques ou non, représentent un atome d'hydrogène, un radical alkyle en C₁-C₃₀, linéaire
ou ramifié éventuellement substitué, un hétérocycle à 5 ou 6 chaînons, comprenant un ou plusieurs hétéroatomes comme l'oxygène, l'azote, éventuellement substitué, éventuellement porteur d'au moins une charge cationique.

Parmi les composés cationiques non métalliques ou métalliques particuliers de type phtalocyanine, utilisables dans le cadre de la présente invention, on peut citer plus particulièrement les composés correspondant à la formule (2) suivante, et ses formes tautomères, portant au moins une charge cationique :

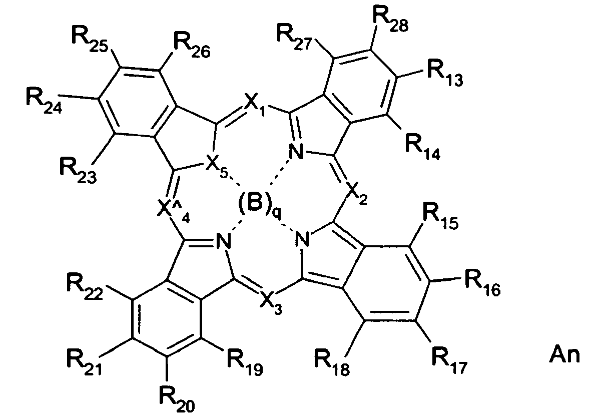

Dans laquelle
**les radicaux R₁₃ à R₂₈,** identiques ou non, représentent :
■ un atome d'hydrogène ;
■ un radical alkyle en C₁-C₃₀, linéaire ou ramifié ; un radical alcényle en C₂-C₃₀, linéaire
ou ramifié ; un radical alcynyle en C₂-C₃₀, linéaire ou ramifié ;
■ lesdits radicaux alkyle, alcényle, alcynyle pouvant éventuellement être substitués par au moins un groupement monovalent choisi parmi les groupements suivants, ou leurs combinaisons :
   - hydroxyle,
   - amino ;
   - amino substitué par un ou deux radicaux alkyle identiques ou différents, en C₁-C₁₀, linéaires ou ramifiés, éventuellement porteur d'un ou plusieurs groupements hydroxyle ou alcoxy, en C₁-C₁₀, linéaires ou ramifiés ;
   - ammonium substitué par un, deux ou trois radicaux alkyle identiques ou différents, en C₁-C₁₀, linéaires ou ramifiés, éventuellement porteur d'un ou plusieurs groupements hydroxyle ou alcoxy, en C₁-C₁₀, linéaires ou ramifiés ;
   - hydrogénocarbonyle (-COH) ;
   - un hétérocycle à 5 ou 6 chaînons, comprenant un ou plusieurs hétéroatomes comme l'oxygène, l'azote, éventuellement substitué, éventuellement porteur d'au moins une charge cationique ; ledit hétérocycle pouvant être condensé avec un noyau aromatique, de préférence à 6 chaînons ;
■ et/ou lesdits radicaux alkyle, alcényle, alcynyle pouvant éventuellement être interrompus par au moins un groupement divalent choisi parmi les groupements :
   - un atome d'oxygène ;
   - amino ;
   - amino substitué par un radical alkyle en C₁-C₁₀, linéaire ou ramifié, éventuellement porteur d'un ou plusieurs groupements hydroxyle ou alcoxy, en C₁-C₁₀, linéaires ou ramifiés ;
   - ammonium substitué par un ou deux radicaux alkyle identiques ou différents, en C₁-C₁₀, linéaires ou ramifiés, éventuellement porteur d'un ou plusieurs groupements hydroxyle ou alcoxy, en C₁-C₁₀, linéaires ou ramifiés ;
   - carbonyle (-CO-) ;
   - un hétérocycle à 5 ou 6 chaînons, comprenant un ou plusieurs hétéroatomes comme l'oxygène, l'azote, éventuellement substitué, éventuellement porteur d'au moins une charge cationique
■ un radical aryle en C₆-C₃₀, éventuellement substitué ; un radical alkyl(C₁-C₃₀)aryle(C₆-C₃₀), éventuellement substitué ; un radical aryl(C₆-C₃₀)alkyle(C₁-C₃₀), éventuellement substitué ;
■ un groupe hydroxyle ;
■ un radical alcoxy en C₁-C₃₀, linéaire ou ramifié, éventuellement substitué ;
■ un radical amino ; un radical amino portant un ou plusieurs radicaux alkyles, linéaires
   ou ramifiés en C₁-C₃₀, éventuellement substitués ;
■ un radical -SO₂-NH₂, -SO₂NH-alkyle, -NH-SO₂-alkyle, dans lesquels le radical alkyle est en C₁-C₃₀, linéaire ou ramifié, éventuellement substitué ;
■ un radical hétérocyclique éventuellement cationique comprenant de 5 à 6 chaînons, de préférence aromatique, comprenant au moins un atome d'azote, et éventuellement au moins un autre hétéroatome, identique ou différent ; ledit radical hétérocyclique étant éventuellement substitué ;
■ l'un au moins des radicaux R₁₃ à R₂₈ porte au moins une charge cationique ;
   **X₁ à X₄,** identiques ou non, représentent un atome d'azote ou un groupement -CR₂₉, avec R₂₉ ayant la même définition que R₁₃ à R₂₈ précités ;
   **X₅** représente un atome d'azote ou un atome d'oxygène ou un atome de soufre ;
   **B** représente un métal ou un ion métallique de la colonne IA, IIA de la classification périodique des éléments, tels que le sodium, le potassium, le magnésium, le calcium ; le zinc ou le silicium ;
   **q** vaut 0, ou 1 ou 2 selon la nature de l'élément B ;
   **An** représentant un ou plusieurs anions cosmétiquement acceptables compensant la charge cationique totale du composé.

Avantageusement, les radicaux R₁₃ à R₂₈, identiques ou non, représentent :
* un atome d'hydrogène ;
* un radical alkyle en C₁-C₁₂, de préférence en C₁-C₈, linéaire ou ramifié, éventuellement substitué par au moins un groupement ammonium substitué par un, deux ou trois radicaux alkyle identiques ou différents, en C₁-C₈, linéaires ou ramifiés, éventuellement porteurs d'un ou plusieurs groupements hydroxyle ou alcoxy, en C₁-C₈, linéaires ou ramifiés ;
* -SO₂NH-alkyle dans lesquels le radical alkyle est en C₁-C₁₂ linéaire ou ramifié, éventuellement substitué par au moins un groupement ammonium substitué par un, deux ou trois radicaux alkyle identiques ou différents, en C₁-C₈, linéaires ou ramifiés, éventuellement porteurs d'un ou plusieurs groupements hydroxyle ou alcoxy, en C₁-C₈, linéaires ou ramifiés ;
* un radical alcoxy en C₁-C₁₂, de préférence en C₁-C₈, linéaire ou ramifié ;
* un radical amino substitué par un ou deux radicaux alkyle identiques ou différents, en C₁-C₁₀, linéaires ou ramifiés, éventuellement porteurs d'un ou plusieurs groupements hydroxyle ou alcoxy, en C₁-C₈, linéaires ou ramifiés ;
* un hétérocycle à 5 ou 6 chaînons, comprenant un ou plusieurs hétéroatomes comme l'oxygène, l'azote, éventuellement substitué, éventuellement porteur d'au moins une charge cationique, de préférence un radical pyridinium, quinolinium ;
* un radical phényle éventuellement substitué par au moins :
   un groupement ammonium substitué par un, deux ou trois radicaux alkyle identiques ou différents, en C₁-C₈, linéaires ou ramifiés, éventuellement porteurs d'un
      ou plusieurs groupements hydroxyle ou alcoxy, en C₁-C₈ linéaires ou ramifiés ;
   un atome de chlore,
   un groupement nitro,
   un groupement hydroxyle,
   un radical alcoxy en C₁-C₈, linéaire ou ramifié ;
   un groupement -CONH₂,
   un groupement -CO-NHR avec R représentant un radical alkyle en C₁-C₈, linéaire ou ramifié ;
   un hétérocycle à 5 ou 6 chaînons, comprenant un ou plusieurs hétéroatomes comme l'oxygène, l'azote, éventuellement substitué, éventuellement porteur d'au moins une charge cationique.

En ce qui concerne les radicaux R₂₉, ces derniers, identiques ou non, représentent plus particulièrement :
* un radical pyridinium rattaché de préférence au moyen de l'un des atomes de carbone de l'hétérocycle ;
* un radical quinolinium éventuellement substitué ;
* un radical phényle éventuellement substitué par au moins :
   un groupement ammonium substitué par un, deux ou trois radicaux alkyle identiques ou différents, en C₁-C₈, linéaires ou ramifiés, éventuellement porteurs d'un
      ou plusieurs groupements hydroxyle ou alcoxy, en C₁-C₈ linéaires ou ramifiés ;
   un atome de chlore,
   un groupement nitro,
   un groupement hydroxyle,
   un radical alcoxy en C₁-C₈, linéaire ou ramifié ;
   un groupement -CONH₂,
   un groupement -CO-NHR avec R représentant un radical alkyle en C₁-C₈, linéaire ou ramifié ;
   un hétérocycle à 5 ou 6 chaînons, comprenant un ou plusieurs hétéroatomes comme l'oxygène, l'azote, éventuellement substitué, éventuellement porteur d'au moins une charge cationique.

En ce qui concerne la synthèse des composés de formule (1) (2) on pourra se référer au brevet US 6 087 493, à la demande de brevet WO 00/74674, ainsi qu'à l'ouvrage « The Porphyrin Handbook », Karl M. KADISH, Kevin M. SMITH, Roger GUILARD ; vol.19, p 105, Edition 2003, la publication « Cis-Pyridyl Core-Modified Porphyrins for the Synthesis of Cationic Water-Soluble Porphyrins and Unsymetrical Non-Covalent Porphyrin Arrays », Sangita SANTRA, Duraisamy KUMARESAN, Neeraj AGARWAL, D. Mangalampalli RAVIKANTH, Tetrahedron (2003) 2353-2362.

Par ailleurs, dans les deux formules ci-dessus, l'anion cosmétiquement acceptable est choisi de préférence parmi les chlorures, les bromures, les iodures, les phosphates, les sulfates, le méthyl sulfate, l'éthyl sulfate, les tosylates, les benzènesulfonates, les citrates, les succinates, les tartrates, les lactates et les acétates.

Habituellement, la teneur en composé(s) cationique(s) non métallique(s) tels que décrits ci-dessus, dans une composition tinctoriale, représente de 0,005 à 20 % en poids par rapport au poids de cette composition, plus particulièrement de 0,05 à 10 % en poids et de préférence de 0,1 à 8 % en poids.

Le milieu de ces compositions tinctoriales, approprié pour la teinture de matières kératiniques, est généralement constitué par de l'eau ou par un mélange d'eau et d'un ou plusieurs solvants organiques usuels.

Plus particulièrement, les solvants organiques, s'ils sont présents, sont choisis parmi les monoalcools ou les polyols, linéaires ou ramifiés, de préférence saturés, comprenant 2 à 10 atomes de carbone, tels que l'alcool éthylique, l'alcool isopropylique, l'hexylèneglycol (2-méthyl 2,4-pentanediol), le néopentylglycol et le 3-méthyl-1,5-pentanediol ; les alcools aromatiques tels que l'alcool benzylique, l'alcool phényléthylique ; les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ; ainsi que les alkyléthers de diéthylèneglycol, notamment en C₁-C₄, comme par exemple, le monoéthyléther ou le monobutyléther du diéthylèneglycol, seuls ou en mélange.

Les solvants décrits ci-dessus, s'ils sont présents, représentent habituellement de 1 à 40 % en poids, plus préférentiellement de 5 à 30 % en poids, par rapport au poids total de la composition tinctoriale.

La composition tinctoriale peut de même comprendre au moins un colorant direct additionnel différent du composé cationique non métallique de type porphyrine et phtalocyanine.

Plus particulièrement, le ou les colorants directs additionnels sont de nature non ionique, cationique ou anionique.

A titre d'exemples non limitatifs, on peut citer les colorants benzéniques nitrés, les colorants azoïques, azométhiniques, méthiniques, tétraazapenthaméthiniques, anthraquinoniques, naphtoquinoniques, benzoquinoniques, phénotiaziniques indigoïdes, xanthéniques, phénanthridiniques, phtalocyanines, ceux dérivés du triarylméthane et les colorants naturels, seuls ou en mélanges.

Il peut par exemple être choisi parmi les colorants benzéniques nitrés rouges ou orangés suivants :
- le 1-hydroxy-3-nitro-4-N-(γ-hydroxypropyl)amino benzène,
- le N-(β-hydroxyéthyl)amino-3-nitro-4-amino benzène,
- le 1-amino-3-méthyl-4-N-(β-hydroxyéthyl)amino-6-nitro benzène,
- le 1-hydroxy-3-nitro-4-N-(β-hydroxyéthyl)amino benzène,
- le 1,4-diamino-2-nitrobenzène,
- le 1-amino-2-nitro-4-méthylamino benzène,
- la N-(β-hydroxyéthyl)-2-nitro-paraphénylènediamine,
- le 1-amino-2-nitro-4-(β-hydroxyéthyl)amino-5-chloro benzène,
- la 2-nitro-4-amino-diphénylamine,
- le1-amino-3-nitro-6-hydroxybenzène.
- le 1-(β-aminoéthyl)amino-2-nitro-4-(β-hydroxyéthyloxy) benzène,
- le 1-(β, γ-dihydroxypropyl)oxy-3-nitro-4-(β-hydroxyéthyl)amino benzène,
- le 1-hydroxy-3-nitro-4-aminobenzène,
- le 1-hydroxy-2-amino-4,6-dinitrobenzène,
- le 1-méthoxy-3-nitro-4-(β-hydroxyéthyl)amino benzène,
- la 2-nitro-4'-hydroxydiphénylamine,
- le 1-amino-2-nitro-4-hydroxy-5-méthylbenzène.

Le colorant direct additionnel peut aussi être choisi parmi les colorants directs benzéniques nitrés jaunes et jaune-verts, on peut par exemple citer les composés choisis parmi:
- le 1-β-hydroxyéthyloxy-3-méthylamino-4-nitrobenzène,
- le 1-méthylamino-2-nitro-5-(β,γ-dihydroxypropyl)oxy benzène,
- le 1-(β-hydroxyéthyl)amino-2-méthoxy-4-nitrobenzène,
- le 1-(β-aminoéthyl)amino-2-nitro-5-méthoxy-benzène,
- le 1,3-di(β-hydroxyéthyl)amino-4-nitro-6-chlorobenzène,
- le 1-amino-2-nitro-6-méthyl-benzène,
- le 1-(β-hydroxyéthyl)amino-2-hydroxy-4-nitrobenzène,
- la N-(β-hydroxyéthyl)-2-nitro-4-trifluorométhylaniline,
- l'acide 4-(β-hydroxyéthyl)amino-3-nitro-benzènesulfonique,
- l'acide 4-éthylamino-3-nitro-benzoïque,
- le 4-(β-hydroxyéthyl)amino-3-nitro-chlorobenzène,
- le 4-(β-hydroxyéthyl)amino-3-nitro-méthylbenzène,
- le 4-(β,γ-dihydroxypropyl)amino-3-nitro-trifluorométhylbenzène,
- le 1-(β-uréidoéthyl)amino-4-nitrobenzène,
- le 1,3-diamino-4-nitrobenzène,
- le 1-hydroxy-2-amino-5-nitrobenzène,
- le 1-amino-2-[tris(hydroxyméthyl)méthyl]amino-5-nitro-benzène,
- le 1-(β-hydroxyéthyl)amino-2-nitrobenzène,
- le 4-(β-hydroxyéthyl)amino-3-nitrobenzamide.

On peut aussi mentionner les colorants directs benzéniques nitrés bleus ou violets, comme par exemple :
- le 1-(β-hydroxyéthyl)amino-4-N,N-bis-(β-hydroxyéthyl)amino 2-nitrobenzène,
- le 1-(γ-hydroxypropyl)amino 4-N,N-bis-(β-hydroxyéthyl)amino 2-nitrobenzène,
- le 1-(β-hydroxyéthyl)amino 4-(N-méthyl, N-β-hydroxyéthyl)amino 2-nitrobenzène,
- le 1-(β-hydroxyéthyl)amino 4-(N-éthyl, N-β-hydroxyéthyl)amino 2-nitrobenzène,
- le 1-(β,γ-dihydroxypropyl)amino 4-(N-éthyl, N-β-hydroxyéthyl)amino 2-nitrobenzène,
- les 2-nitroparaphénylènediamines de formule suivante : 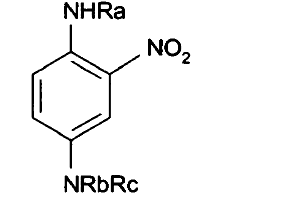
dans laquelle :
- Rb représente un radical alkyle en C₁-C₄, un radical β-hydroxyéthyle ou β-hydroxypropyle ou γ-hydroxypropyle ;
- Ra et Rc, identiques ou différents, représentent un radical β-hydroxyéthyle, -β-hydroxypropyle, γ-hydroxypropyle, ou β,γ-dihydroxypropyle, l'un au moins des radicaux Rb, Rc ou Ra représentant un radical γ-hydroxypropyle et Rb et Rc ne pouvant désigner simultanément un radical β-hydroxyéthyle lorsque Rb est un radical γ-hydroxypropyle, telles que celles décrits dans le brevet français FR 2 692 572.

Parmi les colorants directs azoïques utilisables selon l'invention on peut citer les colorants azoïques cationiques décrits dans les demandes de brevets WO 95/15144, WO 95/01772 et EP 714954, FR 2 822 696, FR 2 825 702, FR 2 825 625, FR 2 822 698, FR 2 822 693, FR 2 822 694, FR 2 829 926, FR 2 807 650, WO 02/078660, WO 02/100834, WO 02/100369, FR 2 844 269.

Parmi ces composés on peut tout particulièrement citer les colorants suivants :
- chlorure de 1,3-diméthyl-2-[[4-(diméthylamino)phényl]azo]-1 H-Imidazolium,
- chlorure de 1 ,3-diméthyl-2-[(4-aminophényl)azo]-1 H-lmidazolium,
- méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3e édition :
- Disperse Red 17
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques on peut citer les colorants suivants :
- Disperse Red 15
- Solvent Violet 13
- Acid Violet 43
- Disperse Violet 1
- Disperse Violet 4
- Disperse Blue 1
- Disperse Violet 8
- Disperse Blue 3
- Disperse Red 11
- Acid Blue 62
- Disperse Blue 7
- Basic Blue 22
- Disperse Violet 15
- Basic Blue 99
   ainsi que les composés suivants :

- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques on peut citer les composés suivants :
- Basic Blue 17
- Basic Red 2.

Parmi les colorants directs méthiniques cationiques, on peut aussi citer le Basic Red 14, le Basic Yellow 13 et le Basic Yellow 29.

Parmi les colorants triarylméthaniques utilisables selon l'invention, on peut citer les composés suivants :
- Basic Green 1
- Acid blue 9
- Basic Violet 3
- Basic Violet 14
- Basic Blue 7
- Acid Violet 49
- Basic Blue 26
- Acid Blue 7

Parmi les colorants indoaminiques utilisables selon l'invention, on peut citer les composés suivants :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine.

La composition peut aussi comprendre des colorants directs naturels comme la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

La teneur en colorants directs additionnels représente avantageusement de 0,0005 à 12 % en poids par rapport au poids de la composition, et de préférence de 0,005 à 6 % en poids par rapport au poids de la composition.

La composition selon l'invention peut de même comprendre au moins une base d'oxydation choisie parmi les bases d'oxydation classiquement utilisées pour la coloration d'oxydation et parmi lesquelles on peut notamment citer les paraphénylènediamines, les bis-phénylalkylènediamines, les para-aminophénols, les ortho-aminophénols et les bases hétérocycliques et leurs sels d'addition avec un acide ou avec un agent alcalin.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N.N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylène diamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylène diamine, la N-(β-méthoxyéthyl) paraphénylènediamine et la 4'aminophényl 1-(3-hydroxy)pyrrolidine, et leurs sels d'addition avec un acide ou avec un agent alcalin.

Parmi les paraphénylènediamines citées ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide ou avec un agent alcalin.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-amino-phényl) tétraméthylènediamine, la N,N'-bis-(4-méthyl-aminophényl) tétraméthylène diamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide ou avec un agent alcalin.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide ou avec un agent alcalin.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide ou avec un agent alcalin.

Parmi les bases hétérocycliques, on peut plus particulièrement citer à titre d'exemple, les dérivés pyridiniques, les dérivés pyrimidiniques et les dérivés pyrazoliques et leurs sels d'addition avec un acide ou avec un agent alcalin.

Lorsqu'elles sont utilisées, la ou les bases d'oxydation représentent avantageusement de 0,0005 à 12 % en poids par rapport au poids total de la composition, et de préférence de 0,005 à 6 % en poids.

La composition peut également comprendre, associé à au moins une base d'oxydation, au moins un coupleur, de façon à modifier ou à enrichir en reflets les nuances obtenues.

Le ou les coupleurs utilisables peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques et leurs sels d'addition avec un acide ou avec un agent alcalin.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, et leurs sels d'addition avec un acide ou avec un agent alcalin.

Lorsqu'ils sont présents, le ou les coupleurs représentent plus particulièrement de 0,0001 à 10 % en poids, et de préférence de 0,005 à 5 % en poids, par rapport au poids total de la composition.

D'une manière générale, les sels d'addition avec un acide sont notamment choisis parmi les chlorhydrates, les bromhydrates, les sulfates, les citrates, les succinates, les tartrates, les tosylates, les benzènesulfonates, les lactates et les acétates.

La composition tinctoriale selon l'invention peut également comprendre un ou plusieurs polymères et/ou agents tensioactifs.

Lorsque la composition comprend au moins un polymère, celui-ci est choisi de préférence parmi les polymères épaississants associatifs ou non, parmi les polymères conditionneurs ou parmi les polymères fixants.

II est précisé que les polymères associatifs sont des polymères hydrophiles capables, dans un milieu aqueux, de s'associer réversiblement entre eux ou avec d'autres molécules.

Leur structure chimique comprend plus particulièrement au moins une zone hydrophile et au moins une zone hydrophobe.

Les polymères associatifs présents dans la composition selon l'invention peuvent être de nature non ionique, anionique, cationique ou amphotère.

Parmi les dérivés de polyuréthanes associatifs, on peut citer les copolymères anioniques obtenus par polymérisation :
- environ 20% à 70% en poids d'un acide carboxylique à insaturation α,β-monoéthylénique,
- environ 20 à 80% en poids d'un monomère à insaturation α,β-monoéthylénique non-tensioactif différent du précédent,
- environ 0,5 à 60% en poids d'un mono-uréthane non-ionique qui est le produit de réaction d'un tensioactif monohydroxylé avec un monoisocyanate à insaturation monoéthylénique.

De tels sont notamment décrits dans EP 173109 et plus particulièrement dans l'exemple 3. Plus précisément, ce polymère est un terpolymère acide méthacrylique /acrylate de méthyle / diméthyl métaisopropényl benzyl isocyanate d'alcool béhényle éthoxylé (40OE) en dispersion aqueuse à 25%.Ce produit est proposé sous la référence VISCOPHOBE DB1000 par la Société AMERCHOL.

Conviennent aussi citer les polyuréthanes associatifs cationiques dont la famille a été décrite dans la demande FR 0009609.

Les dérivés de polyuréthanes associatifs de l'invention peuvent être aussi des polyuréthanes polyéthers non ioniques. Plus particulièrement, lesdits polymères comportent dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

De préférence, ces polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.
Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.
Les polyéthers polyuréthanes non ioniques à chaîne grasse peuvent être des copolymères triblocs dont la séquence hydrophile est une chaîne polyoxyéthylénée comportant de 50 à 1000 groupements oxyéthylénés.

A titre d'exemples de polyéthers polyuréthanes non ioniques à chaîne hydrophobe utilisables dans l'invention, on peut utiliser aussi le Rhéolate 205® à fonction urée vendu par la société RHEOX ou encore les Rhéolates® 208 , 204 ou 212, ainsi que l'Acrysol RM 184®.

On peut également citer le produit ELFACOS T210® à chaîne alkyle en C₁₂-C₁₄ et le produit ELFACOS T212® à chaîne alkyle en C₁₈ de chez AKZO.
Le produit DW 1206B® de chez ROHM & HAAS à chaîne alkyle en C₂₀ et à liaison uréthane, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemples, de tels polymères on peut citer, le Rhéolate® 255, le Rhéolate® 278 et le Rhéolate® 244 vendus par la société RHEOX. On peut aussi utiliser le produit DW 1206F et le DW 1206J proposés par la société ROHM & HAAS.

Les polyéthers polyuréthanes utilisables selon l'invention peuvent aussi être choisis parmi ceux décrits dans l'article de G. Fonnum, J. Bakke et Fk. Hansen - Colloid Polym. Sci 271, 380.389 (1993).

Plus particulièrement encore, on peut utiliser un polyéther polyuréthane susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

De tels polyéther polyuréthanes sont vendus notamment par la société ROHM & HAAS sous les appellations Aculyn 46® et Aculyn 44® [l'ACULYN 46® est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44® est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

Parmi les polymères dérivés de celluloses associatifs utilisables selon l'invention on peut citer :
- les celluloses cationiques quaternisées modifiées par des groupements comportant au moins une chaîne hydrophobe, tels que les groupes alkyle, arylalkyle, alkylaryle comportant au moins 8 atomes de carbone, ou des mélanges de ceux-ci,
- les hydroxyéthylcelluloses cationiques quaternisées modifiées par des groupements comportant au moins une chaîne hydrophobe, tels que les groupes alkyle, arylalkyle, alkylaryle comportant au moins 8 atomes de carbone, ou des mélanges de ceux-ci.
   Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle.
   On peut indiquer comme exemples d'alkylhydroxyéthylcelluloses quaternisées à chaîne hydrophobe en C₈-C₃₀, les produits QUATRISOFT LM 200®, QUATRISOFT LM-X 529-18-A®, QUATRISOFT LM-X 529-18B® (alkyle en C₁₂) et QUATRISOFT LM-X 529-8® (alkyle en C₁₈) commercialisés par la société AMERCHOL et les produits CRODACEL QM®, CRODACEL QL® (alkyle en C₁₂) et CRODACEL QS® (alkyle en C18) commercialisés par la société CRODA.
- les dérivés de cellulose non ioniques tels que les hydroxyéthylcelluloses modifiées par des groupements comportant au moins une chaîne hydrophobe tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS® (alkyles en C₁₆) vendu par la société AQUALON, ou le produit BERMOCOLL EHM 100® vendu par la société BEROL NOBEL,
- les dérivés de cellulose modifiée par des groupes polyalkylène glycol éther d'alkyl phénol, tel que le produit AMERCELL POLYMER HM-1500® vendu par la société AMERCHOL.

En ce qui concerne les polyvinyllactames associatifs utilisables dans le cadre de la présente invention, ces derniers peuvent notamment être choisis parmi ceux décrits dans FR 0101106.

On peut aussi citer les terpolymères comprenant, en poids, 40 à 95% de monomère (a), 0,1 à 55% de monomère (c) et 0,25 à 50% de monomère (b).

De tels polymères sont notamment décrits dans la demande de brevet WO 00/68282 dont le contenu fait partie intégrante de l'invention.

Comme polymères poly(vinyllactame) selon l'invention, on utilise notamment les terpolymères vinylpyrrolidone / diméthylaminopropylméthacrylamide / tosylate de dodécyl diméthylméthacrylamidopropylammonium, les terpolymères vinylpyrrolidone / diméthyl aminopropylméthacrylamide / tosylate de cocoyldiméthylméthacrylamido propyl ammonium, les terpolymères vinylpyrrolidone / diméthylaminopropyl méthacrylamide / tosylate ou chlorure de lauryldiméthylméthacrylamidopropylammonium.

Les dérivés de polyvinyllactames associatifs de l'invention peuvent être aussi des copolymères non ioniques de vinylpyrrolidone et de monomères hydrophobes à chaîne hydrophobe dont on peut citer à titre d'exemple :
- les produits ANTARON V216® ou GANEX V216® (copolymère vinylpyrrolidone /hexadécène) vendu par la société I.S.P.
- les produits ANTARON V220® ou GANEX V220® (copolymère vinylpyrrolidone /eicosène) vendu par la société I.S.P.

Parmi les dérivés de polyacides insaturés associatifs on peut citer ceux comportant au moins un motif hydrophile de type acide carboxylique insaturé oléfinique, de préférence les acides acrylique, méthacrylique, éthacrylique, et au moins un motif hydrophobe de type ester d'alkyl (C10-C30) d'acide carboxylique insaturé.

Des polymères anioniques de ce type sont par exemple décrits et préparés, selon les brevets US-3 915 921 et 4 509 949.

Dans ce type de polymères associatifs anioniques, on utilise plus particulièrement des polymères formés à partir d'un mélange de monomères comprenant :
(i) essentiellement de l'acide acrylique,
(ii) un (méth)acrylate d'alkyle ayant 12 à 22 atomes de carbone,
(iii) et un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

Parmi ce type de polymères associatifs anioniques, on préfère ceux constitués de 95 à 60% en poids d'acide acrylique (motif hydrophile), de 4 à 40% en poids d'acrylate d'alkyles en C₁₀-C₃₀ (motif hydrophobe), et de 0 à 6% en poids de monomère polymérisable réticulant,
ou bien ceux constitués de 98 à 96% en poids d'acide acrylique (motif hydrophile), de 1 à 4% en poids d'acrylate d'alkyles en C₁₀-C₃₀ (motif hydrophobe), et de 0,1 à 0,6% en poids de monomère polymérisable réticulant tel que ceux décrits précédemment.

Parmi lesdits polymères ci-dessus, on préfère tout particulièrement les produits vendus par la société GOODRICH sous les dénominations commerciales PEMULEN TR1®, PEMULEN TR2®, CARBOPOL 1382®, et encore plus préférentiellement le PEMULEN TR1®, et le produit vendu par la société S.E.P.P.I.C. sous la dénomination COATEX SX®.

Parmi les dérivés de polyacides insaturés associatifs on peut aussi citer ceux comportant parmi leurs monomères un acide carboxylique à insaturation α,β-monoéthylénique et un ester d'acide carboxylique à insaturation α,β-monoéthylénique et d'un alcool gras oxyalkyléné.

Préférentiellement ces composés comprennent également comme monomère un ester d'acide carboxylique à insaturation α,β-monoéthylénique et d'alcool en C1-C4.

A titre d'exemples de ce type de composés, on peut citer l'ACULYN 22® vendu par la société ROHM et HAAS, qui est un terpolymère acide méthacrylique/acrylate d'éthyle/méthacrylate de stéaryle oxyalkyléné.

En ce qui concerne les polymères épaississants non associatifs, ces derniers ne contiennent pas de chaîne grasse en C10-C30.

Parmi les polymères épaississants non associatifs, on peut citer les homopolymères d'acide acrylique réticulés, par exemple par un éther allylique d'alcool de la série du sucre, comme par exemple les produits vendus sous les noms CARBOPOLS 980, 981, 954, 2984 et 5984 par la société NOVEON ou les produits vendus sous les noms SYNTHALEN M et SYNTHALEN K par la société 3 VSA.

On peut aussi citer, parmi les polymères épaississants non associatifs, les homopolymères réticulés d'acide 2-acrylamido-2-méthyl-propane sulfonique et leurs copolymères réticulés d'acrylamide partiellement ou totalement neutralisés. Conviennent notamment, les homopolymères décrits dans la demande EP 815 828 à laquelle on pourra se reporter à ce sujet. Parmi les copolymères réticulés d'acide 2-acrylamido-2-méthylpropane sulfonique et d'acrylamide partiellement ou totalement neutralisés, on peut citer en particulier le produit décrit dans l'exemple 1 du document EP 503 853 et l'on pourra se reporter à ce document pour ce qui a trait à ces polymères. Il est à noter que dans le cas où les composés sont neutralisés, ils le sont notamment par l'emploi d'une base telle que l'hydroxyde de sodium ou de potassium ou une amine.

Conviennent aussi, en tant que polymères épaississants non associatifs, les homopolymères d'acrylate d'ammonium ou les copolymères d'acrylate d'ammonium et d'acrylamide.

A titre d'exemples d'homopolymères d'acrylate d'ammonium, on peut citer le produit vendu sous le nom MICROSAP PAS 5193 par la société HOECHST. Parmi les copolymères d'acrylate d'ammonium et d'acrylamide, on peut citer le produit vendu sous le nom BOZEPOL C NOUVEAU ou le produit PAS 5193 vendus par la société HOECHST. On pourra notamment se référer aux documents FR 2 416 723, US 2798053 et US 2923692 pour ce qui a trait à la description et à la préparation de tels composés.

Les homopolymères de diméthylaminoéthyl-méthacrylate quaternisé par le chlorure de méthyle ou les copolymères de diméthylamino-éthylméthacrylate quaternisé par le chlorure de méthyle et d'acrylamide peuvent aussi être utilisés comme polymères épaississants non associatifs.

Parmi les homopolymères de cette famille, on peut citer les produits vendus sous les noms SALCARE 95 et SALCARE 96 par la société CIBA-ALLIED COLLOIDS. Parmi les copolymères de cette famille, on peut citer le produit SALCARE SC92 vendu par CIBA-ALLIED COLLOIDS ou le produit PAS 5194 vendu par HOECHST. Ces polymères sont notamment décrits et préparés dans le document EP 395282 auquel on pourra se référer.

Conviennent également les gommes de guar non ioniques non modifiées telles que celles vendues sous la dénomination VIDOGUM GH 175 par la société UNIPECTINE et sous la dénomination JAGUAR C par la société MEYHALL.

Il est de même possible d'utiliser des gommes de guar non ioniques modifiées par des groupements hydroxyalkyle en C₁-C₆ (notamment hydroxyméthyle, hydroxyéthyle, hydroxypropyle et hydroxybutyle).

De telles gommes de guar non ioniques modifiées par des groupements hydroxyalkyle sont par exemple vendues sous les dénominations commerciales JAGUAR HP8, JAGUAR HP60 et JAGUAR HP120, JAGUAR DC 293 et JAGUAR HP 105 par la société MEYHALL ou sous la dénomination GALACTASOL 4H4FD2 par la société AQUALON.

Conviennent aussi les gommes de biopolysaccharides d'origine microbienne, comme par exemple les gommes de scléroglucane ou de xanthane ; les gommes issues d'exudats végétaux telles que les gommes arabiques, gommes Ghatti, gommes Karaya et Tragacanthe ; les hydroxypropyl ou carboxyméthyl celluloses ; les pectines et les alginates. De tels composés sont notamment décrits dans l'ouvrage de Robert L. DAVIDSON intitulé "Handbook of Water soluble gums and resins" édité chez Mc Graw Hill Book Company (1980).

La concentration en polymère(s) épaississant(s) associatif(s) ou non, présent(s) dans la composition tinctoriale peut varier de 0,01 à 10% en poids, plus particulièrement de 0,1 à 5% en poids, par rapport au poids de la composition tinctoriale.

Comme indiqué auparavant, la composition selon l'invention peut comprendre au moins un polymère conditionneur ou un polymère fixant.

Il est tout d'abord rappelé que l'on entend par agent conditionneur tout agent ayant pour fonction l'amélioration des propriétés cosmétiques des matières kératiniques telles que les cheveux, en particulier la douceur, le démêlage, le toucher, le lissage, l'électricité statique.

Les polymères conditionneurs convenables sont choisis parmi les polymères cationiques et les polyorganosiloxanes cationiques ou non ioniques.

Par polymère cationique, on entend tout polymère contenant des groupes cationiques et/ou des groupes ionisables en groupes cationiques.

Ces polymères cationiques sont choisis parmi tous ceux déjà connus en tant que tels pour leur capacité d'améliorer les propriétés cosmétiques des cheveux traités par des compositions détergentes. On peut citer notamment ceux décrits dans les demandes de brevet EP 0 337 354, FR 2 270 846, FR 2 383 660, FR 2 598 611, FR 2 470 596 et FR 2 519 863.

Les polymères cationiques préférés sont choisis parmi ceux qui contiennent des motifs comportant des groupements amines primaires, secondaires, tertiaires et/ou quaternaires qui font partie de la chaîne macromoléculaire principale, ou bien sont portés par des groupes latéraux directement reliés à celle-ci.

Parmi les polymères cationiques, on peut citer plus particulièrement les polymères du type polyamine, polyaminoamide et polyammonium quaternaire. II s'agit de produits connus.

Les polymères du type polyamine, polyaminoamide et polyammonium quaternaire, que l'on peut utiliser dans les compositions de la présente invention, sont ceux décrits dans les brevets FR 2 505 348 et FR 2 542 997. Parmi ces polymères, on peut citer en particulier :
**(1)** les homopolymères ou copolymères dérivés d'esters ou d'amides acryliques ou méthacryliques et comportant au moins un des motifs de formules suivantes: 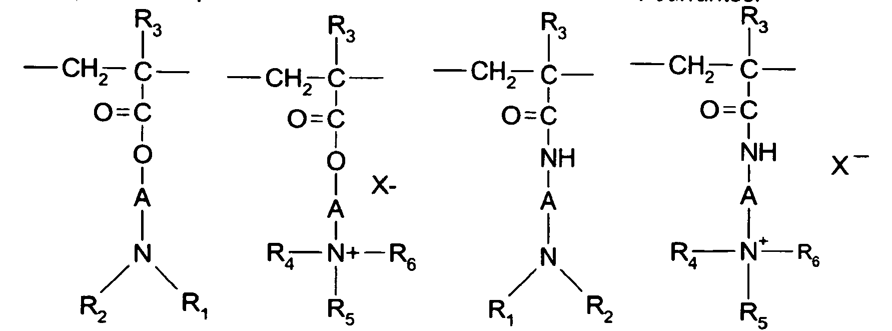 dans lesquelles:
   R₃, identiques ou différents, désignent un atome d'hydrogène ou un groupe CH₃ ;
   A, identiques ou différents, représentent un groupe alkyle, linéaire ou ramifié, comportant de 1 à 6 atomes de carbone, de préférence 2 ou 3 atomes de carbone, ou un groupe hydroxyalkyle comportant de 1 à 4 atomes de carbone ;
   R₄, R₅, R₆, identiques ou différents, représentent un groupe alkyle ayant de 1 à 18 atomes de carbone ou un groupe benzyle, et de préférence un groupe alkyle ayant de 1 à 6 atomes de carbone;
   R₁ et R₂, identiques ou différents, représentent un atome d'hydrogène ou un groupe alkyle ayant de 1 à 6 atomes de carbone, et de préférence un groupe méthyle ou éthyle;
   X- désigne un anion dérivé d'un acide minéral ou organique tel qu'un anion méthosulfate ou un halogénure comme le chlorure ou le bromure.

   Les copolymères de la famille **(1)** peuvent contenir en outre un ou plusieurs motifs dérivant de comonomères choisis dans la famille des acrylamides, méthacrylamides, diacétone-acrylamides, acrylamides et méthacrylamides substitués sur l'atome d'azote par des groupes alkyle inférieur en C₁-C₄, des groupes dérivés des acides acryliques ou méthacryliques ou de leurs esters, de vinyllactames tels que la vinylpyrrolidone ou le vinylcaprolactame, d'esters vinyliques.
   Parmi ces copolymères de la famille **(1)**, on peut citer en particulier :
   - les copolymères d'acrylamide et de méthacrylate de diméthylaminoéthyle quaternisé au sulfate de diméthyle ou avec un halogénure de diméthyle, tels que celui vendu sous la dénomination HERCOFLOC^{®} par la société HERCULES,
   - les copolymères d'acrylamide et de chlorure de méthacryloyloxyéthyltriméthylammonium décrits, par exemple, dans la demande de brevet EP-A-080976 et vendus sous la dénomination BINAQUAT^{®} P 100 par la société CIBA GEIGY,
   - le copolymère d'acrylamide et de méthosulfate de méthacryloyloxyéthyltriméthylammonium vendu sous la dénomination RETEN^{®} par la société HERCULES,
   - les copolymères vinylpyrrolidone/acrylate ou méthacrylate de dialkylaminoalkyle quaternisés ou non, tels que les produits vendus sous la dénomination GAFQUAT^{®} par la société ISP comme, par exemple, GAFQUAT^{®} 734 ou GAFQUAT^{®} 755, ou bien les produits dénommés COPOLYMER 845, 958 et 937. Ces polymères sont décrits en détail dans les brevets FR 2 077 143 et FR 2 393 573,
   - les terpolymères méthacrylate de diméthylaminoéthyle/ vinylcaprolactame /vinylpyrrolidone tels que le produit vendu sous la dénomination GAFFIX^{®} VC 713 par la société ISP,
   - les copolymères vinylpyrrolidone/méthacrylamidopropyl-di-méthylamine commercialisés notamment sous la dénomination STYLEZE^{®} CC 10 par ISP, et
   - les copolymères vinylpyrrolidone/méthacrylamide de diméthylaminopropyle quaternisé, tels que le produit vendu sous la dénomination GAFQUAT^{®} HS 100 par la société ISP.
**(2)** Les dérivés d'éthers de cellulose comportant des groupements ammonium quaternaires décrits dans le brevet FR 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammoniums quaternaires d'hydroxyéthylcellulose ayant réagi avec un époxyde substitué par un groupement triméthylammonium.
**(3)** Les dérivés cationiques de la cellulose tels que les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl-, hydroxyéthyl- ou hydroxypropylcelluloses greffées notamment avec un sel de méthacryloyléthyl-triméthylammonium, de méthacrylamidopropyl-triméthylammonium, de diméthyldiallylammonium.
   Les produits commercialisés répondant à cette définition sont plus particulièrement les produits vendus sous la dénomination Celquat^{®} L 200 et Celquat^{®} H 100 par la Société National Starch.
**(4)** Les polysaccharides cationiques décrits plus particulièrement dans les brevets US 3 589 578 et US 4 031 307, tels que les gommes de guar contenant des groupements cationiques trialkylammonium. On utilise, par exemple, des gommes de guar modifiées par un sel, par exemple le chlorure, de 2,3-époxypropyltriméthylammonium.
   De tels produits sont commercialisés notamment sous les dénominations commerciales de JAGUAR^{®} C13 S, JAGUAR^{®} C 15, JAGUAR^{®} C 17 ou JAGUAR^{®} C162 par la société MEYHALL.
**(5)** Les polymères constitués de motifs pipérazinyle et de groupes divalents alkylène
   ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets FR 2 162 025 et FR 2 280 361.
**(6)** Les polyaminoamides solubles dans l'eau, préparés en particulier par polycondensation d'un composé acide avec une polyamine. Ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épihalohydrine, d'un diépoxyde ou d'un dérivé bis-insaturé, l'agent réticulant étant utilisé dans des proportions allant de 0,025 à 0,35 mole par groupement amine du polyaminoamide. Ces polyaminoamides peuvent être alkylés ou, s'ils comportent une ou plusieurs fonctions amines tertiaires, quaternisées. De tels polymères sont notamment décrits dans les brevets FR 2 252 840 et FR 2 368 508.
**(7)** Les dérivés de polyaminoamides résultant de la condensation de polyalkylènes-polyamines avec des acides polycarboxyliques, suivie d'une alkylation par des agents bifonctionnels. On peut citer, par exemple, les polymères acide adipique/diakylaminohydroxyalkyl-dialkylènetriamine dans lesquels le groupe alkyle comporte de 1 à 4 atomes de carbone et désigne de préférence un groupe méthyle, éthyle
   ou propyle, et le groupe alkylène comporte de 1 à 4 atomes de carbone, et désigne de préférence le groupe éthylène. De tels polymères sont notamment décrits dans le brevet FR 1 583 363.
   Parmi ces dérivés, on peut citer plus particulièrement les polymères acide adipique/diméthylaminohydroxypropyl-diéthylène-triamine vendus sous la dénomination Cartaretine^{®} F, F4 ou F8 par la société Sandoz.
**(8)** Les polymères obtenus par réaction d'une polyalkylène-polyamine comportant deux groupements amine primaire et au moins un groupement amine secondaire, avec un acide dicarboxylique choisi parmi l'acide diglycolique et les acides dicarboxyliques aliphatiques saturés ayant de 3 à 8 atomes de carbone, le rapport molaire de la polyalkylène-polylamine à l'acide dicarboxylique étant compris entre 0,8:1 et 1,4:1. Le polyaminoamide résultant de cette réaction est ensuite amené à réagir avec l'épichlorhydrine dans un rapport molaire d'épichlorhydrine par rapport au groupement amine secondaire du polyaminoamide compris entre 0,5:1 et 1,8:1. De tels polymères sont notamment décrits dans les brevets US 3 227 615 et US 2 961 347.
   Des polymères de ce type sont en particulier commercialisés sous la dénomination Hercosett^{®} 57 par la société Hercules Inc. ou bien sous la dénomination de PD 170 ou Delsette^{®} 101 par la société Hercules dans le cas du copolymère d'acide adipique/époxypropyl-diéthylène-triamine.
**(9)** Les cyclopolymères d'alkyldiallylamine ou de dialkyldiallylammonium tels que les homopolymères ou copolymères comportant, comme constituant principal de la chaîne, des motifs répondant aux formules (Va) ou (Vb) : 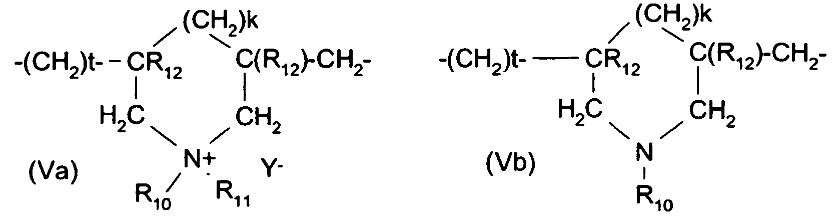 dans lesquelles
   k et t sont égaux à 0 ou 1, la somme k + t étant égale à 1 ;
   R₁₂ désigne un atome d'hydrogène ou un groupe méthyle ;
   R₁₀ et R₁₁, indépendamment l'un de l'autre, désignent un groupement alkyle ayant de 1 à 22 atomes de carbone, un groupement hydroxyalkyle en C₁₋₅, un groupement amidoalkyle inférieur en C₁-C₄, ou bien R₁₀ et R₁₁ peuvent désigner conjointement avec l'atome d'azote auquel ils sont rattachés, des groupements hétérocycliques, tels que pipéridinyle ou morpholinyle ;
   Y⁻ est un anion tel que bromure, chlorure, acétate, borate, citrate, tartrate, bisulfate, bisulfite, sulfate, phosphate.
   Ces polymères sont notamment décrits dans le brevet FR 2 080 759 et dans son certificat d'addition 2 190 406.
   Parmi les polymères définis ci-dessus, on peut citer plus particulièrement l'homopolymère de chlorure de diméthyldiallyl-ammonium vendu sous la dénomination MERQUAT^{®} 100 par la société CALGON (et ses homologues de faibles masses moléculaires moyenne en poids) et les copolymères de chlorure de diallyldiméthylammonium et d'acrylamide commercialisés sous la dénomination MERQUAT^{®} 550.
**(10)** Les polymères de diammonium quaternaire contenant des motifs récurrents répondant à la formule (VI) : 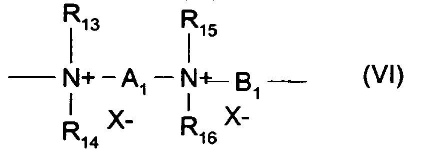 dans laquelle :
   R₁₃, R₁₄, R₁₅ et R₁₆, identiques ou différents, représentent des groupes aliphatiques, alicycliques ou arylaliphatiques contenant de 1 à 20 atomes de carbone ou des groupes hydroxyalkyle aliphatiques inférieurs, ou bien R₁₃, R₁₄, R₁₅ et R₁₆, ensemble ou séparément, forment avec les atomes d'azote auxquels ils sont rattachés des hétérocycles contenant éventuellement un second hétéroatome autre que l'azote, ou bien R₁₃, R₁₄, R₁₅ et R₁₆ représentent un groupe alkyle en C₁₋₆, linéaire ou ramifié, substitué par un groupement nitrile, ester, acyle, amide ou -CO-O-R₁₇-D ou -CO-NH-R₁₇D où R₁₇ est un groupe alkylène et D un groupement ammonium quaternaire ;
   A₁ et B₁ représentent des groupements polyméthyléniques contenant de 2 à 20 atomes de carbone, pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X- désigne un anion dérivé d'un acide minéral ou organique;
   A₁, R₁₃ et R₁₅ peuvent former avec les deux atomes d'azote auxquels ils sont rattachés un cycle pipérazinique ; en outre, si A₁ désigne un groupe alkylène ou hydroxyalkylène linéaire
      ou ramifié, saturé ou insaturé, B₁ peut également désigner un groupement :

      -(CH₂)ₙ-CO-D-OC-(CH₂)ₙ-

      dans lequel D désigne :
      a) un reste de glycol de formule -O-Z-O-, où Z désigne un groupe hydrocarboné linéaire ou ramifié, ou un groupement répondant à l'une des formules suivantes :

         -(CH₂-CH₂-O)ₓ-CH₂-CH₂-

         -[CH₂-CH(CH₃)-O]_{y}-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule -NH-Y-NH-, où Y désigne un groupe hydrocarboné linéaire ou ramifié, ou bien le groupe divalent -CH₂-CH₂-S-S-CH₂-CH₂- ;
      d) un groupement uréylène de formule -NH-CO-NH- .

   De préférence, X⁻ est un anion tel que le chlorure ou le bromure.
   Ces polymères ont une masse moléculaire moyenne en nombre généralement comprise entre 1000 et 100000.
   Des polymères de ce type sont notamment décrits dans les brevets FR 2 320 330, FR 2 270 846, FR 2 316 271, FR 2 336 434 et FR 2 413 907 et les brevets US 2 273 780, US 2 375 853, US 2 388 614, US 2 454 547, US 3 206 462, US 2 261 002, US 2 271 378, US 3 874 870, US 4 001 432, US 3 929 990, US 3 966 904, US 4 005 193, US 4 025 617, US 4 025 627, US 4 025 653, US 4 026 945 et US 4 027 020.
   On peut utiliser plus particulièrement les polymères qui sont constitués de motifs récurrents répondant à la formule : 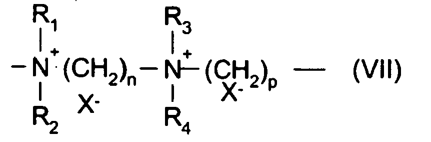 dans laquelle R₁, R₂, R₃ et R₄, identiques ou différents, désignent un groupe alkyle ou hydroxyalkyle ayant de 1 à 4 atomes de carbone environ, n et p sont des nombres entiers variant de 2 à 20 environ et, X⁻ est un anion dérivé d'un acide minéral ou organique.
   Un composé de formule (VII) particulièrement préféré est celui pour lequel R₁, R₂, R₃ et R₄ représentent un groupe méthyle et n=3, p=6 et X=Cl, dénommé chlorure d'hexadiméthrine (CTFA).
**(11)** Les polymères de polyammonium quaternaire, constitués de motifs de formule (VIII) : 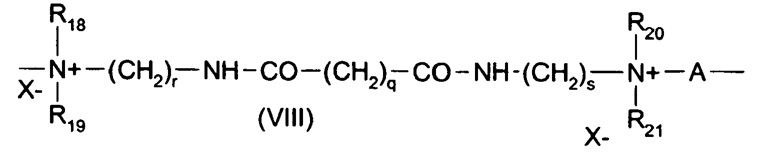 dans laquelle :
   R₁₈, R₁₉, R₂₀ et R_{21,} identiques ou différents, représentent un atome d'hydrogène ou un groupe méthyle, éthyle, propyle, β-hydroxyéthyle, β-hydroxypropyle ou-CH₂CH₂(OCH₂CH₂)ₚOH, où p est égal à 0 ou à un nombre entier compris entre 1 et 6, sous réserve que R₁₈, R₁₉, R₂₀ et R₂₁ ne représentent pas simultanément un atome d'hydrogène, r et s, identiques ou différents, sont des nombres entiers compris entre 1 et 6,
   q est égal à 0 ou à un nombre entier compris entre 1 et 34,
   X⁻ désigne un anion tel qu'un halogénure,
   A désigne un radical d'un dihalogénure ou représente de préférence -CH₂-CH₂-O-CH₂-CH₂-.

   De tels composés sont notamment décrits dans la demande de brevet EP-A-122 324.
   On peut citer parmi ceux-ci, par exemple, les produits Mirapol^{®} A 15, Mirapol^{®} AD1, Mirapol^{®} AZ1 et Mirapol^{®} 175 vendus par la société Miranol.
**(12)** Les polymères quaternaires de vinylpyrrolidone et de vinylimidazole tels que, par exemple, les produits commercialisés sous les dénominations Luviquat^{®} FC 905, FC 550 et FC 370 par la société B.A.S.F. On peut citer notamment les copolymères de vinylpyrrolidone et de chlorure de méthylvinylimidazolium.
**(13)** Les polyamines comme le Polyquart^{®} H vendu par HENKEL, référencées sous le nom de POLYETHYLENEGLYCOL (15) TALLOW POLYAMINE dans le dictionnaire CTFA.
**(14)** Les polymères réticulés ou non réticulés de sels de méthacryloyloxyalkyl(C₁₋₄) trialkyl(C₁₋₄)ammonium, tels que les polymères obtenus par homopolymérisation du méthacrylate de diméthylaminoéthyle quaternisé par le chlorure de méthyle, ou par copolymérisation de l'acrylamide et de méthacrylate de diméthylaminoéthyle quaternisé par le chlorure de méthyle, l'homopolymérisation ou la copolymérisation étant suivie d'une réticulation par un composé à insaturation oléfinique, en particulier le méthylène-bisacrylamide. On peut plus particulièrement utiliser un copolymère réticulé acrylamide/chlorure de méthacryloyloxy-éthyl-triméthylammonium (20/80 en poids) sous forme de dispersion contenant 50 % en poids dudit copolymère dans de l'huile minérale. Cette dispersion est commercialisée sous le nom de SALCARE^{®} SC 92 par la Société ALLIED COLLOIDS. On peut également utiliser un homopolymère réticulé du chlorure de méthacryloyloxyéthyl-triméthylammonium contenant environ 50 % en poids de l'homopolymère dans de l'huile minérale ou dans un ester liquide. Ces dispersions sont commercialisées sous les noms de SALCARE^{®} SC 95 et SALCARE^{®} SC 96 par la Société ALLIED COLLOIDS.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés cationiques de la chitine.

En ce qui concerne les polyorganosiloxanes (ou organosiloxanes ou silicones) aminés
ou non, de préférence non volatiles, peuvent aussi être utilisés comme agent conditionneur. Ils peuvent se présenter en particulier sous forme d'huiles, de cires, de résines ou de gommes.

On peut citer tout particulièrement les polyalkylsiloxanes, les polyarylsiloxanes, les polyalkylarylsiloxanes, les gommes et résines de silicones, les polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de 5.10⁻⁶ à 2,5 m²/s à 25°C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles Silbione^{®} des séries 47 et 70 047 ou les huiles Mirasil^{®} commercialisées par Rhodia Chimie telles que par exemple l'huile 70 047 V 500 000 ;
- les huiles de la série Mirasil commercialisées par la société Rhodia Chimie ;
- les huiles de la série 200 de la société Dow Corning telles que plus particulièrement la DC200 de viscosité 60 000 cSt (mm²/s);
- les huiles Viscasil^{®} de General Electric et certaines huiles des séries SF (SF 96, SF 18) de General Electric.

On peut également citer les polydiméthylsiloxanes à groupements terminaux diméthylsilanol (Dimethiconol selon la dénomination CTFA) tels que les huiles de la série 48 de la société Rhodia Chimie ; mais aussi les produits commercialisés sous les dénominations "Abil^{®} Wax 9800 et 9801" par la société Goldschmidt qui sont des polyalkyl (C₁-C₂₀) siloxanes.

Les polyalkylarylsiloxanes peuvent être plus particulièrement choisis parmi les polydiméthyl méthylphénylsiloxanes, les polydiméthyl diphénylsiloxanes linéaires et/ou ramifiés de viscosité de 1.10⁻⁵ à 5.10⁻²m²/s à 25°C.

Parmi ces polyalkylarylsiloxanes on peut citer à titre d'exemple les produits commercialisés sous les dénominations suivantes :
- les huiles Silbione de la série 70 641 de Rhodia Chimie ;
- les huiles des séries Rhodorsil 70 633 et 763 de Rhodia Chimie ;
- l'huile Dow Corning 556 Cosmetic Grad Fluid de Dow Corning ;
- les silicones de la série PK de Bayer comme le produit PK20 ;
- les silicones des séries PN, PH de Bayer comme les produits PN1000 et PH1000 ;
- certaines huiles des séries SF de General Electric telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants de type polydiméthylsiloxane, les gommes polydiméthylsiloxane / méthylvinylsiloxane, les polydiméthylsiloxane /diphénylsiloxane, les polydiméthylsiloxane / phénylméthylsiloxane, les polydiméthylsiloxane /diphénylsiloxane/méthylvinylsiloxane.

Des produits plus particulièrement utilisables conformément à invention sont des mélanges tels que :
- les mélanges formés à partir d'un polydiméthylsiloxane hydroxylé en bout de chaîne (dénommé diméthiconol selon la nomenclature du dictionnaire CTFA) et d'un polydiméthylsiloxane cyclique (dénommé cyclométhicone selon la nomenclature du dictionnaire CTFA) tel que le produit Q2 1401 commercialisé par la société Dow Corning ;
- les mélanges formés à partir d'une gomme polydiméthylsiloxane avec une silicone cyclique tel que le produit SF 1214 Silicone Fluid de la société General Electric, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société General Electric. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les unités : R₂SiO_{2/2}, R₃SiO_{1/2}, RSiO_{3/2} et SiO_{4/2}
dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "Dow Corning 593" ou ceux commercialisés sous les dénominations "Silicone Fluid SS 4230 et SS 4267" par la société General Electric et qui sont des silicones de structure diméthyl/triméthyl siloxane.

On peut également citer les résines du type triméthylsiloxysilicate commercialisées notamment sous les dénominations X22-4914, X21-5034 et X21-5037 par la société Shin-Etsu.

Les silicones organo-modifiées utilisables conformément à l'invention sont des silicones telles que définies précédemment et comportant dans leur structure un ou plusieurs groupements organofonctionnels fixés par l'intermédiaire d'un radical hydrocarboné. Ces composés organofonctionnels sont différents des groupements aminés.

Parmi les silicones organo-modifiées différentes de celles de formules (I) ou (II), on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société Dow Corning sous la dénomination DC 1248 et l'alkyl (C₁₂) méthicone copolyol commercialisée par la société Dow Corning sous la dénomination Q2 5200 ;
- des groupements thiols, comme les produits commercialisés sous les dénominations "GP 72 A" et "GP 71" de Genesee ;
- des groupements alcoxylés, comme le produit commercialisé sous la dénomination "Silicone Copolymer F-755" par SWS Silicones et Abil Wax 2428, 2434 et 2440 par la société Goldschmidt ;
- des groupements hydroxylés, comme les polyorganosiloxanes à fonction hydroxyalkyle décrits dans FR 8516334 ;
- des groupements acyloxyalkyle, tels que par exemple les polyorganosiloxanes décrits dans US 4957732 ;
- des groupements anioniques du type carboxylique, comme par exemple dans les produits décrits dans EP 186507 de la société Chisso Corporation, ou de type alkylcarboxyliques comme ceux présents dans le produit X-22-3701 E de la société Shin-Etsu ; 2-hydroxyalkylsulfonate ; 2-hydroxyalkylthiosulfate tels que les produits commercialisés par la société Goldschmidt sous les dénominations "Abil S201" et "Abil S255".

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans EP 412704, EP 412707, EP 640105 et WO 95/00578, EP 582152, WO 93/23009, US 4693935, US 4728571 et US 4972037. Ces polymères sont de préférence anioniques ou non ioniques.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

II est à noter que les silicones peuvent également être utilisées sous forme d'émulsions, de nanoémulsions ou de micrémulsions.

Par polymères fixants, on entend au sens de la présente invention tout polymère permettant de donner ou de maintenir une forme à la chevelure.

De préférence, les polymères fixants susceptibles d'être utilisés sont choisis parmi les polymères anioniques, amphotères, non ioniques et leurs mélanges.

Les polymères fixants peuvent être solubles dans le milieu cosmétiquement acceptable
ou insolubles dans ce même milieu et utilisés dans ce cas sous forme de dispersions de particules solides ou liquides de polymère (latex ou pseudolatex).

Les polymères fixants anioniques généralement utilisés sont des polymères comportant des groupements dérivés d'acide carboxylique, sulfonique ou phosphorique et ont une masse moléculaire moyenne en nombre comprise entre environ 500 et 5 000 000.

Les groupements carboxyliques sont apportés par des monomères mono ou diacides carboxyliques insaturés tels que ceux répondant à la formule : 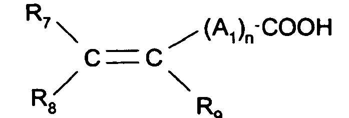 dans laquelle n est un nombre entier de 0 à 10, A₁ désigne un groupement méthylène, éventuellement relié à l'atome de carbone du groupement insaturé ou au groupement méthylène voisin lorsque n est supérieur à 1, par l'intermédiaire d'un hétéroatome tel qu'oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle.

Dans la formule précitée, un groupement alkyle inférieur désigne de préférence un groupement ayant 1 à 4 atomes de carbone et en particulier, les groupements méthyle et éthyle.

Les polymères fixants anioniques à groupements carboxyliques préférés selon l'invention sont :
A) Les homo- ou copolymères d'acide acrylique ou méthacrylique ou leurs sels et en particulier les produits vendus sous les dénominations VERSICOL^{®} E ou K par la société ALLIED COLLOID et ULTRAHOLD^{®} par la société BASF, les copolymères d'acide acrylique et d'acrylamide, les sels de sodium des acides polyhydroxycarboxyliques.
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français n° 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevets luxembourgeois n^{os} 75370 et 75371. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀, par exemple, de lauryle, tels que celui commercialisé par la société ISP sous la dénomination ACRYLIDONE^{®} LM et les terpolymères acide méthacrylique/acrylate d'éthyle/acrylate de tertiobutyle tels que le produit commercialisé sous la dénomination LUVIMER^{®} 100 P par la société BASF.
C) Les copolymères dérivés d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés et réticulés, ou encore un autre monomère ester vinylique, allylique ou méthallylique d'un acide carboxylique α- ou β-cyclique. De tels polymères sont décrits entre autres dans les brevets français n^{os} 1 222 944, 1 580 545, 2 265 782, 2 265 781, 1 564 110 et 2 439 798. Un produit commercial entrant dans cette classe est la résine 28-29-30 commercialisée par la société National Starch.
D) Les copolymères dérivés d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US nos 2 047 398, 2 723 248, 2 102 113, le brevet GB n° 839 805. Des produits commerciaux sont notamment ceux vendus sous les dénominations GANTREZ^{®} AN ou ES par la société ISP.
   - les copolymères comprenant (i) un ou plusieurs motifs anhydrides maléique, citraconique, itaconique et (ii) un ou plusieurs monomères choisis parmi les esters allyliques
   ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne,
   les fonctions anhydrides de ces copolymères étant éventuellement monoestérifiées ou monoamidifiées.
   Ces polymères sont par exemple décrits dans les brevets français n^{os} 2 350 384 et 2 357 241 de la demanderesse.
E) Les polyacrylamides comportant des groupements carboxylates.

Les polymères comprenant les groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :
- les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone.
- les sels de l'acide polystyrène-sulfonique tels que les sels de sodium vendus par exemple sous la dénomination Flexan^{®} 130 par National Starch. Ces composés sont décrits dans le brevet FR 2 198 719.
- les sels d'acides polyacrylamide-sulfoniques tels que ceux mentionnés dans le brevet US 4 128 631, et plus particulièrement l'acide polyacrylamidoéthylpropane-sulfonique.

Selon l'invention, parmi les polymères fixants anioniques cités ci-dessus, on préférera les copolymères d'acide acrylique tels que les terpolymères acide acrylique/acrylate d'éthyle/N-tertiobutylacrylamide vendus notamment sous la dénomination ULTRAHOLD^{®} STRONG par la société BASF, les copolymères dérivés d'acide crotonique tels que les terpolymères acétate de vinyle/tertio-butylbenzoate de vinyle/acide crotonique et les terpolymères acide crotonique/acétate de vinyle/ néododécanoate de vinyle vendus notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifié vendus, par exemple, sous la dénomination GANTREZ^{®} par la société ISP, les copolymères d'acide méthacrylique et de méthacrylate de méthyle vendus sous la dénomination EUDRAGIT^{®} L par la société ROHM PHARMA, les copolymères d'acide méthacrylique et d'acrylate d'éthyle vendus sous la dénomination LUVIMER^{®} MAEX ou MAE par la société BASF et les copolymères acétate de vinyle/acide crotonique et les copolymères acétate de vinyle/acide crotonique greffés par du polyéthylèneglycol vendus sous la dénomination ARISTOFLEX^{®} A par la société BASF.

Les polymères fixants anioniques cités ci-dessus, les plus particulièrement préférés sont choisis parmi les copolymères méthylvinyléther/anhydride maléique monoestérifiés vendus sous la dénomination GANTREZ^{®} ES 425 par la société ISP, les terpolymères acide acrylique/acrylate d'éthyle/N-tertiobutylacrylamide vendus sous la dénomination ULTRAHOLD^{®} STRONG par la société BASF, les copolymères d'acide méthacrylique et de méthacrylate de méthyle vendus sous la dénomination EUDRAGIT^{®} L par la société ROHM PHARMA, les terpolymères acétate de vinyle/tertio-butylbenzoate de vinyle/acide crotonique et les terpolymères acide crotonique/acétate de vinyle/ néododécanoate de vinyle vendus sous la dénomination Résine 28-29-30 par la société NATIONAL STARCH, les copolymères d'acide méthacrylique et d'acrylate d'éthyle vendus sous la dénomination LUVIMER^{®} MAEX OU MAE par la société BASF, les terpolymères vinylpyrrolidone/acide acrylique/méthacrylate de lauryle vendus sous la dénomination ACRYLIDONE^{®} LM par la société ISP.

Les polymères fixants amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques, ou bien B et C peuvent désigner des groupements dérivant de monomères zwittérioniques de carboxybétaïnes ou de sulfobétaïnes;

B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-α,β-dicarboxylique dont l'un des groupements carboxyliques a été amené à réagir avec une polyamine comportant un ou plusieurs groupements amine primaire ou secondaire.

Les polymères fixants amphotères répondant à la définition donnée ci-dessus plus particulièrement préférés sont choisis parmi les polymères suivants :
(1) les polymères résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique, tel que plus particulièrement les dialkylaminoalkylméthacrylate et acrylate, les dialkylaminoalkylméthacrylamide et acrylamide. De tels composés sont décrits dans le brevet américain n° 3 836 537.
(2) les polymères comportant des motifs dérivant :
   a) d'au moins un monomère choisi parmi les acrylamides ou les méthacrylamides substitués sur l'atome d'azote par un groupe alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou diéthyle.

   Les acrylamides ou méthacrylamides N-substitués plus particulièrement préférés selon l'invention sont les composés dont les groupes alkyle comportent de 2 à 12 atomes de carbone, et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléïque, fumarique ainsi que les monoesters d'alkyle ayant 1 à 4 atomes de carbone des acides ou des anhydrides maléïque ou fumarique.
   Les comonomères basiques préférés sont des méthacrylates d'aminoéthyle, de butylaminoéthyle, de N,N'-diméthylaminoéthyle, de N-tertio-butylaminoéthyle.
   On utilise particulièrement les copolymères dont la dénomination CTFA (4ème Ed., 1991) est Octylacrylamide/acrylates/butylaminoethyl-methacrylate copolymer, tels que les produits vendus sous la dénomination AMPHOMER^{®} ou LOVOCRYL^{®} 47 par la société NATIONAL STARCH.
(3) les polyaminoamides réticulés et acylés partiellement ou totalement dérivant de poyaminoamides de formule générale :

   ⁅CO-R₁₀-CO-Z⁆

   dans laquelle R₁₀ représente un groupe divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un groupe dérivant de l'addition de l'un quelconque desdits acides avec une amine bis-primaire ou bis-secondaire, et Z désigne un groupe dérivant d'une polyalkylène-polyamine bis-primaire, mono- ou bis-secondaire et de préférence représente :
   a) dans les proportions de 60 à 100 % en moles, le groupe 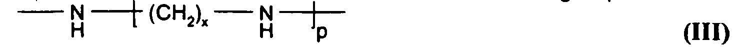 où x=2 et p=2 ou 3, ou bien x=3 et p=2
      ce groupe dérivant de la diéthylène-triamine, de la triéthylène-tétraamine ou de la dipropylène-triamine;
   b) dans les proportions de 0 à 40 % en moles, le groupe (IIIbis) ci-dessus, dans lequel x=2 et p=1 et qui dérive de l'éthylène-diamine, ou le groupe dérivant de la pipérazine : 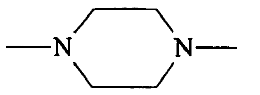
   c) dans les proportions de 0 à 20 % en moles, le groupe -NH-(CH₂)₆-NH- dérivant de l'hexaméthylènediamine,
      ces polyaminoamides étant réticulés par réaction d'addition d'un agent réticulant bifonctionnel choisi parmi les épihalohydrines, les diépoxydes, les dianhydrides, les dérivés bis-insaturés, au moyen de 0,025 à 0,35 mole d'agent réticulant par groupement amine du polyaminoamide, et acylés par action d'acide acrylique, d'acide chloracétique ou d'une alcane-sultone ou de leurs sels.

   Les acides carboxyliques saturés sont choisis de préférence parmi les acides ayant 6 à 10 atomes de carbone tels que les acides adipique, triméthyl-2,2,4-adipique et triméthyl-2,4,4-adipique, téréphtalique, les acides à double liaison éthylénique comme, par exemple, les acides acrylique, méthacrylique, itaconique.
   Les alcane-sultones utilisées dans l'acylation sont de préférence la propane- ou la butane-sultone, les sels des agents d'acylation sont de préférence les sels de sodium ou de potassium.
(4) les polymères comportant des motifs zwittérioniques de formule : 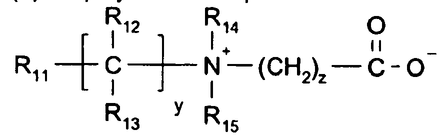 dans laquelle R₁₁ désigne un groupement insaturé polymérisable tel qu'un groupement acrylate, méthacrylate, acrylamide ou méthacrylamide, y et z représentent un nombre entier de 1 à 3, R₁₂ et R₁₃ représentent un atome d'hydrogène, un groupe méthyle, éthyle ou propyle, R₁₄ et R₁₅ représentent un atome d'hydrogène ou un groupe alkyle de telle façon que la somme des atomes de carbone dans R₁₄ et R₁₅ ne dépasse pas 10.
   Les polymères comprenant de telles unités peuvent également comporter des motifs dérivés de monomères non zwittérioniques tels que l'acrylate ou le méthacrylate de diméthyl-
   ou diéthylaminoéthyle ou des acrylates ou méthacrylates d'alkyle, des acrylamides ou méthacrylamides, ou l'acétate de vinyle.
   A titre d'exemple, on peut citer les copolymères méthacrylate de méthyle/diméthylcarboxyméthylammonioéthyl méthacrylate de méthyle.
(5) les polymères dérivés du chitosane comportant des motifs monomères répondant aux formules suivantes : 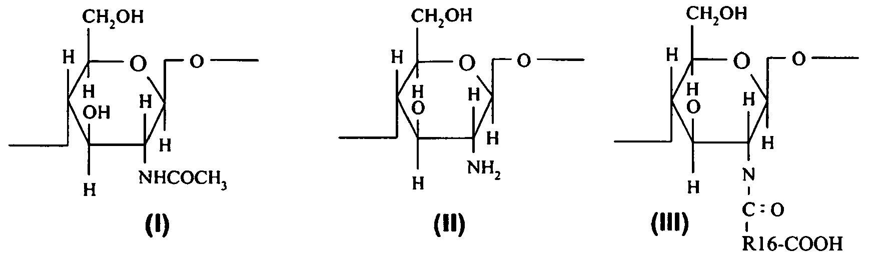 le motif (I) étant présent dans des proportions comprises entre 0 et 30%, le motif (11) dans des proportions comprises entre 5 et 50% et le motif (III) dans des proportions comprises entre 30 et 90%, étant entendu que dans ce motif (III), R₁₆ représente un groupe de formule : 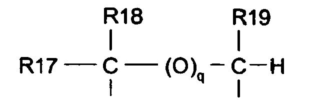 dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des groupes R₁₇, R₁₈ et R₁₉ étant dans ce cas un atome d'hydrogène ;
   ou si q=1, R₁₇, R₁₈ et R₁₉ représentent chacun un atome d'hydrogène, ainsi que les sels formés par ces composés avec des bases ou des acides.
(6) Les polymères répondant à la formule générale (V) sont, par exemple, décrits dans le brevet français 1 400 366 : 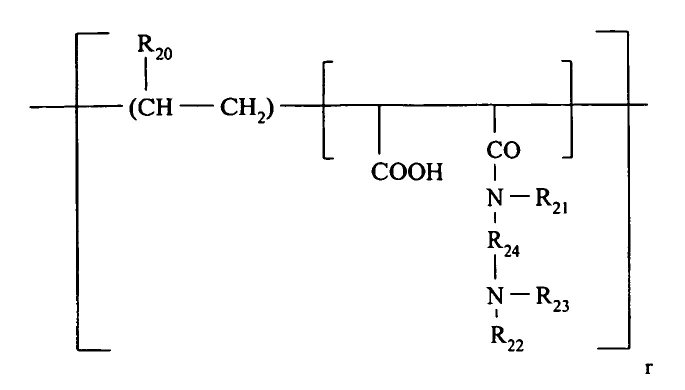 dans laquelle R₂₀ représente un atome d'hydrogène, un groupe CH₃O, CH₃CH₂O, phényle, R₂₁ désigne un atome d'hydrogène ou un groupe alkyle inférieur tel que méthyle, éthyle, R₂₂ désigne un atome d'hydrogène ou un groupe alkyle inférieur en C₁-C₆ tel que méthyle, éthyle, R₂₃ désigne un groupe alkyle inférieur en C₁-C₆ tel que méthyle, éthyle ou un groupe répondant à la formule : -R₂₄-N(R₂₂)₂, R₂₄ repré-sentant un groupement -CH₂-CH₂-,
   - CH₂-CH₂-CH₂-, -CH₂-CH(CH₃)-, R₂₂ ayant les significations mentionnées ci-dessus.
(7) Les polymères dérivés de la N-carboxyalkylation du chitosane comme le N-carboxyméthyl-chitosane ou le N-carboxybutyl-chitosane.
(8) Les polymères amphotères du type -D-X-D-X choisis parmi:
   a) les polymères obtenus par action de l'acide chloracétique ou le chloracétate de sodium sur les composés comportant au moins un motif de formule :

      -D-X-D-X-D- (VI)

      où D désigne un groupe 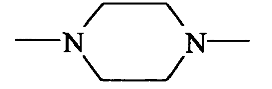 et X désigne le symbole E ou E', E ou E' identiques ou différents désignent un groupe bivalent qui est un groupe alkylène à chaîne droite ou ramifiée, comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   b) Les polymères de formule:

      -D-X-D-X- (VI')
   où D désigne un groupe 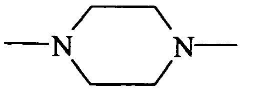 et X désigne le symbole E ou E' et au moins une fois E' ; E ayant la signification indiquée ci-dessus et E' est un groupe bivalent qui est un groupe alkylène à chaîne droite ou ramifiée, ayant jusqu'à 7 atomes de carbone dans la chaîne principale, substitué ou non par un ou plusieurs groupes hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) les copolymères alkyl(C₁-C₅)vinylétherlanhydride maléique modifiés partiellement par semiamidification avec une N,N-dialkylaminoalkylamine telle que la N,N-diméthylaminopropylamine ou par semiestérification avec un N,N-dialkylaminoalcanol. Ces copolymères peuvent également comporter d'autres comonomères vinyliques tels que le vinylcaprolactame.

Parmi les polymères fixants amphotères cités ci-dessus les plus particulièrement préférés selon l'invention, on citera ceux de la famille (3) tels que les copolymères dont la dénomination CTFA est Octylacrylamide/acrylates/butylamino-ethylmethacrylate copolymer, tels que les produits vendus sous les dénominations AMPHOMER^{®}, AMPHOMER^{®} LV 71 ou LOVOCRYL^{®} 47 par la société NATIONAL STARCH et ceux de la famille (4) tels que les copolymères méthacrylate de méthyle/diméthyl-carboxyméthylammonio-éthylméthacrylate de méthyle.

Les polymères fixants non ioniques utilisables selon la présente invention sont choisis, par exemple, parmi :
- les polyalkyloxazolines ;
- les homopolymères d'acétate de vinyle ;
- les copolymères d'acétate de vinyle et d'ester acrylique ;
- les copolymères d'acétate de vinyle et d'éthylène ;
- les copolymères d'acétate de vinyle et d'ester maléïque, par exemple, de maléate de dibutyle ;
- les copolymères d'esters acryliques tels que, par exemple, les copolymères d'acrylates d'alkyle et de méthacrylates d'alkyle tels que les produits proposés par la société ROHM & HAAS sous les dénominations PRIMAL^{®} AC-261 K et EUDRAGIT^{®} NE 30 D, par la société BASF sous la dénomination 8845, par la société HOECHST sous la dénomination APPRETAN^{®} N9212 ;
- les copolymères d'acrylonitrile et d'un monomère non ionique choisis, par exemple, parmi le butadiène et les (méth)acrylates d'alkyle ; on peut citer les produits proposés sous la dénomination CJ 0601 B par la société ROHM & HAAS ;
- les homopolymères de styrène ;
- les copolymères de styrène et de (méth)acrylate d'alkyle tels que les produits MOWILITH^{®} LDM 6911, MOWILITH^{®} DM 611 et MOWILITH^{®} LDM 6070 proposés par la société HOECHST, les produits RHODOPAS^{®} SD 215 et RHODOPAS^{®} DS 910 proposés par la société RHODIA CHIMIE;
- les copolymères de styrène, de méthacrylate d'alkyle et d'acrylate d'alkyle ;
- les copolymères de styrène et de butadiène ;
- les copolymères de styrène, de butadiène et de vinylpyridine ;
- les copolymères d'acrylate d'alkyle et d'uréthanne ;
- les polyamides,
- les homopolymères et copolymères de vinyllactame.

Les groupes alkyle des polymères non ioniques mentionnés ci-dessus ont, de préférence, de 1 à 6 atomes de carbone.

Selon la présente invention, les polymères fixants non-ioniques à motifs vinyllactames peuvent être ceux décrits dans les brevets US 3 770 683, US 3 929 735, US 4 521 504, US 5 158 762, US 5 506 315 et dans les demandes de brevet WO 94/121148, WO 96/06592 et WO 96/10593. lls peuvent se présenter sous forme pulvérulente ou sous forme de solution
ou de suspension.

Les homopolymères ou copolymères à motifs vinyllactame comprennent des motifs de formule (IX): 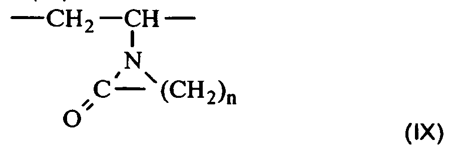 dans laquelle n est indépendamment 3, 4 ou 5.
La masse moléculaire en nombre des polymères à motifs vinyllactames est généralement supérieure à environ 5 000, de préférence comprise entre 10 000 et 1 000 000 environ, plus préférentiellement comprise entre 10 000 et 100 000 environ.

Parmi ces polymères fixants, on peut citer les polyvinylpyrrolidones telles que celles commercialisées sous la dénomination Luviskol^{®} K30 par la société BASF ; les polyvinylcaprolactames tels que ceux commercialisés sous la dénomination Luviskol^{®} PLUS par la société BASF ; les copolymères poly(vinylpyrrolidone/acétate de vinyle) tels que ceux commercialisés sous la dénomination PVPVA^{®} S630L par la société ISP, Luviskol^{®} VA 73, VA 64, VA 55, VA 37 et VA 28 par la société BASF ; et les terpolymères poly(vinylpyrrolidone/acétate de vinyle/propionate de vinyle) tels que, par exemple, ceux commercialisés sous la dénomination Luviskol^{®} VAP 343 par la société BASF.

Les polymères fixants selon l'invention peuvent aussi être choisis parmi les polyuréthanes non ioniques ou anioniques éventuellement siliconés.

La teneur en polymère conditionneur ou en polymère fixant varie avantageusement de 0,01 à 20 % en poids, par rapport au poids de la composition tinctoriale, et de préférence de 0,1 à 5 % en poids, par rapport au poids de la composition tinctoriale.

Lorsque la composition comprend au moins un tensioactif, ce dernier est choisi parmi les espèces non ioniques, anioniques, cationiques, amphotères ou zwittérioniques.

A titre d'exemple de tensioactifs anioniques utilisables, seuls ou mélanges, on peut citer notamment les sels des composés suivants, en particulier les sels de métaux alcalins
ou alcalino-terreux, notamment le sodium, le magnésium ; les sels d'ammonium, les sels d'amines, les sels d'aminoalcools : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates.

On peut également utiliser les esters d'alkyl(C₆-C₂₄)polyglycosides carboxyliques tels que les alkylglucoside citrates, les alkylpolyglycoside tartrate et les alkylpolyglycoside sulfosuccinates, les alkylsulfosuccinamates ; les acyliséthionates et les N-acyltaurates, le radical alkyle ou acyle des composés précités est de préférence en C₁₂-C₂₀, et le radical aryle désigne de préférence un groupement phényle ou benzyle.

Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle est en C₈-C₂₀.

On peut également utiliser les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

A titre d'exemple de tensioactifs non ioniques, on peut citer entre autres les alcools, les alpha-diols, les alkylphénols polyéthoxylés et/ou polypropoxylés, en C₈-C₁₈, le nombre de groupements oxyde d'éthylène et/ou oxyde de propylène étant compris entre 2 et 50.

On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine.

Pour ce qui a trait aux tensioactifs amphotères ou zwittérioniques, on peut mentionner sans avoir l'intention de s'y limiter, les dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₂ -CONHCH₂CH₂ -N(R₃)(R₄)(CH₂COO⁻)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₂'-CONHCH₂CH₂-N(B)(C)

dans laquelle :
B représente -CH₂CH₂OX', C représente -(CH₂)_{z}-Y', avec z = 1 ou 2,
X' désigne le groupement -CH₂CH₂-COOH ou un atome d'hydrogène
Y' désigne -COOH ou le radical -CH₂-CHOH-SO₃H
R₂' désigne un radical alkyle d'un acide R₉-COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid, disodium cocoamphocarboxy ethyl hydroxypropyl sulfonate.

A titre d'exemples on peut citer le cocoamphodiacetate commercialisé sous la dénomination commerciale MIRANOL^{®} C2M concentré par la société Rhodia Chimie.

Parmi les tensioactifs cationiques on peut citer en particulier les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium; les dérivés d'imidazoline ; ou les oxydes d'amines à caractère cationique ; les mono- di- esters quaternaires appelés esterquat.

La teneur en agents tensioactifs, lorsqu'ils sont présents, représente plus particulièrement de 0,001 à 30 % en poids et de préférence de 0,05 à 30 % en poids, par rapport au poids de la composition tinctoriale.

La composition peut encore comprendre des additifs usuels dans le domaine comme notamment des agents épaississants minéraux, des agents antioxydants, des agents de pénétration, des agents séquestrants, des parfums, des tampons, des agents dispersants, des agents de conditionnement tels que par exemple des cations, des silicones volatiles ou non volatiles, modifiées ou non modifiées, des agents filmogènes, des céramides, des agents conservateurs, des agents stabilisants, des agents opacifiants.

Bien entendu, l'homme de l'art veillera à choisir ce ou ces éventuels composés complémentaires de manière telle que les propriétés avantageuses attachées intrinsèquement à la composition conforme à l'invention ne soient pas, ou substantiellement pas, altérées par la ou les adjonctions envisagées.

Le pH de la composition conforme à l'invention est généralement compris entre 3 et 12 environ, et de préférence entre 4 et 10 environ.

Il peut être ajusté à la valeur désirée au moyen d'agents acidifiants ou alcalinisants.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (A) suivante : 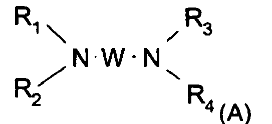 dans laquelle W est un reste propylène éventuellement substitué par un groupement hydroxyle ou un radical alkyle en C₁-C₆ ; R₁, R₂, R₃ et R₄, identiques ou différents, représentent un atome d'hydrogène, un radical alkyle en C₁-C₆ ou hydroxyalkyle en C₁-C₆.

La composition selon l'invention peut se présenter sous des formes diverses, telles que sous forme de liquides, de shampooings, de crèmes, de gels, de mousse, ou sous toute autre forme appropriée.

La présente invention a de même pour objet une composition prête à l'emploi comprenant la composition tinctoriale comprenant au moins un composé de formule (1) ou (2).

On rappelle qu'une composition prête à l'emploi correspond à une composition destinée à être appliquée immédiatement sur les fibres kératiniques, c'est à dire qu'elle peut être stockée telle quelle ou résulter du mélange extemporané de deux ou plusieurs compositions avant utilisation.

Selon une première variante de l'invention, la composition tinctoriale qui vient d'être décrite est une composition qui peut être appliquée directement sur les matières kératiniques. Plus particulièrement, cette composition est exempte d'agent oxydant.

Une telle composition est appropriée notamment lorsque la composition comprend le
ou les composés porphyrine ou phtalocyanine, éventuellement un ou plusieurs colorants directs additionnels, et que l'on ne souhaite pas obtenir un effet d'éclaircissement des fibres.

Selon une deuxième variante de l'invention, la composition prête à l'emploi comprend, outre le ou les composés porphyrine ou phtalocyanine, au moins un agent oxydant. Dans ce cas, la composition peut aussi éventuellement comprendre au moins un colorant direct additionnel, au moins une base d'oxydation et/ou au moins un coupleur. Elle peut également comprendre au moins un agent tensioactif et/ou polymère.

L'agent oxydant peut être choisi par exemple parmi le peroxyde d'hydrogène, le peroxyde d'urée, les bromates de métaux alcalins, les persels tels que les perborates et persulfates, et les enzymes telles que les peroxydases et les oxydo-réductases à deux ou à quatre électrons. L'utilisation du peroxyde d'hydrogène ou des enzymes est particulièrement préférée.

La composition prête à l'emploi conforme à cette deuxième variante peut être obtenue par mélange avant l'utilisation, d'une ou plusieurs compositions tinctoriales comprenant le ou les colorants directs (porphyrine et/ou phtalocyanine et colorants directs additionnels s'ils sont présents) et/ou précurseurs de colorants (base/coupleur) associés ou non dans une ou plusieurs compositions, avec une composition comprenant au moins un agent oxydant (composition oxydante).

La teneur en agent oxydant est en général comprise entre 1 et 40 % en poids, par rapport au poids de la composition prêt à l'emploi de préférence entre 1 et 20 % en poids par rapport au poids de la composition prêt à l'emploi.

Généralement, la composition oxydante utilisée est une composition aqueuse et peut se trouver sous la forme d'une solution ou encore d'une émulsion.

Habituellement, on mélange la composition exempte d'agent oxydant avec environ 0,5 à 10 équivalents en poids de la composition oxydante.

Notons que le pH de la composition prête à l'emploi est plus particulièrement compris entre 3 et 12, de préférence entre 4 et 10.

Le pH de la composition prête à l'emploi peut être ajusté au moyen d'un agent alcalinisant ou acidifiant notamment choisi parmi ceux mentionnés auparavant.

Comme indiqué auparavant, la présente invention concerne de plus un procédé de coloration des matières kératiniques mettant en oeuvre la composition selon l'invention.

Selon une première variante, le procédé consiste à appliquer la composition en l'absence d'un agent oxydant, sur les matières kératiniques, de préférence des fibres, sèches ou humides, avec ou sans rinçage final de la composition.

Selon une deuxième variante de l'invention, le procédé consiste à appliquer la composition selon l'invention, en présence d'un agent oxydant, sur les matières kératiniques, sèches ou humides, puis à laisser poser pendant une durée suffisante pour obtenir la coloration souhaitée.

La composition appliquée peut être la composition prête à l'emploi, c'est-à-dire une composition obtenue par mélange extemporané d'une ou compositions tinctoriales comprenant les colorants avec une composition comprenant au moins un agent oxydant.

Le procédé selon cette deuxième variante peut consister aussi à appliquer une composition tinctoriale exempte d'agent oxydant puis une composition comprenant au moins un agent oxydant, ou l'inverse, avec ou sans rinçage intermédiaire.

Le temps nécessaire au développement de la coloration est d'environ quelques secondes à 60 minutes et plus particulièrement d'environ 1 à 40 minutes.

La température nécessaire au développement de la coloration est généralement comprise entre la température ambiante (15 à 25°C) et 250°C, plus particulièrement entre la température ambiante et 180°c, de préférence entre la température ambiante et 60 °C.

Une fois le temps nécessaire au développement de la coloration écoulé, on élimine de préférence la composition. Ceci peut avoir lieu de manière classique, soit en mettant en oeuvre au moins un rinçage, soit en mettant en oeuvre un ou plusieurs lavages et rinçage(s), soit en mettant en oeuvre leur combinaison. Enfin, on sèche les matières kératiniques ou on les laisse sécher.

Au cas où la composition prête à l'emploi ne comprend pas de précurseur(s) de colorant(s) d'oxydation(s) (base et/ou coupleur) mais seulement, en tant que colorant, un ou plusieurs colorants directs de formule (1) et/ou (2) et éventuellement un ou plusieurs colorants directs additionnels différents, le procédé peut être réalisé dans des conditions dites éclaircissantes.

Au cas où la composition est appliquée en présence d'un agent oxydant, le procédé peut comprendre une étape préliminaire consistant à stocker sous forme séparée, d'une part, une ou plusieurs compositions comprenant, dans un milieu approprié pour la teinture des fibres kératiniques humaines, le ou les composés de formule (1) et/ou (2), éventuellement au moins un colorant direct additionnel, éventuellement au moins une base d'oxydation et/ou au moins un coupleur et, d'autre part, une composition comprenant, dans un milieu approprié pour la teinture des fibres kératiniques humaines, au moins un agent oxydant, puis à procéder à leur mélange au moment de l'emploi avant d'appliquer ce mélange sur les matières kératiniques, sèches ou humides, pendant un temps suffisant pour développer la coloration désirée, après quoi, éventuellement après avoir effectué un rinçage des matières kératiniques, on les lave éventuellement au shampooing, on les rince à nouveau et on les sèche.

Le temps nécessaire au développement de la coloration est d'environ quelques secondes à 60 minutes et plus particulièrement d'environ 1 à 40 minutes.

La température nécessaire au développement de la coloration est généralement comprise entre la température ambiante (15 à 25°C) et 250°C, plus particulièrement entre la température ambiante et 180°c, de préférence entre la température ambiante et 60 °C.

Un autre objet de l'invention est un dispositif à plusieurs compartiments pour la coloration des matières kératiniques et notamment des fibres, comprenant un ou plusieurs compartiments avec, dans un milieu approprié pour la teinture des fibres kératiniques humaines, le ou les composés de type porphyrine et/ou phtalocyanine cationique non métallique ou cationique métallique ne comprenant à titre d'élément métallique qu'un ou plusieurs métaux ou ion métallique de la colonne IA, IIA de la classification périodique des éléments, de zinc et le silicium, éventuellement au moins un colorant direct additionnel, éventuellement au moins une base d'oxydation et/ou au moins un coupleur ; ces composés étant présents dans une ou plusieurs compositions, associés ou non, et, d'autre part, un compartiment avec une composition comprenant, dans un milieu approprié pour la teinture des fibres kératiniques humaines, au moins un agent oxydant.

Ce dispositif peut être équipé d'un moyen permettant de délivrer sur les fibres le mélange souhaité, tel que les dispositifs décrits dans le brevet FR 2 586 913.

L'invention concerne enfin un composé de type phtalocyanine cationique de formule suivante : 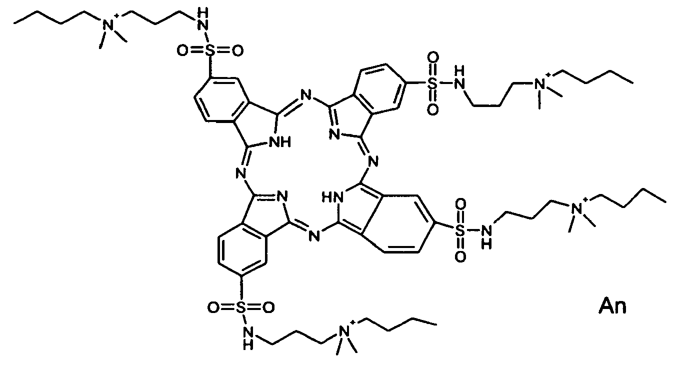 An étant défini comme précédemment.

Des exemples concrets mais non limitatifs de l'invention vont maintenant être présentés.

### EXEMPLES

### EXEMPLE 1 :

### Colorant 1: 5,10,15-20-tétra(1-méthyl-4-pyridino)porphyrin tétra(p-toluènesulfonate)

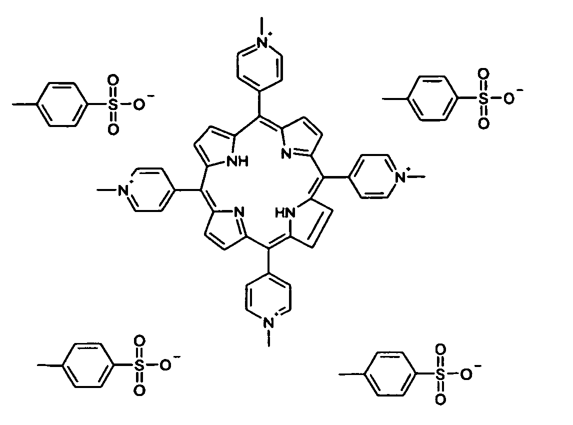

On prépare la composition A suivante :

| **Composé** | **Concentration (g%)** |
|---|---|
| Alkyl (C8/C10 50/50) polyglucoside (2) en solution aqueuse à 60 % tamponnée | 10 |
| Alcool benzylique | 10 |
| Polyéthylène glycol 400 (8EO) | 12 |
| Colorant 1 | 0.5 |
| Ammoniaque à 20.6% | 13 |
| Eau | qsp 100 |

### Montée du colorant

- La formule A est mélangée à de l'eau oxygénée commerciale 40 volumes, (mélange poids pour poids), puis appliquée sur mèches de cheveux permanentés à 90% de blancs (rapport massique formule/mèche: 10/1),
- Au bout de 20 minutes de pause à température ambiante (T = 25°C ± 3°C), les mèches sont rincées et subissent 1 shampooing (Elsève multivitamines)
- Les montées de couleur sont mesurées à l'aide d'un colorimètre (colorimètre CM2002, illuminant D65-10° SCI).

Comme le montre le tableau ci-dessous, le colorant aune bonne montée sur le cheveu.

| | **Couleur** |
|---|---|
| **Mèche non colorée** | **Gris** |
| **Colorant 1** | Marron puissant |

### Ténacité aux shampooings répétés

- La formule A est mélangée à de l'eau oxygénée commerciale 40 volumes, (mélange poids pour poids), puis appliquée sur des mèches de cheveux gris à 90% de blancs, sensibilisée par décoloration. Le rapport massique formule/mèche est de 10/1,
- Au bout de 20 minutes de pause à température ambiante (T = 25°C ± 3°C), les mèches sont rincées, séchées et les valeurs colorimétriques sont mesurées
- Les mèches subissent ensuite 6 shampooings, au bout desquels les valeurs colorimétriques sont de nouveau mesurées
- Les valeurs colorimétriques mesurées après les six shampooings sont comparées aux valeurs colorimétriques avant shampooing.

Les résultats montrent que la couleur obtenue sur cheveux sensibilisés (marron) a une très bonne ténacité aux shampooings répétés.

**Colorant 1**

| | Δ**E*ab (avant/après shampooing)** |
|---|---|
| **Colorant 1 avant shampooing** | 1,19 |
| **Colorant 1 après 6 shampooings** | |

### EXEMPLE 2 :

### Colorant 2: formule C₆₈H₁₀₂N₁₆O₈S₄I₄

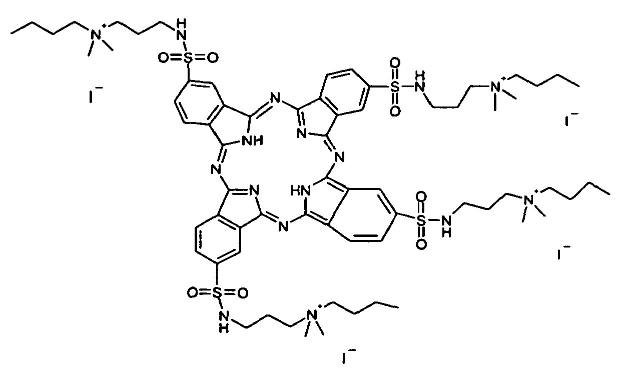

On prépare la composition B suivante :

| **Composé** | **Concentration (g%)** |
|---|---|
| Alkyl (C8/C10 50/50) polyglucoside (2) en solution aqueuse à 60 % tamponnée | 10 |
| Alcool benzylique | 10 |
| Polyéthylène glycol 400 (8EO) | 12 |
| Colorant 2 | 0.5 |
| Ammoniaque à 20.6% | 13 |
| Eau | qsp 100 |

### Montée du colorant

- La formule B est mélangée à de l'eau oxygénée commerciale 40 volumes, (mélange poids pour poids), puis appliquée sur mèches de cheveux permanentés à 90% de blancs (rapport massique formule/mèche: 10/1),
- Au bout de 20 minutes de pause à température ambiante (T = 25°C ± 3°C), les mèches sont rincées et subissent 1 shampooing (Elsève multivitamines)
- Les montées de couleur sont mesurées à l'aide d'un colorimètre (colorimètre CM2002, illuminant D65-10° SCI).

Comme le montre le tableau ci-dessous, le colorant aune bonne montée sur le cheveu.

| | **Couleur** |
|---|---|
| **Mèche non colorée** | Gris |
| **Colorant 2** | Turquoise puissant |

### Ténacité aux shampooings répétés

- La formule B est mélangée à de l'eau oxygénée commerciale 40 volumes, (mélange poids pour poids), puis appliquées sur une mèches de cheveux gris à 90% de blancs, sensibilisées par décoloration. Le rapport massique formule/mèche est de 10/1,
- Au bout de 20 minutes de pause à température ambiante (T = 25°C ± 3°C), les mèches sont rincées, séchées et les valeurs colorimétriques sont mesurées
- Les mèches subissent ensuite 6 shampooings, au bout desquels les valeurs colorimétriques sont de nouveau mesurées
- Les valeurs colorimétriques mesurées après les six shampooings sont comparées aux valeurs colorimétriques avant shampooing.

Les résultats montrent que la couleur obtenue sur cheveux sensibilisés (turquoise) a une très bonne ténacité aux shampooings répétés.

**Colorant 2**

| | Δ**E*ab (avant/après shampooing)** |
|---|---|
| **Colorant 2 avant shampooing** | 1,68 |
| **Colorant 2 après 6 shampooings** | |

### Stabilité lumière du colorant 2

- La formule B est mélangée à de l'eau oxygénée commerciale 40 volumes, (mélange poids pour poids), puis appliquée sur mèches de cheveux permanentés (BP) à 90% de blancs (rapport massique formule/mèche: 10/1),
- Au bout de 20 minutes de pause à température ambiante (T = 25°C ± 3°C), les mèches sont rincées et séchées,
- La moitié de chaque mèche est ensuite placée dans le suntest pendant 18 heures (intensité équivalente à une exposition de 3 semaines en plein mois d'août), l'autre moitié non exposée servant de témoin,
- Les valeurs colorimétriques mesurées après exposition à la lumière sont comparées aux valeurs avant exposition.

Comme le montre le tableau ci-dessous, le colorant 2 présente une très bonne stabilité à la lumière.

| | Δ**E*ab (avant/après test lumière)** |
|---|---|
| **Colorant 2 avant test lumière** | 2,28 |
| **Colorant 2 après test lumière** | |

### EXEMPLE 3 :

### Colorant 3: zinc 5,10,15-20-tétra(4-(n-méthylpyridinium)21H,23H-porphyrine tétratosylate

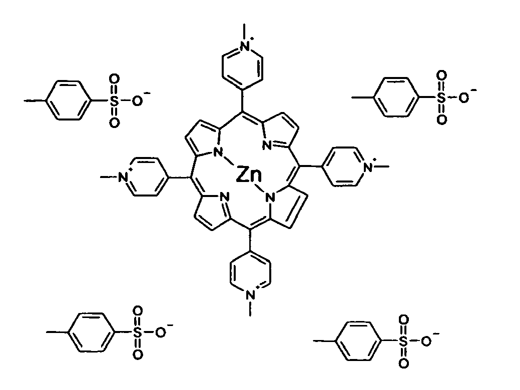

On prépare la composition C suivante :

| **Constituant** | **g%** |
|---|---|
| Alcool oléïque polyglycérolé à 2 moles de glycérol | 4 |
| Alcool oléïque polyglycérolé à 4 moles de glycérol | 5.69 MA |
| Acide oléïque | 3 |
| Aminel oléïque à 2 moles d'oxyde d'éthylène vendue sous la dénomination commerciale ETHOMEEN 012 par la société Akzo | 7 |
| Laurylamino succinamate de diéthylaminopropyle , sel de sodium à 55% de M.A. | 3.0 MA |
| Alcool oléïque | 5 |
| Diethanolamide d'acide oléïque | 12 |
| Alcool éthylique | 7 |
| Propylène glycol | 3.5 |
| Dipropylèneglycol | 0.5 |
| Monométhylether de propylèneglycol | 9 |
| Antioxydant /séquestrant | qs |
| Acétate d'ammonium | 0.8 |
| Métabisulfite de sodium en solution aqueuse à 35% | 0.455 |
| Colorant 3 | 3.71 |
| Ammoniaque à 20 % | 10 |
| Eau déminéralisée q.s.p. | 100 g |

### Montée du colorant

- La formule C est mélangée à de l'eau oxygénée commerciale 20 volumes, (mélange poids pour poids), puis appliquée sur mèches de cheveux décolorés à 90% de blancs (rapport massique formule/mèche: 10/1),
- Au bout de 20 minutes de pause à température ambiante (T = 25°C ± 3°C), les mèches sont rincées et subissent 1 shampooing (Elsève multivitamines)
- Les montées de couleur sont mesurées à l'aide d'un colorimètre (colorimètre CM2002, illuminant D65-10° SCI).

Comme le montre le tableau ci-dessous, le colorant a une bonne montée sur le cheveu.

| | **Couleur** |
|---|---|
| **Mèche non colorée** | Gris |
| **Colorant 3** | vert puissant |

### Stabilité lumière du colorant 3

- La mèche colorée avec le protocole décrit précédemment (voir paragraphe montée du colorant) est placée dans le suntest pendant 18 heures (intensité équivalente à une exposition de 3 semaines en plein mois d'août)
- Les valeurs colorimétriques mesurées après exposition à la lumière sont comparées aux valeurs avant exposition.

Comme le montre le tableau ci-dessous, le colorant 3 présente une bonne stabilité à la lumière.

| | Δ**E*ab (avant/après test lumière)** |
|---|---|
| **Colorant 3 avant test lumière** | 4,51 |
| **Colorant 3 après test lumière** | |

## Revendications

1. Utilisation d'au moins un composé de type porphyrine ou phtalocyanine cationique non métallique ou cationique métallique ne comprenant à titre d'élément métallique qu'un ou plusieurs métaux ou ions métalliques de la colonne lA, IIA de la classification périodique des éléments, le zinc et le silicium, comme colorant direct pour la coloration de matières kératiniques humaines, et notamment de fibres.

2. Utilisation selon la revendication précédente, **caractérisée en ce que** le composé cationique correspond à la formule (1) suivante, et ses formes tautomères, portant au moins une charge cationique : 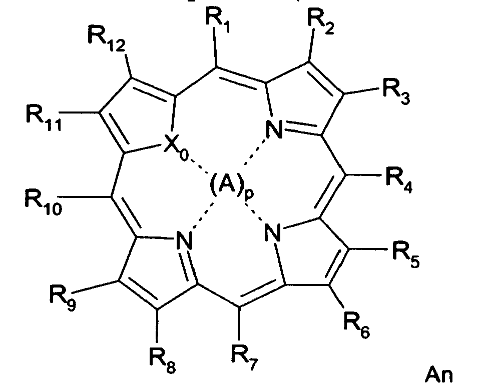 Dans laquelle :
**les radicaux R₁ à R₁₂,** identiques ou non, représentent :
■ un atome d'hydrogène ;
■ un radical alkyle en C₁-C₃₀, linéaire ou ramifié ; un radical alcényle en C₂-C₃₀, linéaire ou ramifié ; un radical alcynyle en C₂-C₃₀, linéaire ou ramifié ;
■ lesdits radicaux alkyle, alcényle, alcynyle pouvant éventuellement être substitués par au moins un groupement monovalent choisi parmi les groupements suivants, ou leurs combinaisons :
• hydroxyle,
• amino ;
• amino substitué par un ou deux radicaux alkyle identiques ou différents, en C₁-C₁₀, linéaires ou ramifiés, éventuellement porteur d'un ou plusieurs groupements hydroxyle ou alcoxy, en C₁-C₁₀, linéaires ou ramifiés ;
• ammonium substitué par un, deux ou trois radicaux alkyle identiques ou différents, en C₁-C₁₀, linéaires ou ramifiés, éventuellement porteur d'un ou plusieurs groupements hydroxyle ou alcoxy, en C₁-C₁₀, linéaires ou ramifiés ;
• hydrogénocarbonyle (-COH) ;
• un hétérocycle à 5 ou 6 chaînons, comprenant un ou plusieurs hétéroatomes comme l'oxygène, l'azote, éventuellement substitué, éventuellement porteur d'au moins une charge cationique ; ledit hétérocycle pouvant être condensé avec un noyau aromatique, de préférence à 6 chaînons ;
■ et/ou lesdits radicaux alkyle, alcényle, alcynyle pouvant éventuellement être interrompus par au moins un groupement divalent choisi parmi les groupements :
• un atome d'oxygène ;
• amino ;
• amino substitué par un radical alkyle en C₁-C₁₀, linéaire ou ramifié, éventuellement porteur d'un ou plusieurs groupements hydroxyle ou alcoxy, en C₁-C₁₀, linéaires ou ramifiés ;
• ammonium substitué par un ou deux radicaux alkyle identiques ou différents, en C₁-C₁₀, linéaires ou ramifiés, éventuellement porteur d'un ou plusieurs groupements hydroxyle ou alcoxy, en C₁-C₁₀, linéaires ou ramifiés ;
• carbonyle (-CO-) ;
• un hétérocycle à 5 ou 6 chaînons, comprenant un ou plusieurs hétéroatomes comme l'oxygène, l'azote, éventuellement substitué, éventuellement porteur d'au moins une charge cationique
■ un radical aryle en C₆-C₃₀, éventuellement substitué ; un radical alkyl(C₁-C₃₀)aryle(C₆-C₃₀), éventuellement substitué ; un radical aryl(C₆-C₃₀)alkyle(C₁-C₃₀), éventuellement substitué ;
■ un groupe hydroxyle ;
■ un radical alcoxy en C₁-C₃₀, linéaire ou ramifié, éventuellement substitué ;
■ un radical amino ; un radical amino portant un ou plusieurs radicaux alkyles, linéaires ou ramifiés en C₁-C₃₀, éventuellement substitués ;
■ un radical -SO₂-NH₂, -SO₂NH-alkyle, -NH-SO₂-alkyle, dans lesquels le radical alkyle est en C₁-C₃₀, linéaire ou ramifié, éventuellement substitué ;
■ un radical hétérocyclique éventuellement cationique comprenant de 5 à 6 chaînons, de préférence aromatique, comprenant au moins un atome d'azote, et éventuellement au moins un autre hétéroatome, identique ou différent ; ledit radical hétérocyclique étant éventuellement substitué ;
■ l'un au moins des radicaux R₁ à R₁₂ porte au moins une charge cationique ;
**X₀** représente un atome d'azote ou un atome d'oxygène ;
**A** représente un métal ou un ion métallique de la colonne IA, IIA de la classification périodique des éléments, tels que le sodium, le potassium, le magnésium, le calcium ; le zinc ou le silicium ;
**p** vaut 0, ou 1 ou 2 selon la nature de l'élément A ;
**An** représentant un ou plusieurs anions cosmétiquement acceptables compensant la charge cationique totale du composé.

3. Utilisation selon l'une quelconque des revendications précédentes, **caractérisée en ce que** le composé cationique correspond à la formule (2) suivante, et ses formes tautomères, portant au moins une charge cationique : 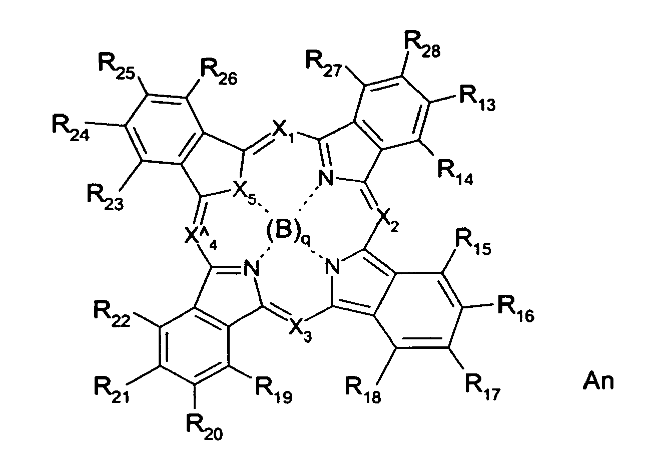 Dans laquelle
**les radicaux R**₁₃ **à R**₂₈, identiques ou non, représentent :
■ un atome d'hydrogène ;
■ un radical alkyle en C₁-C₃₀, linéaire ou ramifié ; un radical alcényle en C₂-C₃₀, linéaire ou ramifié ; un radical alcynyle en C₂-C₃₀, linéaire ou ramifié ;
■ lesdits radicaux alkyle, alcényle, alcynyle pouvant éventuellement être substitués par au moins un groupement monovalent choisi parmi les groupements suivants, ou leurs combinaisons :
• hydroxyle,
• amino ;
• amino substitué par un ou deux radicaux alkyle identiques ou différents, en C₁-C₁₀, linéaires ou ramifiés, éventuellement porteur d'un ou plusieurs groupements hydroxyle ou alcoxy, en C₁-C₁₀, linéaires ou ramifiés ;
• ammonium substitué par un, deux ou trois radicaux alkyle identiques ou différents, en C₁-C₁₀, linéaires ou ramifiés, éventuellement porteur d'un ou plusieurs groupements hydroxyle ou alcoxy, en C₁-C₁₀, linéaires ou ramifiés ;
• hydrogénocarbonyle (-COH) ;
• un hétérocycle à 5 ou 6 chaînons, comprenant un ou plusieurs hétéroatomes comme l'oxygène, l'azote, éventuellement substitué, éventuellement porteur d'au moins une charge cationique ; ledit hétérocycle pouvant être condensé avec un noyau aromatique, de préférence à 6 chaînons ;
■ et/ou lesdits radicaux alkyle, alcényle, alcynyle pouvant éventuellement être interrompus par au moins un groupement divalent choisi parmi les groupements :
• un atome d'oxygène ;
• amino ;
• amino substitué par un radical alkyle en C₁-C₁₀, linéaire ou ramifié, éventuellement porteur d'un ou plusieurs groupements hydroxyle ou alcoxy, en C₁-C₁₀, linéaires ou ramifiés ;
• ammonium substitué par un ou deux radicaux alkyle identiques ou différents, en C₁-C₁₀, linéaires ou ramifiés, éventuellement porteur d'un ou plusieurs groupements hydroxyle ou alcoxy, en C₁-C₁₀, linéaires ou ramifiés ;
• carbonyle (-CO-) ;
• un hétérocycle à 5 ou 6 chaînons, comprenant un ou plusieurs hétéroatomes comme l'oxygène, l'azote, éventuellement substitué, éventuellement porteur d'au moins une charge cationique
■ un radical aryle en C₆-C₃₀, éventuellement substitué ; un radical alkyl(C₁-C₃₀)aryle(C₆-C₃₀), éventuellement substitué ; un radical aryl(C₆-C₃₀)alkyle(C₁-C₃ₒ), éventuellement substitué ;
■ un groupe hydroxyle ;
■ un radical alcoxy en C₁-C₃₀, linéaire ou ramifié, éventuellement substitué ;
■ un radical amino ; un radical amino portant un ou plusieurs radicaux alkyles, linéaires ou ramifiés en C₁-C₃₀, éventuellement substitués ;
■ un radical -SO₂-NH₂, -SO₂NH-alkyle, -NH-SO₂-alkyle, dans lesquels le radical alkyle est en C₁-C₃₀, linéaire ou ramifié, éventuellement substitué ;
■ un radical hétérocyclique éventuellement cationique comprenant de 5 à 6 chaînons, de préférence aromatique, comprenant au moins un atome d'azote, et éventuellement au moins un autre hétéroatome, identique ou différent ; ledit radical hétérocyclique étant éventuellement substitué ;
■ l'un au moins des radicaux R₁₃ à R₂₈ porte au moins une charge cationique ;
**X₁ à X₄,** identiques ou non, représentent un atome d'azote ou un groupement -CR₂₉, avec
R₂₉ ayant la même définition que R₁₃ à R₂₈ précités ;
**X₅** représente un atome d'azote ou un atome d'oxygène ou un atome de soufre ;
**B** représente un métal ou un ion métallique de la colonne IA, IIA de la classification périodique des éléments, tels que le sodium, le potassium, le magnésium, le calcium ; le zinc ou le silicium ;
**q** vaut 0, ou 1 ou 2 selon la nature de l'élément B ;
**An** représentant un ou plusieurs anions cosmétiquement acceptables compensant la charge cationique totale du composé.

4. Utilisation selon l'une quelconque des revendications précédentes, **caractérisée en ce que** le composé cationique non métallique est mis en ceuvre en présence d'au moins un agent oxydant.

5. Utilisation selon l'une quelconque des revendications précédentes, **caractérisée en ce que** le composé cationique non métallique est mis en oeuvre en l'absence d'agent oxydant.

6. Composition tinctoriale comprenant, dans un milieu approprié pour la teinture des matières kératiniques humaines, au moins un composé de type porphyrine ou phtalocyanine cationique non métallique ou cationique métallique ne comprenant à titre d'élément métallique qu'un ou plusieurs métaux ou ions métalliques de la colonne IA, IIA de la classification périodique des éléments, de zinc et le silicium, comme colorant direct pour la coloration de matières kératiniques humaines, et notamment de fibres, et au moins un polymère et/ou au moins un agent tensioactif.

7. Composition selon la revendication 6, **caractérisée en ce que** le composé cationique correspond à la formule (1) suivante, et ses formes tautomères, portant au moins une charge cationique : 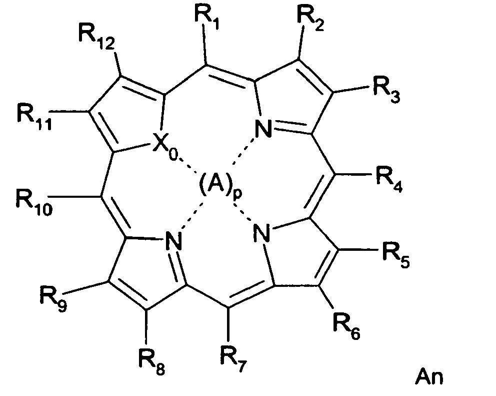 Dans laquelle :
**les radicaux R₁ à R₁₂,** identiques ou non, représentent :
■ un atome d'hydrogène ;
■ un radical alkyle en C₁-C₃₀, linéaire ou ramifié ; un radical alcényle en C₂-C₃₀, linéaire ou ramifié ; un radical alcynyle en C₂-C₃₀, linéaire ou ramifié ;
■ lesdits radicaux alkyle, alcényle, alcynyle pouvant éventuellement être substitués par au moins un groupement monovalent choisi parmi les groupements suivants, ou leurs combinaisons :
• hydroxyle,
• amino ;
• amino substitué par un ou deux radicaux alkyle identiques ou différents, en C₁-C₁₀, linéaires ou ramifiés, éventuellement porteur d'un ou plusieurs groupements hydroxyle ou alcoxy, en C₁-C₁₀, linéaires ou ramifiés ;
• ammonium substitué par un, deux ou trois radicaux alkyle identiques ou différents, en C₁-C₁₀, linéaires ou ramifiés, éventuellement porteur d'un ou plusieurs groupements hydroxyle ou alcoxy, en C₁-C₁₀, linéaires ou ramifiés ;
• hydrogénocarbonyle (-COH) ;
• un hétérocycle à 5 ou 6 chaînons, comprenant un ou plusieurs hétéroatomes comme l'oxygène, l'azote, éventuellement substitué, éventuellement porteur d'au moins une charge cationique ; ledit hétérocycle pouvant être condensé avec un noyau aromatique, de préférence à 6 chaînons ;
■ et/ou lesdits radicaux alkyle, alcényle, alcynyle pouvant éventuellement être interrompus par au moins un groupement divalent choisi parmi les groupements :
• un atome d'oxygène ;
• amino ;
• amino substitué par un radical alkyle en C₁-C₁₀, linéaire ou ramifié, éventuellement porteur d'un ou plusieurs groupements hydroxyle ou alcoxy, en C₁-C₁₀, linéaires ou ramifiés ;
• ammonium substitué par un ou deux radicaux alkyle identiques ou différents, en C₁-C₁₀, linéaires ou ramifiés, éventuellement porteur d'un ou plusieurs groupements hydroxyle ou alcoxy, en C₁-C₁₀, linéaires ou ramifiés ;
• carbonyle (-CO-) ;
• un hétérocycle à 5 ou 6 chaînons, comprenant un ou plusieurs hétéroatomes comme l'oxygène, l'azote, éventuellement substitué, éventuellement porteur d'au moins une charge cationique
■ un radical aryle en C₆-C₃₀, éventuellement substitué ; un radical alkyl(C₁-C₃₀)aryle(C₆-C₃₀), éventuellement substitué ; un radical aryl(C₆-C₃₀)alkyle(C₁-C₃₀), éventuellement substitué ;
■ un groupe hydroxyle ;
■ un radical alcoxy en C₁-C₃₀, linéaire ou ramifié, éventuellement substitué ;
■ un radical amino ; un radical amino portant un ou plusieurs radicaux alkyles, linéaires ou ramifiés en C₁-C₃₀, éventuellement substitués ;
■ un radical -SO₂-NH₂, -SO₂NH-alkyle, -NH-SO₂-alkyle, dans lesquels le radical alkyle est en C₁-C₃₀, linéaire ou ramifié, éventuellement substitué ;
■ un radical hétérocyclique éventuellement cationique comprenant de 5 à 6 chaînons, de préférence aromatique, comprenant au moins un atome d'azote, et éventuellement au moins un autre hétéroatome, identique ou différent ; ledit radical hétérocyclique étant éventuellement substitué ;
■ l'un au moins des radicaux R₁ à R₁₂ porte au moins une charge cationique ;
**X₀** représente un atome d'azote ou un atome d'oxygène ;
**A** représente un métal ou un ion métallique de la colonne IA, IIA de la classification périodique des éléments, tels que le sodium, le potassium, le magnésium, le calcium ; le zinc ou le silicium ;
**p** vaut 0, ou 1 ou 2 selon la nature de l'élément A ;
**An** représentant un ou plusieurs anions cosmétiquement acceptables compensant la charge cationique totale du composé.

8. Composition selon l'une quelconque des revendications 6 ou 7, **caractérisée en ce que** le composé cationique correspond à la formule suivante, et ses formes tautomères, portant au moins une charge cationique : 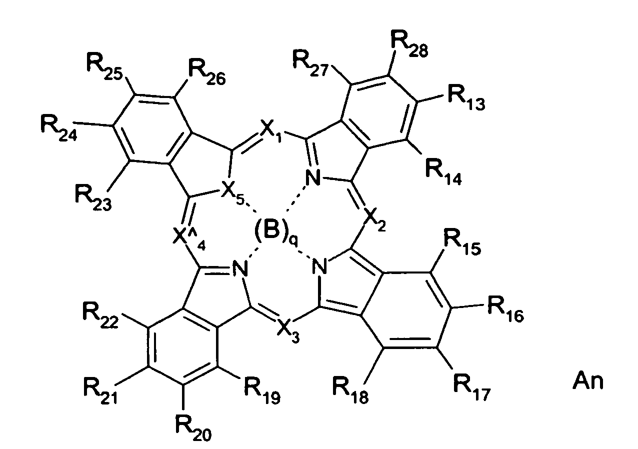 Dans laquelle
**les radicaux R₁₃ à R₂₈,** identiques ou non, représentent :
■ un atome d'hydrogène ;
■ un radical alkyle en C₁-C₃₀, linéaire ou ramifié ; un radical alcényle en C₂-C₃₀, linéaire ou ramifié ; un radical alcynyle en C₂-C₃₀, linéaire ou ramifié ;
■ lesdits radicaux alkyle, alcényle, alcynyle pouvant éventuellement être substitués par au moins un groupement monovalent choisi parmi les groupements suivants, ou leurs combinaisons :
• hydroxyle,
• amino ;
• amino substitué par un ou deux radicaux alkyle identiques ou différents, en C₁-C₁₀, linéaires ou ramifiés, éventuellement porteur d'un ou plusieurs groupements hydroxyle ou alcoxy, en C₁-C₁₀, linéaires ou ramifiés ;
• ammonium substitué par un, deux ou trois radicaux alkyle identiques ou différents, en C₁-C₁₀, linéaires ou ramifiés, éventuellement porteur d'un ou plusieurs groupements hydroxyle ou alcoxy, en C₁-C₁₀, linéaires ou ramifiés ;
• hydrogénocarbonyle (-COH) ;
• un hétérocycle à 5 ou 6 chaînons, comprenant un ou plusieurs hétéroatomes comme l'oxygène, l'azote, éventuellement substitué, éventuellement porteur d'au moins une charge cationique ; ledit hétérocycle pouvant être condensé avec un noyau aromatique, de préférence à 6 chaînons ;
■ et/ou lesdits radicaux alkyle, alcényle, alcynyle pouvant éventuellement être interrompus par au moins un groupement divalent choisi parmi les groupements :
• un atome d'oxygène ;
• amino ;
• amino substitué par un radical alkyle en C₁-C₁₀, linéaire ou ramifié, éventuellement porteur d'un ou plusieurs groupements hydroxyle ou alcoxy, en C₁-C₁₀, linéaires ou ramifiés ;
• ammonium substitué par un ou deux radicaux alkyle identiques ou différents, en C₁-C₁₀, linéaires ou ramifiés, éventuellement porteur d'un ou plusieurs groupements hydroxyle ou alcoxy, en C₁-C₁₀, linéaires ou ramifiés ;
• carbonyle (-CO-) ;
• un hétérocycle à 5 ou 6 chaînons, comprenant un ou plusieurs hétéroatomes comme l'oxygène, l'azote, éventuellement substitué, éventuellement porteur d'au moins une charge cationique
■ un radical aryle en C₆-C₃₀, éventuellement substitué ; un radical alkyl(C₁-C₃₀)aryle(C₆-C₃₀), éventuellement substitué ; un radical aryl(C₆-C₃₀)alkyle(C₁-C₃₀), éventuellement substitué ;
■ un groupe hydroxyle ;
■ un radical alcoxy en C₁-C₃₀, linéaire ou ramifié, éventuellement substitué ;
■ un radical amino ; un radical amino portant un ou plusieurs radicaux alkyles, linéaires ou ramifiés en C₁-C₃₀, éventuellement substitués ;
■ un radical -SO₂-NH₂, -SO₂NH-alkyle, -NH-SO₂-alkyle, dans lesquels le radical alkyle est en C₁-C₃₀, linéaire ou ramifié, éventuellement substitué ;
■ un radical hétérocyclique éventuellement cationique comprenant de 5 à 6 chaînons, de préférence aromatique, comprenant au moins un atome d'azote, et éventuellement au moins un autre hétéroatome, identique ou différent ; ledit radical hétérocyclique étant éventuellement substitué ;
■ l'un au moins des radicaux R₁₃ à R₂₈ porte au moins une charge cationique ;
**X₁ à X₄**, identiques ou non, représentent un atome d'azote ou un groupement -CR₂₉, avec R₂₉ ayant la même définition que R₁₃ à R₂₈ précités ;
**X**₅ représente un atome d'azote ou un atome d'oxygène ou un atome de soufre ;
**B** représente un métal ou un ion métallique de la colonne IA, IIA de la classification périodique des éléments, tels que le sodium, le potassium, le magnésium, le calcium ; le zinc ou le silicium ;
**q** vaut 0, ou 1 ou 2 selon la nature de l'élément B ;
**An** représentant un ou plusieurs anions cosmétiquement acceptables compensant la charge cationique totale du composé.

9. Composition selon l'une quelconque des revendications 7 ou 8, **caractérisée en ce que** la teneur en composé cationique représente de 0,0005 à 20 % en poids par rapport au poids de la composition tinctoriale.

10. Composition selon l'une quelconque des revendications 7 à 9, **caractérisée en ce que** le ou les agents tensioactifs sont de nature non ionique, anionique, cationique ou amphotère.

11. Composition selon la revendication précédente, **caractérisée en ce que** la teneur en agents tensioactifs varie de 0,001 à 30 % en poids par rapport au poids de la composition tinctoriale.

12. Composition selon l'une quelconque des revendications 7 à 11, **caractérisée en ce que** le polymère est de nature non ionique, cationique, anionique ou amphotère.

13. Composition selon l'une quelconque des revendications 7 à 12, **caractérisée en ce que** le polymère est un polymère épaississant, associatif ou non.

14. Composition selon la revendication précédente, **caractérisée en ce que** la teneur en en polymère(s) épaississant(s) associatif(s) ou non, varie de 0,01 à 10% en poids, plus particulièrement de 0,1 à 5% en poids, par rapport au poids de la composition tinctoriale.

15. Composition selon l'une quelconque des revendications 7 à 14, **caractérisée en ce que** le polymère est un polymère conditionneur ou un polymère fixant.

16. Composition selon la revendication précédente, **caractérisée en ce que** la teneur en polymère conditionneur ou en polymère fixant varie de 0,01 à 20 % en poids, par rapport au poids de la composition tinctoriale, et de préférence de 0,1 à 5 % en poids, par rapport au poids de la composition tinctoriale.

17. Composition selon l'une quelconque des revendications 7 à 16, **caractérisée en ce qu'**elle comprend au moins un colorant direct additionnel, différent du composé cationique non métallique.

18. Composition selon la revendication précédente, **caractérisée en ce que** la teneur en colorant direct additionnel représente de 0,0005 à 12 % en poids par rapport au poids de la composition.

19. Composition selon l'une quelconque des revendications 7 à 18, **caractérisée en ce qu'**elle comprend au moins une base d'oxydation, éventuellement associée à au moins un coupleur.

20. Composition selon la revendication précédente, **caractérisée en ce que** la teneur en base d'oxydation représente, pour chacune, de 0,0005 à 12 % en poids par rapport au poids total de la composition tinctoriale.

21. Composition selon la revendication 19, **caractérisée en ce que** la teneur en coupleur représente, pour chacun, de 0,0001 à 10 % en poids par rapport au poids total de la composition tinctoriale.

22. Composition selon l'une quelconque des revendications 7 à 21, comprenant au moins un agent oxydant.

23. Composition prête à l'emploi comprenant, dans un milieu approprié pour la teinture des matières kératiniques humaines, au moins un composé de type porphyrine ou phtalocyanine cationique non métallique ou cationique métallique ne comprenant à titre d'élément métallique qu'un ou plusieurs métaux ou ions métalliques de la colonne IA, IIA de la classification périodique des éléments, de zinc et le silicium, et au moins un agent oxydant.

24. Procédé de teinture de matières kératiniques humaines, et, notamment des fibres, consistant à appliquer la composition selon l'une quelconque des revendications 7 à 21 sur les matières kératiniques, de préférence des fibres, sèches ou humides, sans rinçage final de la composition.

25. Procédé de teinture de matières kératiniques humaines, et, notamment des fibres, consistant à appliquer la composition selon l'une quelconque des revendications 7 à 22, sur les matières kératiniques, sèches ou humides, en présence d'au moins un agent oxydant, à laisser poser pendant une durée suffisante pour obtenir la coloration souhaitée, puis à éliminer la composition.

26. Dispositif à plusieurs compartiments pour la mise en oeuvre du procédé selon la revendication 25, pour la coloration des matières kératiniques et notamment des fibres, comprenant un ou plusieurs compartiments avec, dans un milieu approprié pour la teinture des fibres kératiniques humaines, au moins un composé de type porphyrine ou phtalocyanine cationique non métallique ou cationique métallique ne comprenant à titre d'élément métallique qu'un ou plusieurs métaux ou ions métalliques de la colonne IA, IIA de la classification périodique des éléments, de zinc et le silicium, éventuellement au moins un colorant direct additionnel, éventuellement au moins une base d'oxydation et/ou au moins un coupleur ; ces composés étant présents dans une ou plusieurs compositions, associés ou non, et, d'autre part, un compartiment avec une composition comprenant, dans un milieu approprié pour la teinture des fibres kératiniques humaines, au moins un agent oxydant.

27. Composé de formule suivante :

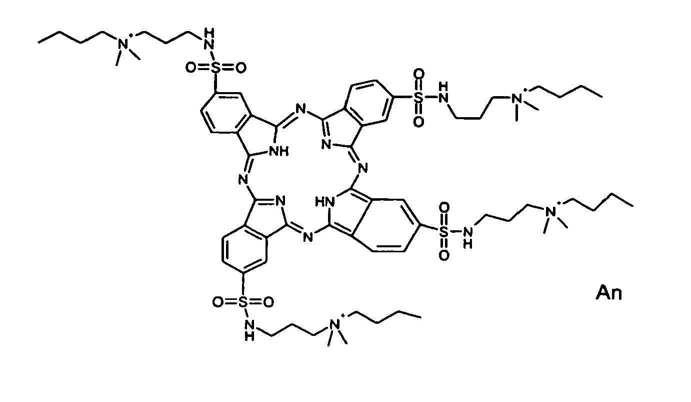

## Claims

1. Use of at least one non-metallic cationic or metallic cationic compound of porphyrin or phthalocyanin type comprising as metal element only one or more metals or metal ions from column IA or IIA of the Periodic Table of the Elements, zinc and silicon, as direct dye for dyeing human keratin materials, and especially fibres.

2. Use according to the preceding claim, **characterized in that** the cationic compound corresponds to formula (1) below, and the tautomeric forms thereof, bearing at least one cationic charge: 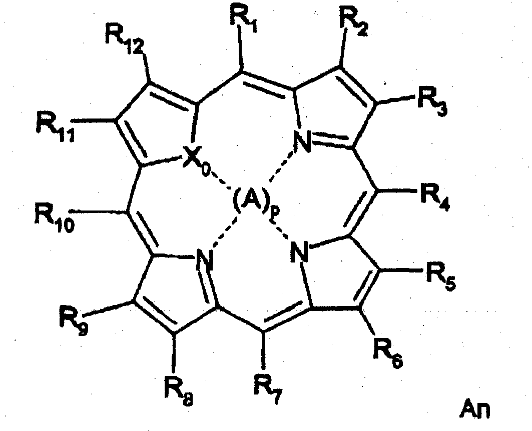 in which:
**the radicals R**₁ **to R**₁₂, which may be identical or different, represent:
• a hydrogen atom;
• a linear or branched C₁-C₃₀ alkyl radical; a linear or branched C₂-C₃₀ alkenyl radical; a linear or branched C₂-C₃₀ alkynyl radical;
• the said alkyl, alkenyl and alkynyl radicals possibly being substituted with at least one monovalent group chosen from the following groups, or combinations thereof:
• hydroxyl;
• amino;
• amino substituted with one or two linear or branched C₁-C₁₀ alkyl radicals, which may be identical or different, optionally bearing one or more hydroxyl or linear or branched C₁-C₁₀ alkoxy groups;
• ammonium substituted with one, two or three linear or branched C₁-C₁₀ alkyl radicals, which may be identical or different, optionally bearing one or more hydroxyl or linear or branched C₁-C₁₀ alkoxy groups;
• hydrogenocarbonyl (-COH);
• an optionally substituted 5- or 6-membered heterocycle, comprising one or more heteroatoms, for instance oxygen or nitrogen, optionally bearing at least one cationic charge; the said heterocycle possibly being fused with an aromatic nucleus, which is preferably 6-membered;
• and/or the said alkyl, alkenyl and alkynyl radicals possibly being interrupted with at least one divalent group chosen from the following groups:
• an oxygen atom;
• amino;
• amino substituted with a linear or branched C₁-C₁₀ alkyl radical, optionally bearing one or more hydroxyl or linear or branched C₁-C₁₀ alkoxy groups;
• ammonium substituted with one or two linear or branched C₁-C₁₀ alkyl radicals, which may be identical or different, optionally bearing one or more hydroxyl or linear or branched C₁-C₁₀ alkoxy groups;
• carbonyl (-CO-);
• an optionally substituted 5- or 6-membered heterocycle, comprising one or more heteroatoms, for instance oxygen or nitrogen, optionally bearing at least one cationic charge;
• an optionally substituted C₆-C₃₀ aryl radical; an optionally substituted (C₁-C₃₀)alkyl(C₆-C₃₀)aryl radical; an optionally substituted (C₆-C₃₀)aryl(C₁-C₃₀)alkyl radical;
• a hydroxyl group;
• an optionally substituted linear or branched C₁-C₃₀ alkoxy radical;
• an amino radical; an amino radical bearing one or more optionally substituted linear or branched C₁-C₃₀ alkyl radicals;
• a radical -SO₂-NH₂, -SO₂NH-alkyl or -NH-SO₂-alkyl, in which the alkyl radical is an optionally substituted linear or branched C₁-C₃₀ alkyl;
• an optionally cationic, preferably aromatic, 5- or 6-membered heterocyclic radical, comprising at least one nitrogen atom, and optionally at least one other heteroatom, which may be identical or different; the said heterocyclic radical being optionally substituted;
• at least one of the radicals R₁ to R₁₂ bears at least one cationic charge;
**X₀** represents a nitrogen atom or an oxygen atom;
**A** represents a metal or metal ion from column IA or IIA of the Periodic Table of the Elements, such as sodium, potassium, magnesium or calcium; zinc or silicon;
**p** is 0, 1 or 2 depending on the nature of the element A;
**An** representing one or more cosmetically acceptable anions that compensate the total cationic charge of the compound.

3. Use according to either of the preceding claims, **characterized in that** the cationic compound corresponds to formula (2) below, and the tautomeric forms thereof, bearing at least one cationic charge: 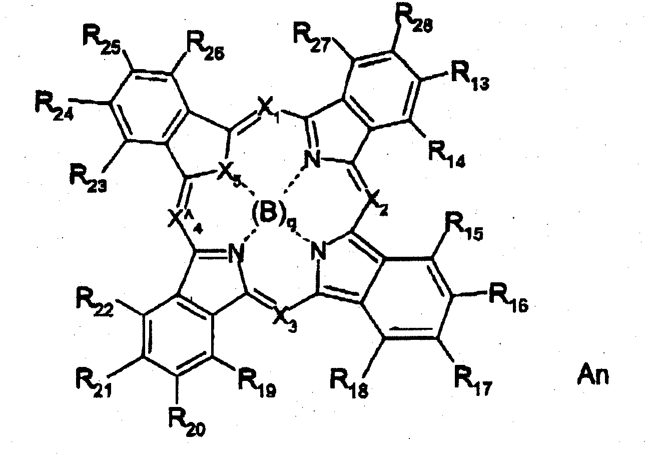 in which:
**the radicals R₁₃ to R₂₈**, which may be identical or different, represent:
• a hydrogen atom;
• a linear or branched C₁-C₃₀ alkyl radical; a linear or branched C₂-C₃₀ alkenyl radical; a linear or branched C₂-C₃₀ alkynyl radical;
• the said alkyl, alkenyl and alkynyl radicals possibly being substituted with at least one monovalent group chosen from the following groups, or combinations thereof:
• hydroxyl;
• amino;
• amino substituted with one or two linear or branched C₁-C₁₀ alkyl radicals, which may be identical or different, optionally bearing one or more hydroxyl or linear or branched C₁-C₁₀ alkoxy groups;
• ammonium substituted with one, two or three linear or branched C₁-C₁₀ alkyl radicals, which may be identical or different, optionally bearing one or more hydroxyl or linear or branched C₁-C₁₀ alkoxy groups;
• hydrogenocarbonyl (-COH);
• an optionally substituted 5- or 6-membered heterocycle, comprising one or more heteroatoms, for instance oxygen or nitrogen, optionally bearing at least one cationic charge; the said heterocycle possibly being fused with an aromatic nucleus, which is preferably 6-membered;
• and/or the said alkyl, alkenyl and alkynyl radicals possibly being interrupted with at least one divalent group chosen from the following groups:
• an oxygen atom;
• amino;
• amino substituted with a linear or branched C₁-C₁₀ alkyl radical, optionally bearing one or more hydroxyl or linear or branched C₁-C₁₀ alkoxy groups;
• ammonium substituted with one or two linear or branched C₁-C₁₀ alkyl radicals, which may be identical or different, optionally bearing one or more hydroxyl or linear or branched C₁-C₁₀ alkoxy groups;
• carbonyl (-CO-);
• an optionally substituted 5- or 6-membered heterocycle, comprising one or more heteroatoms, for instance oxygen or nitrogen, optionally bearing at least one cationic charge;
• an optionally substituted C₆-C₃₀ aryl radical; an optionally substituted (C₁-C₃₀)alkyl(C₆-C₃₀)aryl radical; an optionally substituted (C₆-C₃₀)aryl(C₁-C₃₀) alkyl radical;
• a hydroxyl group;
• an optionally substituted linear or branched C₁-C₃₀ alkoxy radical;
• an amino radical; an amino radical bearing one or more optionally substituted linear or branched C₁-C₃₀ alkyl radicals;
• a radical -SO₂-NH₂, -SO₂NH-alkyl or -NH-SO₂-alkyl, in which the alkyl radical is an optionally substituted linear or branched C₁-C₃₀ alkyl;
• an optionally cationic, preferably aromatic, 5- or 6-membered heterocyclic radical, comprising at least one nitrogen atom, and optionally at least one other heteroatom, which may be identical or different; the said heterocyclic radical being optionally substituted;
• at least one of the radicals R₁₃ to R₂₈ bears at least one cationic charge;
**X₁ to X₄**, which may be identical or different, represent a nitrogen atom or a group -CR₂₉, with R₂₉ having the same definition as the abovementioned R₁₃ to R₂₈;
**X₅** represents a nitrogen atom, an oxygen atom or a sulfur atom;
**B** represents a metal or metal ion from column IA or IIA of the Periodic Table of the Elements, such as sodium, potassium, magnesium or calcium; zinc or silicon;
**q** is 0, 1 or 2 depending on the nature of the element B;
**An** representing one or more cosmetically acceptable anions that compensate the total cationic charge of the compound.

4. Use according to any one of the preceding claims, **characterized in that** the non-metallic cationic compound is used in the presence of at least one oxidizing agent.

5. Use according to any one of the preceding claims, **characterized in that** the non-metallic cationic compound is used in the absence of an oxidizing agent.

6. Dye composition comprising, in a medium that is suitable for dyeing human keratin materials, at least one non-metallic cationic or metallic cationic compound of porphyrin or phthalocyanin type comprising, as metal element, only one or more metals or metal ions from column IA or IIA of the Periodic Table of the Elements, zinc and silicon, as direct dye for dyeing human keratin materials, and especially fibres, and at least one polymer and/or at least one surfactant.

7. Composition according to Claim 6, **characterized in that** the cationic compound corresponds to formula (1) below, and the tautomeric forms thereof, bearing at least one cationic charge: 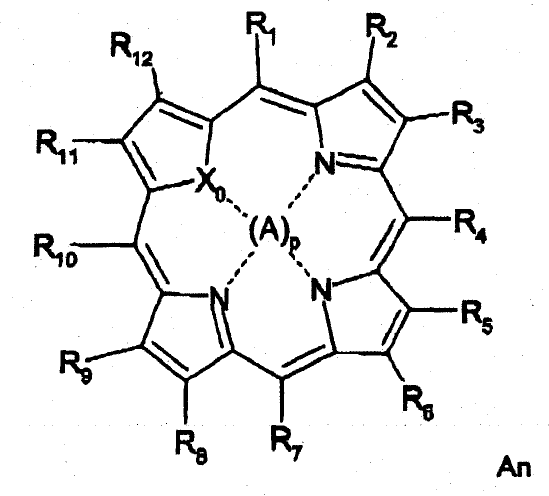 in which:
**the radicals R₁ to R₁₂**, which may be identical or different, represent:
• a hydrogen atom;
• a linear or branched C₁-C₃₀ alkyl radical; a linear or branched C₂-C₃₀ alkenyl radical; a linear or branched C₂-C₃₀ alkynyl radical;
• the said alkyl, alkenyl and alkynyl radicals possibly being substituted with at least one monovalent group chosen from the following groups, or combinations thereof:
• hydroxyl;
• amino;
• amino substituted with one or two linear or branched C₁-C₁₀ alkyl radicals, which may be identical or different, optionally bearing one or more hydroxyl or linear or branched C₁-C₁₀ alkoxy groups;
• ammonium substituted with one, two or three linear or branched C₁-C₁₀ alkyl radicals, which may be identical or different, optionally bearing one or more hydroxyl or linear or branched C₁-C₁₀ alkoxy groups;
• hydrogenocarbonyl (-COH);
• an optionally substituted 5- or 6-membered heterocycle, comprising one or more heteroatoms, for instance oxygen or nitrogen, optionally bearing at least one cationic charge; the said heterocycle possibly being fused with an aromatic nucleus, which is preferably 6-membered;
• and/or the said alkyl, alkenyl and alkynyl radicals possibly being interrupted with one or more divalent groups chosen from the following groups:
• an oxygen atom;
• amino;
• amino substituted with a linear or branched C₁-C₁₀ alkyl radical, optionally bearing one or more hydroxyl or linear or branched C₁-C₁₀ alkoxy groups;
• ammonium substituted with one or two linear or branched C₁-C₁₀ alkyl radicals, which may be identical or different, optionally bearing one or more hydroxyl or linear or branched C₁-C₁₀ alkoxy groups;
• carbonyl (-CO-);
• an optionally substituted 5- or 6-membered heterocycle, comprising one or more heteroatoms, for instance oxygen or nitrogen, optionally bearing at least one cationic charge;
• an optionally substituted C₆-C₃₀ aryl radical; an optionally substituted (C₁-C₃₀) alkyl (C₆-C₃₀)aryl radical; an optionally substituted (C₆-C₃₀)aryl(C₁-C₃₀)alkyl radical;
• a hydroxyl group;
• an optionally substituted linear or branched C₁-C₃₀ alkoxy radical;
• an amino radical; an amino radical bearing one or more optionally substituted linear or branched C₁-C₃₀ alkyl radicals;
• a radical -SO₂-NH₂, -SO₂NH-alkyl or -NH-SO₂-alkyl, in which the alkyl radical is an optionally substituted linear or branched C₁-C₃₀ alkyl;
• an optionally cationic, preferably aromatic, 5- or 6-membered heterocyclic radical, comprising at least one nitrogen atom, and optionally at least one other heteroatom, which may be identical or different; the said heterocyclic radical being optionally substituted;
• at least one of the radicals R₁ to R₁₂ bears at least one cationic charge;
**X**₀ represents a nitrogen atom or an oxygen atom;
**A** represents a metal or metal ion from column IA or IIA of the Periodic Table of the Elements, such as sodium, potassium, magnesium or calcium; zinc or silicon;
**p** is 0, 1 or 2 depending on the nature of the element A;
**An** representing one or more cosmetically acceptable anions that compensate the total cationic charge of the compound.

8. Composition according to either of Claims 6 and 7, **characterized in that** the cationic compound corresponds to the following formula, and the tautomeric forms thereof, bearing at least one cationic charge: 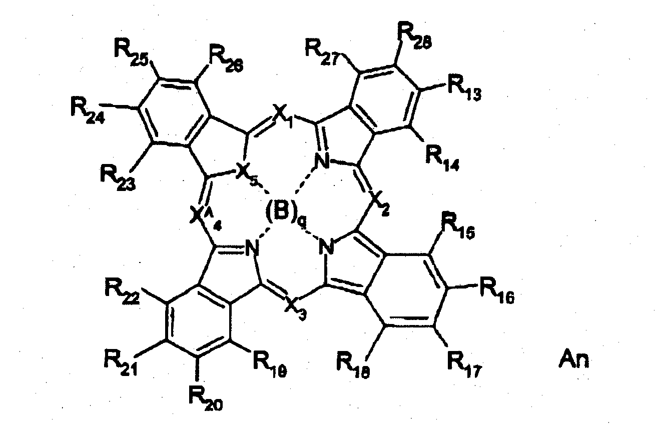 in which:
**the radicals R₁₃ to R₂₈**, which may be identical or different, represent:
• a hydrogen atom;
• a linear or branched C₁-C₃₀ alkyl radical; a linear or branched C₂-C₃₀ alkenyl radical; a linear or branched C₂-C₃₀ alkynyl radical;
• the said alkyl, alkenyl and alkynyl radicals possibly being substituted with at least one monovalent group chosen from the following groups, or combinations thereof:
• hydroxyl;
• amino;
• amino substituted with one or two linear or branched C₁-C₁₀ alkyl radicals, which may be identical or different, optionally bearing one or more hydroxyl or linear or branched C₁-C₁₀ alkoxy groups;
• ammonium substituted with one, two or three linear or branched C₁-C₁₀ alkyl radicals, which may be identical or different, optionally bearing one or more hydroxyl or linear or branched C₁-C₁₀ alkoxy groups;
• hydrogenocarbonyl (-COH);
• an optionally substituted 5- or 6-membered heterocycle, comprising one or more heteroatoms, for instance oxygen or nitrogen, optionally bearing at least one cationic charge; the said heterocycle possibly being fused with an aromatic nucleus, which is preferably 6-membered;
• and/or the said alkyl, alkenyl and alkynyl radicals possibly being interrupted with one or more divalent groups chosen from the following groups:
• an oxygen atom;
• amino;
• amino substituted with a linear or branched C₁-C₁₀ alkyl radical, optionally bearing one or more hydroxyl or linear or branched C₁-C₁₀ alkoxy groups;
• ammonium substituted with one or two linear or branched C₁-C₁₀ alkyl radicals, which may be identical or different, optionally bearing one or more hydroxyl or linear or branched C₁-C₁₀ alkoxy groups;
• carbonyl (-CO-);
• an optionally substituted 5- or 6-membered heterocycle, comprising one or more heteroatoms, for instance oxygen or nitrogen, optionally bearing at least one cationic charge;
• an optionally substituted C₆-C₃₀ aryl radical; an optionally substituted (C₁-C₃₀)alkyl(C₆-C₃₀)aryl radical; an optionally substituted (C₆-C₃₀)aryl(C₁-C₃₀)alkyl radical;
• a hydroxyl group;
• an optionally substituted linear or branched C₁-C₃₀ alkoxy radical;
• an amino radical; an amino radical bearing one or more optionally substituted linear or branched C₁-C₃₀ alkyl radicals;
• a radical -SO₂-NH₂, -SO₂NH-alkyl or -NH-SO₂-alkyl, in which the alkyl radical is an optionally substituted linear or branched C₁-C₃₀ alkyl;
• an optionally cationic, preferably aromatic, 5- or 6₋membered heterocyclic radical, comprising at least one nitrogen atom, and optionally at least one other heteroatom, which may be identical or different; the said heterocyclic radical being optionally substituted;
• at least one of the radicals R₁₃ to R₂₈ bears at least one cationic charge;
**X₁ to X₄,** which may be identical or different, represent a nitrogen atom or a group -CR₂₉, with R₂₉ having the same definition as the abovementioned R₁₃ to R₂₈;
**X₅** represents a nitrogen atom, an oxygen atom or a sulfur atom;
**B** represents a metal or metal ion from column IA or IIA of the Periodic Table of the Elements, such as sodium, potassium, magnesium or calcium; zinc or silicon;
**q** is 0, 1 or 2 depending on the nature of the element B;
**An** representing one or more cosmetically acceptable anions that compensate the total cationic charge of the compound.

9. Composition according to either of Claims 7 and 8, **characterized in that** the content of cationic compound represents from 0.0005% to 20% by weight relative to the weight of the dye composition.

10. Composition according to any one of Claims 7 to 9, **characterized in that** the surfactant(s) is (are) of nonionic, anionic, cationic or amphoteric nature.

11. Composition according to the preceding claim, **characterized in that** the content of surfactants ranges from 0.001% to 30% by weight relative to the weight of the dye composition.

12. Composition according to any one of Claims 7 to 11, **characterized in that** the polymer is of nonionic, cationic, anionic or amphoteric nature.

13. Composition according to any one of Claims 7 to 12, **characterized in that** the polymer is an associative or non-associative thickening polymer.

14. Composition according to the preceding claim, **characterized in that** the content of associative or non-associative thickening polymer(s) ranges from 0.01% to 10% by weight and more particularly from 0.1% to 5% by weight relative to the weight of the dye composition.

15. Composition according to any one of Claims 7 to 14, **characterized in that** the polymer is a conditioning polymer or a fixing polymer.

16. Composition according to the preceding claim, **characterized in that** the content of conditioning polymer or of fixing polymer ranges from 0.01% to 20% by weight relative to the weight of the dye composition, and preferably from 0.1% to 5% by weight relative to the weight of the dye composition.

17. Composition according to any one of Claims 7 to 16, **characterized in that** it comprises at least one additional direct dye other than the non-metallic cationic compound.

18. Composition according to the preceding claim, **characterized in that** the content of additional direct dye represents from 0.0005% to 12% by weight relative to the weight of the composition.

19. Composition according to any one of Claims 7 to 18, **characterized in that** it comprises at least one oxidation base, optionally combined with at least one coupler.

20. Composition according to the preceding claim, **characterized in that** the content of oxidation base represents, for each, from 0.0005% to 12% by weight relative to the total weight of the dye composition.

21. Composition according to Claim 19, **characterized in that** the content of coupler represents, for each, from 0.0001% to 10% by weight relative to the total weight of the dye composition.

22. Composition according to any one of Claims 7 to 21, comprising at least one oxidizing agent.

23. Ready-to-use composition comprising, in a medium that is suitable for dyeing human keratin materials, at least one non-metallic cationic or metallic cationic compound of porphyrin or phthalocyanin type comprising as metal element only one or more metals or metal ions from column IA or IIA of the Periodic Table of the Elements, zinc and silicon, and at least one oxidizing agent.

24. Process for dyeing human keratin materials, and especially fibres, which consists in applying the composition according to any one of Claims 7 to 21 to keratin materials, preferably wet or dry fibres, without final rinsing of the composition.

25. Process for dyeing human keratin materials, and especially fibres, which consists in applying the composition according to any one of Claims 7 to 22 to wet or dry keratin materials, in the presence of at least one oxidizing agent, leaving the composition to act for a time that is sufficient to obtain the desired coloration, and then removing the composition.

26. Multi-compartment device for implementing the process according to Claim 25, for dyeing keratin materials and especially fibres, comprising one or more compartments with, in a medium that is suitable for dyeing human keratin fibres, at least one non-metallic cationic or metallic cationic compound of porphyrin or phthalocyanin type comprising as metal element only one or more metals or metal ions from column IA or IIA of the Periodic Table of the Elements, zinc and silicon, optionally at least one additional direct dye, optionally at least one oxidation base and/or at least one coupler; these compounds being present in one or more compositions, which may or may not be combined, and, moreover, a compartment with a composition comprising, in a medium that is suitable for dyeing human keratin fibres, at least one oxidizing agent.

27. Compound having the following formula:

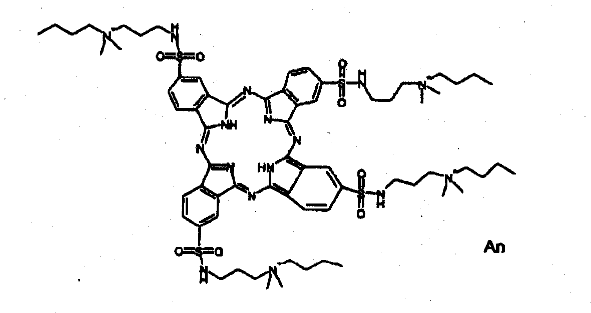

## Patentansprüche

1. Verwendung mindestens einer Verbindung vom Typ der kationischen nichtmetallischen oder kationischen metallischen Porphyrine oder Phthalocyanine, die als metallisches Element nur ein oder mehrere Metalle oder Metallionen der Gruppe IA, IIA des Periodensystems der Elemente, Zink oder Silicium enthalten, als Direktfarbstoff zum Färben von menschlichen Keratinsubstanzen und insbesondere Fasern.

2. Verwendung nach dem vorhergehenden Anspruch, **dadurch gekennzeichnet, dass** die kationische Verbindung der folgenden Formel (1) und ihren tautomeren Formen entspricht, die mindestens eine kationische Ladung aufweisen: 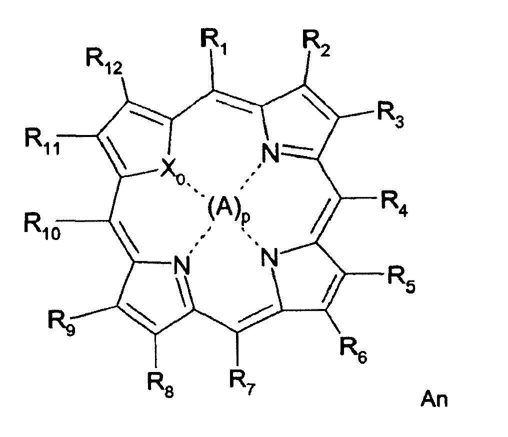 in der Formel:
**die Gruppen R₁ bis R₁₂,** die gleich oder verschieden sind, bedeuten:
■ ein Wasserstoffatom;
■ eine geradkettige oder verzweigte C₁₋₃₀-Alkylgruppe; eine geradkettige oder verzweigte C₂₋₃₀-Alkenylgruppe; eine geradkettige oder verzweigte C₂₋₃₀-Alkinylgruppe;
■ wobei die Alkylgruppen, Alkenylgruppen und Alkinylgruppen gegebenenfalls mit mindestens einer einwertigen Gruppe substituiert sein können, die unter den folgenden Gruppen oder ihren Kombinationen ausgewählt sind:
■ Hydroxy,
■ Amino;
■ einer Aminogruppe, die mit einer oder zwei, gleichen oder verschiedenen C₁₋₁₀-Alkylgruppen substituiert ist, die gegebenenfalls eine oder mehrere Hydroxygruppen oder geradkettige oder verzweigte C₁₋₁₀-Alkoxygruppen aufweisen;
■ einer Ammoniumgruppe, die mit einer, zwei oder drei, gleichen oder verschiedenen C₁₋₁₀-Alkylgruppen substituiert ist, die gegebenenfalls eine oder mehrere Hydroxygruppen oder geradkettige oder verzweigte C₁₋₁₀-Alkoxygruppen aufweisen;
■ Formyl (-COH);
■ einem 5- oder 6-gliedrigen Heterocyclus, der ein oder mehrere Heteroatome enthält, wie ein Sauerstoffatom oder ein gegebenenfalls substituiertes Stickstoffatom, und gegebenenfalls mindestens eine kationische Ladung aufweist; wobei der Heterocyclus mit einem vorzugsweise 6-gliedrigen, aromatischen Ring kondensiert sein kann;
■ und/oder wobei die Alkylgruppen, Alkenylgruppen und Alkinylgruppen gegebenenfalls durch mindestens eine zweiwertige Gruppe unterbrochen sein können, die unter den folgenden Gruppen ausgewählt ist:
■ einem Sauerstoffatom;
■ Amino;
■ einer Aminogruppe, die mit einer geradkettigen oder verzweigten C₁₋₁₀-Alkylgruppe substituiert ist, die gegebenenfalls eine oder mehrere Hydroxygruppen oder geradkettige oder verzweigte C₁₋₁₀-Alkoxygruppen aufweist;
■ einer Ammoniumgruppe, die mit einer oder zwei, gleichen oder verschiedenen, geradkettigen oder verzweigten C₁₋₁₀-Alkylgruppen substituiert ist, die gegebenenfalls eine oder mehrere Hydroxygruppen oder geradkettige oder verzweigte C₁₋₁₀-Alkoxygruppen aufweisen;
■ Carbonyl (-CO-);
■ einem 5- oder 6-gliedrigen Heterocyclus, der ein oder mehrere Heteroatome enthält, wie ein Sauerstoffatom oder ein gegebenenfalls substituiertes Stickstoffatom, und gegebenenfalls mindestens eine kationische Ladung aufweist;
■ eine gegebenenfalls substituierte C₆₋₃₀-Arylgruppe; eine gegebenenfalls substituierte Alkyl(C₁₋₃₀)aryl(C₆₋₃₀)gruppe; eine gegebenenfalls substituierte Aryl(C₆₋₃₀)alkyl(C₁₋₃₀)gruppe;
■ eine Hydroxygruppe;
■ eine geradkettige oder verzweigte, gegebenenfalls substituierte C₁₋₃₀-Alkoxygruppe;
■ eine Aminogruppe; eine Aminogruppe, die eine oder mehrere geradkettige oder verzweigte, gegebenenfalls substituierte C₁₋₃₀-Alkylgruppen trägt;
■ eine Gruppe -SO₂-NH₂, -SO₂NH-alkyl, -NH-SO₂-alkyl, bei denen die geradkettige oder verzweigte, gegebenenfalls substituierte Alkylgruppe 1 bis 30 Kohlenstoffatome aufweist;
■ eine gegebenenfalls kationische, 5- bis 6-gliedrige heterocyclische Gruppe, die vorzugsweise aromatisch ist und die mindestens ein Stickstoffatom und gegebenenfalls mindestens ein weiteres Heteroatom enthält, die gleich oder verschieden sind; wobei die heterocyclische Gruppe gegebenenfalls substituiert ist;
■ wobei mindestens eine der Gruppen R₁ bis R₁₂ mindestens eine kationische Ladung aufweist;
**X₀** bedeutet ein Stickstoffatom oder ein Sauerstoffatom;
**A** bedeutet ein Metall oder Metallion der Gruppe IA, IIA des Periodensystems der Elemente, wie Natrium, Kalium, Magnesium, Calcium; Zink oder Silicium;
**p** ist in Abhängigkeit von der Art des Elements A 0 oder 1 oder 2;
**An** bedeutet ein oder mehrere kosmetisch akzeptable Anionen, die die kationische Gesamtladung der Verbindung ausgleichen.

3. Verwendung nach einem der vorhergehenden Ansprüche, **dadurch gekennzeichnet, dass** die kationische Verbindung der folgenden Formel (2) und ihren tautomeren Formen entspricht, die mindestens eine kationische Ladung aufweisen: 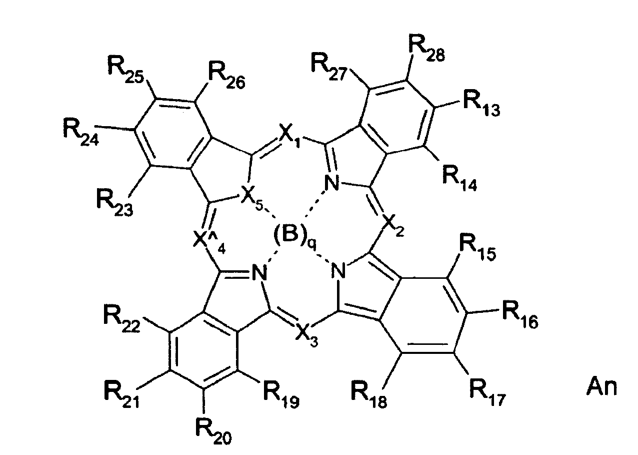 in der Formel:
**die Gruppen R₁₃ bis R₂₈,** die gleich oder verschieden sind, bedeuten:
■ ein Wasserstoffatom;
■ eine geradkettige oder verzweigte C₁₋₃₀-Alkylgruppe; eine geradkettige oder verzweigte C₂₋₃₀-Alkenylgruppe; eine geradkettige oder verzweigte C₂₋₃₀-Alkinylgruppe;
■ wobei die Alkylgruppen, Alkenylgruppen und Alkinylgruppen gegebenenfalls mit mindestens einer einwertigen Gruppe substituiert sein können, die unter den folgenden Gruppen oder ihren Kombinationen ausgewählt sind:
■ Hydroxy,
■ Amino;
■ einer Aminogruppe, die mit einer oder zwei, gleichen oder verschiedenen C₁₋₁₀-Alkylgruppen substituiert ist, die gegebenenfalls eine oder mehrere Hydroxygruppen oder geradkettige oder verzweigte C₁₋₁₀-Alkoxygruppen aufweisen;
■ einer Ammoniumgruppe, die mit einer, zwei oder drei, gleichen oder verschiedenen C₁₋₁₀-Alkylgruppen substituiert ist, die gegebenenfalls eine oder mehrere Hydroxygruppen oder geradkettige oder verzweigte C₁₋₁₀-Alkoxygruppen aufweisen;
■ Formyl (-COH);
■ einem 5- oder 6-gliedrigen Heterocyclus, der ein oder mehrere Heteroatome enthält, wie ein Sauerstoffatom oder ein gegebenenfalls substituiertes Stickstoffatom, und gegebenenfalls mindestens eine kationische Ladung aufweist; wobei der Heterocyclus mit einem vorzugsweise 6-gliedrigen, aromatischen Ring kondensiert sein kann;
■ und/oder wobei die Alkylgruppen, Alkenylgruppen und Alkinylgruppen gegebenenfalls durch mindestens eine zweiwertige Gruppe unterbrochen sein können, die unter den folgenden Gruppen ausgewählt ist:
■ einem Sauerstoffatom;
■ Amino;
■ einer Aminogruppe, die mit einer geradkettigen oder verzweigten C₁₋₁₀-Alkylgruppe substituiert ist, die gegebenenfalls eine oder mehrere Hydroxygruppen oder geradkettige oder verzweigte C₁₋₁₀-Alkoxygruppen aufweist;
■ einer Ammoniumgruppe, die mit einer oder zwei, gleichen oder verschiedenen, geradkettigen oder verzweigten C₁₋₁₀-Alkylgruppen substituiert ist, die gegebenenfalls eine oder mehrere Hydroxygruppen oder geradkettige oder verzweigte C₁₋₁₀-Alkoxygruppen aufweisen;
■ Carbonyl (-CO-);
■ einem 5- oder 6-gliedrigen Heterocyclus, der ein oder mehrere Heteroatome enthält, wie ein Sauerstoffatom oder ein gegebenenfalls substituiertes Stickstoffatom, und gegebenenfalls mindestens eine kationische Ladung aufweist;
■ eine gegebenenfalls substituierte C₆₋₃₀-Arylgruppe; eine gegebenenfalls substituierte Alkyl(C₁₋₃₀)aryl(C₆₋₃₀)gruppe; eine gegebenenfalls substituierte Aryl(C₆₋₃₀)alkyl(C₁₋₃₀)gruppe;
■ eine Hydroxygruppe;
■ eine geradkettige oder verzweigte, gegebenenfalls substituierte C₁₋₃₀-Alkoxygruppe;
■ eine Aminogruppe; eine Aminogruppe, die eine oder mehrere geradkettige oder verzweigte, gegebenenfalls substituierte C₁₋₃₀-Alkylgruppen trägt;
■ eine Gruppe -SO₂-NH₂, -SO₂NH-alkyl, -NH-SO₂-alkyl, bei denen die geradkettige oder verzweigte, gegebenenfalls substituierte Alkylgruppe 1 bis 30 Kohlenstoffatome aufweist;
■ eine gegebenenfalls kationische, 5- bis 6-gliedrige heterocyclische Gruppe, die vorzugsweise aromatisch ist und die mindestens ein Stickstoffatom und gegebenenfalls mindestens ein weiteres Heteroatom enthält, die gleich oder verschieden sind; wobei die heterocyclische Gruppe gegebenenfalls substituiert ist;
■ wobei mindestens eine der Gruppen R₁₃ bis R₂₈ mindestens eine kationische Ladung aufweist;
**X₁ bis X₄,** die gleich oder verschieden sind, bedeuten ein Stickstoffatom oder eine Gruppe -CR₂₉, wobei R₂₉ die für R₁₃ bis R₂₈ angegebenen Bedeutungen aufweist;
**X₅** bedeutet ein Stickstoffatom oder ein Sauerstoffatom oder ein Schwefelatom;
**B** bedeutet ein Metall oder Metallion der Gruppe IA, IIA des Periodensystems der Elemente, wie Natrium, Kalium, Magnesium, Calcium; Zink oder Silicium;
**q** ist in Abhängigkeit von der Art des Elements B 0 oder 1 oder 2; **An** bedeutet ein oder mehrere kosmetisch akzeptable Anionen, die die kationische Gesamtladung der Verbindung ausgleichen.

4. Verwendung nach einem der vorhergehenden Ansprüche, **dadurch gekennzeichnet, dass** die nichtmetallische kationische Verbindung in Gegenwart mindestens eines Oxidationsmittels verwendet wird.

5. Verwendung nach einem der vorhergehenden Ansprüche, **dadurch gekennzeichnet, dass** die nichtmetallische kationische Verbindung ohne ein Oxidationsmittel verwendet wird.

6. Farbmittelzusammensetzung, die in einem zum Färben von menschlichen Keratinsubstanzen geeigneten Medium mindestens eine Verbindung vom Typ der kationischen nichtmetallischen oder kationischen metallischen Porphyrine oder Phthalocyanine, die als metallisches Element nur ein oder mehrere Metalle oder Metallionen der Gruppe IA, IIA des Periodensystems der Elemente, Zink oder Silicium enthalten, als Direktfarbstoff zum Färben von menschlichen Keratinsubstanzen und insbesondere Keratinfasern und mindestens ein Polymer und/oder mindestens einen grenzflächenaktiven Stoff enthält.

7. Zusammensetzung nach Anspruch 6, **dadurch gekennzeichnet, dass** die kationische Verbindung der folgenden Formel (1) und ihren tautomeren Formen entspricht, die mindestens eine kationische Ladung aufweisen: 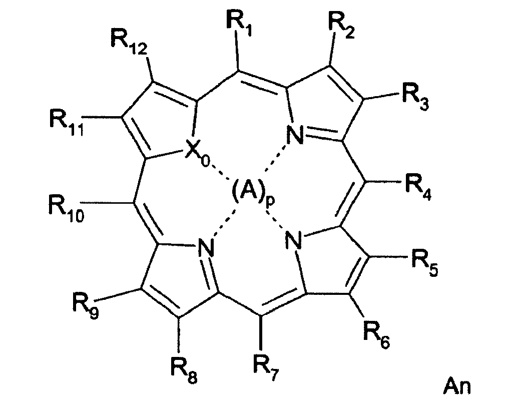 in der Formel:
**die Gruppen R₁ bis R₁₂,** die gleich oder verschieden sind, bedeuten:
■ ein Wasserstoffatom;
■ eine geradkettige oder verzweigte C₁₋₃₀-Alkylgruppe; eine geradkettige oder verzweigte C₂₋₃₀-Alkenylgruppe; eine geradkettige oder verzweigte C₂₋₃₀-Alkinylgruppe;
■ wobei die Alkylgruppen, Alkenylgruppen und Alkinylgruppen gegebenenfalls mit mindestens einer einwertigen Gruppe substituiert sein können, die unter den folgenden Gruppen oder ihren Kombinationen ausgewählt sind:
■ Hydroxy,
■ Amino;
■ einer Aminogruppe, die mit einer oder zwei, gleichen oder verschiedenen C₁₋₁₀-Alkylgruppen substituiert ist, die gegebenenfalls eine oder mehrere Hydroxygruppen oder geradkettige oder verzweigte C₁₋₁₀-Alkoxygruppen aufweisen;
■ einer Ammoniumgruppe, die mit einer, zwei oder drei, gleichen oder verschiedenen C₁₋₁₀-Alkylgruppen substituiert ist, die gegebenenfalls eine oder mehrere Hydroxygruppen oder geradkettige oder verzweigte C₁₋₁₀-Alkoxygruppen aufweisen;
■ Formyl (-COH);
■ einem 5- oder 6-gliedrigen Heterocyclus, der ein oder mehrere Heteroatome enthält, wie ein Sauerstoffatom oder ein gegebenenfalls substituiertes Stickstoffatom, und gegebenenfalls mindestens eine kationische Ladung aufweist; wobei der Heterocyclus mit einem vorzugsweise 6-gliedrigen, aromatischen Ring kondensiert sein kann;
■ und/oder wobei die Alkylgruppen, Alkenylgruppen und Alkinylgruppen gegebenenfalls durch mindestens eine zweiwertige Gruppe unterbrochen sein können, die unter den folgenden Gruppen ausgewählt ist:
■ einem Sauerstoffatom;
■ Amino;
■ einer Aminogruppe, die mit einer geradkettigen oder verzweigten C₁₋₁₀-Alkylgruppe substituiert ist, die gegebenenfalls eine oder mehrere Hydroxygruppen oder geradkettige oder verzweigte C₁₋₁₀-Alkoxygruppen aufweist;
■ einer Ammoniumgruppe, die mit einer oder zwei, gleichen oder verschiedenen, geradkettigen oder verzweigten C₁₋₁₀-Alkylgruppen substituiert ist, die gegebenenfalls eine oder mehrere Hydroxygruppen oder geradkettige oder verzweigte C₁₋₁₀-Alkoxygruppen aufweisen;
■ Carbonyl (-CO-);
■ einem 5- oder 6-gliedrigen Heterocyclus, der ein oder mehrere Heteroatome enthält, wie ein Sauerstoffatom oder ein gegebenenfalls substituiertes Stickstoffatom, und gegebenenfalls mindestens eine kationische Ladung aufweist;
■ eine gegebenenfalls substituierte C₆₋₃₀-Arylgruppe; eine gegebenenfalls substituierte Alkyl(C₁₋₃₀)aryl(C₆₋₃₀)gruppe; eine gegebenenfalls substituierte Aryl(C₆₋₃₀)alkyl(C₁₋₃₀)gruppe;
■ eine Hydroxygruppe;
■ eine geradkettige oder verzweigte, gegebenenfalls substituierte C₁₋₃₀-Alkoxygruppe;
■ eine Aminogruppe; eine Aminogruppe, die eine oder mehrere geradkettige oder verzweigte, gegebenenfalls substituierte C₁₋₃₀-Alkylgruppen trägt;
■ eine Gruppe -SO₂-NH₂, -SO₂NH-alkyl, -NH-SO₂-alkyl, bei denen die geradkettige oder verzweigte, gegebenenfalls substituierte Alkylgruppe 1 bis 30 Kohlenstoffatome aufweist;
■ eine gegebenenfalls kationische, 5- bis 6-gliedrige heterocyclische Gruppe, die vorzugsweise aromatisch ist und die mindestens ein Stickstoffatom und gegebenenfalls mindestens ein weiteres Heteroatom enthält, die gleich oder verschieden sind; wobei die heterocyclische Gruppe gegebenenfalls substituiert ist;
■ wobei mindestens eine der Gruppen R₁ bis R₁₂ mindestens eine kationische Ladung aufweist;
**X₀** bedeutet ein Stickstoffatom oder ein Sauerstoffatom;
**A** bedeutet ein Metall oder Metallion der Gruppe IA, IIA des Periodensystems der Elemente, wie Natrium, Kalium, Magnesium, Calcium; Zink oder Silicium;
**p** ist in Abhängigkeit von der Art des Elements A 0 oder 1 oder 2; **An** bedeutet ein oder mehrere kosmetisch akzeptable Anionen, die die kationische Gesamtladung der Verbindung ausgleichen.

8. Zusammensetzung nach einem der Ansprüche 6 oder 7, **dadurch gekennzeichnet, dass** die kationische Verbindung der folgenden Formel und ihren tautomeren Formen entspricht, die mindestens eine kationische Ladung aufweisen: 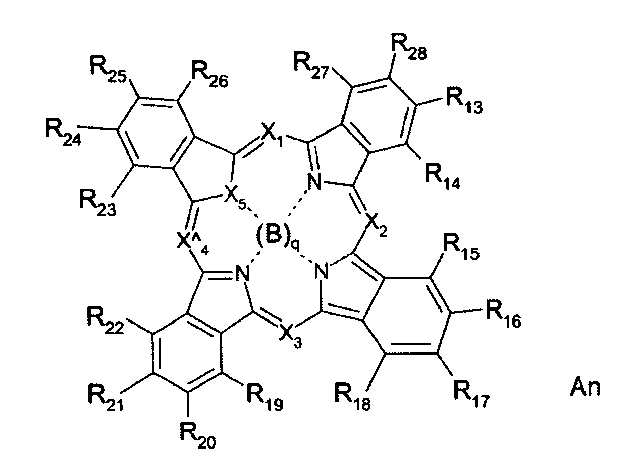 in der Formel:
**die Gruppen R₁₃ bis R₂₈,** die gleich oder verschieden sind, bedeuten:
■ ein Wasserstoffatom;
■ eine geradkettige oder verzweigte C₁₋₃₀-Alkylgruppe; eine geradkettige oder verzweigte C₂₋₃₀-Alkenylgruppe; eine geradkettige oder verzweigte C₂₋₃₀-Alkinylgruppe;
■ wobei die Alkylgruppen, Alkenylgruppen und Alkinylgruppen gegebenenfalls mit mindestens einer einwertigen Gruppe substituiert sein können, die unter den folgenden Gruppen oder ihren Kombinationen ausgewählt sind:
■ Hydroxy,
■ Amino;
■ einer Aminogruppe, die mit einer oder zwei, gleichen oder verschiedenen C₁₋₁₀-Alkylgruppen substituiert ist, die gegebenenfalls eine oder mehrere Hydroxygruppen oder geradkettige oder verzweigte C₁₋₁₀-Alkoxygruppen aufweisen;
■ einer Ammoniumgruppe, die mit einer, zwei oder drei, gleichen oder verschiedenen C₁₋₁₀-Alkylgruppen substituiert ist, die gegebenenfalls eine oder mehrere Hydroxygruppen oder geradkettige oder verzweigte C₁₋₁₀-Alkoxygruppen aufweisen;
■ Formyl (-COH);
■ einem 5- oder 6-gliedrigen Heterocyclus, der ein oder mehrere Heteroatome enthält, wie ein Sauerstoffatom oder ein gegebenenfalls substituiertes Stickstoffatom, und gegebenenfalls mindestens eine kationische Ladung aufweist; wobei der Heterocyclus mit einem vorzugsweise 6-gliedrigen, aromatischen Ring kondensiert sein kann;
■ und/oder wobei die Alkylgruppen, Alkenylgruppen und Alkinylgruppen gegebenenfalls durch mindestens eine zweiwertige Gruppe unterbrochen sein können, die unter den folgenden Gruppen ausgewählt ist:
■ einem Sauerstoffatom;
■ Amino;
■ einer Aminogruppe, die mit einer geradkettigen oder verzweigten C₁₋₁₀-Alkylgruppe substituiert ist, die gegebenenfalls eine oder mehrere Hydroxygruppen oder geradkettige oder verzweigte C₁₋₁₀-Alkoxygruppen aufweist;
■ einer Ammoniumgruppe, die mit einer oder zwei, gleichen oder verschiedenen, geradkettigen oder verzweigten C₁₋₁₀-Alkylgruppen substituiert ist, die gegebenenfalls eine oder mehrere Hydroxygruppen oder geradkettige oder verzweigte C₁₋₁₀-Alkoxygruppen aufweisen;
■ Carbonyl (-CO-);
■ einem 5- oder 6-gliedrigen Heterocyclus, der ein oder mehrere Heteroatome enthält, wie ein Sauerstoffatom oder ein gegebenenfalls substituiertes Stickstoffatom, und gegebenenfalls mindestens eine kationische Ladung aufweist;
■ eine gegebenenfalls substituierte C₆₋₃₀-Arylgruppe; eine gegebenenfalls substituierte Alkyl(C₁₋₃₀)aryl(C₆₋₃₀)gruppe; eine gegebenenfalls substituierte Aryl(C₆₋₃₀)alkyl(C₁₋₃₀)gruppe;
■ eine Hydroxygruppe;
■ eine geradkettige oder verzweigte, gegebenenfalls substituierte C₁₋₃₀-Alkoxygruppe;
■ eine Aminogruppe; eine Aminogruppe, die eine oder mehrere geradkettige oder verzweigte, gegebenenfalls substituierte C₁₋₃₀-Alkylgruppen trägt;
■ eine Gruppe -SO₂-NH₂, -SO₂NH-alkyl, -NH-SO₂-alkyl, bei denen die geradkettige oder verzweigte, gegebenenfalls substituierte Alkylgruppe 1 bis 30 Kohlenstoffatome aufweist;
■ eine gegebenenfalls kationische, 5- bis 6-gliedrige heterocyclische Gruppe, die vorzugsweise aromatisch ist und die mindestens ein Stickstoffatom und gegebenenfalls mindestens ein weiteres Heteroatom enthält, die gleich oder verschieden sind; wobei die heterocyclische Gruppe gegebenenfalls substituiert ist;
■ wobei mindestens eine der Gruppen R₁₃ bis R₂₈ mindestens eine kationische Ladung aufweist;
**X₁ bis X₄,** die gleich oder verschieden sind, bedeuten ein Stickstoffatom oder eine Gruppe -CR₂₉, wobei R₂₉ die für R₁₃ bis R₂₈ angegebenen Bedeutungen aufweist;
**X₅** bedeutet ein Stickstoffatom oder ein Sauerstoffatom oder ein Schwefelatom;
**B** bedeutet ein Metall oder Metallion der Gruppe IA, IIA des Periodensystems der Elemente, wie Natrium, Kalium, Magnesium, Calcium; Zink oder Silicium;
**q** ist in Abhängigkeit von der Art des Elements B 0 oder 1 oder 2; **An** bedeutet ein oder mehrere kosmetisch akzeptable Anionen, die die kationische Gesamtladung der Verbindung ausgleichen.

9. Zusammensetzung nach einem der Ansprüche 7 oder 8, **dadurch gekennzeichnet, dass** der Mengenanteil der kationischen Verbindung 0,0005 bis 20 Gew.-%, bezogen auf das Gesamtgewicht der Farbmittelzusammensetzung, ausmacht.

10. Zusammensetzung nach einem der Ansprüche 7 bis 9, **dadurch gekennzeichnet, dass** oder oder die grenzflächenaktiven Stoffe vom nichtionischen, anionischen oder amphoteren Typ sind.

11. Zusammensetzung nach dem vorhergehenden Anspruch, **dadurch gekennzeichnet, dass** der Mengenanteil der grenzflächenaktiven Stoffe im Bereich von 0,001 bis 30 Gew.-%, bezogen auf das Gesamtgewicht der Farbmittelzusammensetzung, liegt.

12. Zusammensetzung nach einem der Ansprüche 7 bis 11, **dadurch gekennzeichnet, dass** das Polymer vom nichtionischen, kationischen, anionischen oder amphoteren Typ ist.

13. Zusammensetzung nach einem der Ansprüche 7 bis 12, **dadurch gekennzeichnet, dass** es sich bei dem Polymer um ein verdickendes, assoziatives oder nichtassoziatives Polymer handelt.

14. Zusammensetzung nach dem vorhergehenden Anspruch, **dadurch gekennzeichnet, dass** der Mengenanteil des (der) verdickenden, assoziativen oder nichtassoziativen Polymers (Polymere) im Bereich von 0,01 bis 10 Gew.-% und insbesondere 0,1 bis 5 Gew.-%, bezogen auf das Gewicht der Farbmittelzusammensetzung, liegt.

15. Zusammensetzung nach einem der Ansprüche 7 bis 14, **dadurch gekennzeichnet, dass** es sich bei dem Polymer um ein konditionierendes Polymer oder ein fixierendes Polymer handelt.

16. Zusammensetzung nach dem vorhergehenden Anspruch, **dadurch gekennzeichnet, dass** der Mengenanteil des konditionierenden oder fixierenden Polymers im Bereich von 0,01 bis 20 Gew.-%, bezogen auf das Gewicht der Farbmittelzusammensetzung, und vorzugsweise 0,1 bis 5 Gew.-%, bezogen auf das Gewicht der Farbmittelzusammensetzung, liegt.

17. Zusammensetzung nach einem der Ansprüche 7 bis 16, **dadurch gekennzeichnet, dass** sie ferner einen ergänzenden Direktfarbstoff enthält, der von der nichtmetallischen kationischen Verbindung verschieden ist.

18. Zusammensetzung nach dem vorhergehenden Anspruch, **dadurch gekennzeichnet, dass** der Mengenanteil des ergänzenden Direktfarbstoffs 0,0005 bis 12 Gew.-%, bezogen auf das Gewicht der Zusammensetzung, ausmacht.

19. Zusammensetzung nach einem der Ansprüche 7 bis 18, **dadurch gekennzeichnet, dass** sie mindestens eine Oxidationsbase gegebenenfalls in Kombination mit mindestens einem Kuppler enthält.

20. Zusammensetzung nach dem vorhergehenden Anspruch, **dadurch gekennzeichnet, dass** der Mengenanteil jeder Oxidationsbase im Bereich von 0,0005 bis 12 Gew.-%, bezogen auf das Gesamtgewicht der Farbmittelzusammensetzung, liegt.

21. Zusammensetzung nach Anspruch 19, **dadurch gekennzeichnet, dass** der Mengenanteil jedes Kupplers im Bereich von 0,0001 bis 10 Gew.-%, bezogen auf das Gesamtgewicht der Farbmittelzusammensetzung, liegt.

22. Zusammensetzung nach einem der Ansprüche 7 bis 21, die mindestens ein Oxidationsmittel enthält.

23. Gebrauchsfertige Zusammensetzung, die in einem zum Färben von menschlichen Keratinsubstanzen geeigneten Medium mindestens eine Verbindung vom Typ der kationischen nichtmetallischen oder kationischen metallischen Porphyrine oder Phthalocyanine, die als metallisches Element nur ein oder mehrere Metalle oder Metallionen der Gruppe IA, IIA des Periodensystems der Elemente, Zink oder Silicium aufweisen, und mindestens ein Oxidationsmittel enthält.

24. Verfahren zum Färben von menschlichen Keratinsubstanzen und insbesondere Fasern, das darin besteht, die Zusammensetzung nach einem der Ansprüche 7 bis 21 auf die trockenen oder feuchten Keratinsubstanzen und vorzugsweise Fasern aufzutragen, ohne die Zusammensetzung am Ende auszuspülen.

25. Verfahren zum Färben von menschlichen Keratinsubstanzen und insbesondere Fasern, das darin besteht, die Zusammensetzung nach einem der Ansprüche 7 bis 22 in Gegenwart mindestens eines Oxidationsmittels auf die trockenen oder feuchten Keratinsubstanzen aufzutragen, während einer Zeitspanne, die für die Bildung der gewünschten Färbung ausreichend ist, einwirken zu lassen und anschließen die Zusammensetzung zu entfernen.

26. Vorrichtung mit mehreren Abteilungen für die Durchführung des Verfahrens nach Anspruch 25 zum Färben von Keratinsubstanzen und insbesondere Fasern, umfassend eine oder mehrere Abteilungen mit mindestens einer Verbindung vom Typ der kationischen nichtmetallischen oder kationischen metallischen Porphyrine oder Phthalocyanine, die als metallisches Element nur ein oder mehrere Metalle oder Metallionen der Gruppe IA, IIA des Periodensystems der Elemente, Zink oder Silicium enthält, gegebenenfalls einem ergänzenden Direktfarbstoff, gegebenenfalls mindestens einer Oxidationsbase und/oder mindestens einem Kuppler in einem zum Färben von menschlichen Keratinfasern geeigneten Medium; wobei diese Verbindungen gegebenenfalls kombiniert in einer oder mehreren Zusammensetzungen vorliegen, und andererseits eine Abteilung mit einer Zusammensetzung, die in einem zum Färben von menschlichen Keratinfasern geeigneten Medium mindestens ein Oxidationsmittel enthält.

27. Verbindung der folgenden Formel:
